# EUROPEAN PATENT APPLICATION

(11) **EP 3 800 203 A1**
(43) Date of publication of application: **07.04.2021**
(21) Application number: 20207020.7
(22) Date of filing: 27.02.2018
(51) Int. Cl.: C07K 16/30, C07K 16/28, A61K 39/00, A61K 39/395, A61P 35/00, C07K 16/46, C07K 16/40

(54) **MULTISPECIFIC BINDING PROTEINS TARGETING CAIX, ANO1, MESOTHELIN,TROP2, CEA, OR CLAUDIN-18.2**

(30) Priority: 27.02.2017 US 201762464347 P; 27.02.2017 US 201762464344 P; 27.02.2017 US 201762464341 P; 06.03.2017 US 201762467557 P; 20.03.2017 US 201762473652 P; 20.03.2017 US 201762473659 P
(62) Divisional of application: 18756655.9
(71) Applicant: Dragonfly Therapeutics, Inc., Waltham, MA 02451 (US)
(72) Inventor: CHANG, Gregory, P., Medford, MA 02155 (US); CHEUNG, Ann, F., Lincoln, MA 01773 (US); GUTIERREZ, Eva, Waltham, MA 02451 (US); HANEY, William, Wayland, MA 01778 (US); LUNDE, Bradley, M., Lebanon, NH 03766 (US); PRINZ, Bianka, Lebanon, NH 03766 (US)
(74) Representative: Haseltine Lake Kempner LLP

(57) **Abstract**

Multi-specific binding proteins that bind the NKG2D receptor, CD 16, and a tumor-associated antigen selected from carbonic anhydrase 9 (CAIX), anoctamin-1 (ANOI), mesothelin, TROP2, CEA and claudin-18.2 are described, as well as pharmaceutical compositions and therapeutic methods useful for the treatment of cancer.

## Description

### CROSS-REFERENCE TO RELATED APPLICATIONS

This application claims the benefit of and priority to U.S. application number 62/464,341 filed February 27, 2017, U.S. application number 62/464,344 filed February 27, 2017, U.S. application number 62/464,347 filed February 27, 2017, U.S. application number 62/467,557 filed March 06, 2017, U.S. application number 62/473,652 filed March 20, 2017, and U.S. application number 62/473,659 filed March 20, 2017, the entire contents of each of which are hereby incorporated by reference in their entireties for all purposes.

### SEQUENCE LISTING

The instant application contains a Sequence Listing which has been submitted electronically in ASCII format and is hereby incorporated by reference in its entirety. Said ASCII copy, created on February 27, 2018, is named DFY-012WO_ST25.txt and is 212,480 bytes in size.

### FIELD OF THE INVENTION

The invention relates to multi-specific binding proteins that bind to the NKG2D receptor, CD16, and a tumor-associated antigen selected from carbonic anhydrase 9 (CAIX), anoctamin-1 (ANO1), mesothelin, trophoblast cell-surface antigen (TROP2), carcinoembryonic antigen (CEA) and Claudin-18.2.

### BACKGROUND

Cancer continues to be a significant health problem despite the substantial research efforts and scientific advances reported in the literature for treating this disease. Some of the most frequently diagnosed cancers include prostate cancer, breast cancer, and lung cancer. Prostate cancer is the most common form of cancer in men. Breast cancer remains a leading cause of death in women. Small cell lung cancer remains among the most deadly of malignancies. Current treatment options for these cancers are not effective for all patients and/or can have substantial adverse side effects. Other types of cancer also remain challenging to treat using existing therapeutic options.

Cancer immunotherapies are desirable because they are highly specific and can facilitate destruction of cancer cells using the patient's own immune system. Fusion proteins such as bi-specific T-cell engagers are cancer immunotherapies described in the literature that bind to tumor cells and T-cells to facilitate destruction of tumor cells. Antibodies that bind to certain tumor-associated antigens and to certain immune cells have been described in the literature. *See, e.g.,* WO 2016/134371 and WO 2015/095412.

Natural killer (NK) cells are a component of the innate immune system and make up approximately 15% of circulating lymphocytes. NK cells infiltrate virtually all tissues and were originally characterized by their ability to kill tumor cells effectively without the need for prior sensitization. Activated NK cells kill target cells by means similar to cytotoxic T cells - *i.e.,* via cytolytic granules that contain perforin and granzymes as well as via death receptor pathways. Activated NK cells also secrete inflammatory cytokines such as IFN-γ and chemokines that promote the recruitment of other leukocytes to the target tissue.

NK cells respond to signals through a variety of activating and inhibitory receptors on their surface. For example, when NK cells encounter healthy self-cells, their activity is inhibited through activation of the killer-cell immunoglobulin-like receptors (KIRs). Alternatively, when NK cells encounter foreign cells or cancer cells, they are activated via their activating receptors (*e.g.,* NKG2D, NCRs, DNAM1). NK cells are also activated by the constant region of some immunoglobulins through CD16 receptors on their surface. The overall sensitivity of NK cells to activation depends on the sum of stimulatory and inhibitory signals.

Carbonic anhydrase 9 (CAIX) is a zinc metalloenzyme and belongs to a family of enzymes that are involved in reversible hydration of carbon dioxide to form bicarbonate and hydrogen ions. CAIX has limited expression in normal tissues, but is overexpressed in a wide variety of solid tumors and plays an important role in tumor development. Induced by hypoxic tumor microenvironments, CAIX functions to combat the deleterious effects of a high rate of glycolytic metabolism in tumor cells by maintaining an intracellular pH favorable for tumor cell growth and survival, while at the same time generating an acidic extracellular space facilitating tumor cell invasiveness. So far, overexpression of CAIX has been detected in renal cell carcinoma, breast cancer, glioblastoma, head and neck cancer, gastric cancers, bladder cancer, ovarian cancer, as well as in common epithelial cancers such as carcinomas of the esophagus, lung, colon, kidney, cervix and non-small cell lung carcinoma.

Mesothelin is a glycosylphosphatidylinositol (GPI) anchored cell surface protein, and is expressed in mesothelial cells of the pleura, peritoneum, and pericardium. In addition to its normal expression, mesothelin is overexpressed in a variety of cancers, including mesothelioma, ovarian cancer, pancreatic cancer, non-small cell lung cancer, breast cancer, cholangiocarcinoma, gastric cancer, uterine serous carcinoma, thymic carcinoma, and acute myeloid leukemia. It is suggested that mesothelin may play an important role in tumor progression including cell adherence, cell survival/proliferation and chemoresistance.

Anoctamin-1 (ANO1) is a voltage-sensitive calcium-activated chloride channel. It is also known by other names, such as TMEM16, TAOS2, ORAOV2, and DOG-1. Calcium-activated chloride channels function in many physiological processes, including transepithelial secretion, cardiac and neuronal excitation, sensory transduction, smooth muscle contraction, and fertilization. ANO1 is potentially involved in epithelial fluid secretion, olfactory and phototransduction, neuronal and cardiac excitability, and regulation of vascular tone including gut motility. ANO1 is also highly expressed in some cancers, where it is thought to augment cell proliferation, cell migration and metastasis, for example, in esophageal squamous cell cancer (ESCC), gastrointestinal stromal tumor (GIST), head and neck squamous cell carcinoma (HNSCC), pancreatic cancer, breast cancer, prostate cancer, and sarcoma. ∼ 85% of HNSCC tumors are found to be ANO1 positive, and HNSCC patients with high-level tumor expression of ANO1 have decreased overall survival; overexpression of ANO1 is also found to be closely correlated with malignancy of prostate cancer and poor prognosis of breast cancer.

TROP2 is a transmembrane glycoprotein and a Ca²⁺-signal transducer. In addition to its expression in certain normal tissues, TROP2 is overexpressed in a variety of cancers including breast, lung, gastric, colorectal, pancreatic, prostatic, cervical, head-and-neck cancer, nasopharyngeal carcinoma, and ovarian carcinoma. It signals cancer cells for proliferation, invasion, and survival.

Carcinoembryonic antigen (CEA), also known as CD66e or CEACAM5 is a member of the immunoglobulin superfamily. It is a large cell surface glycoprotein, and mainly serves as a cell adhesion molecule mediating intercellular contact. Besides its functions in cell adhesion and migration, CEA is found to be over-expressed in a high percentage of human cancers, including 90% of gastrointestinal, colorectal and pancreatic cancers, 70% of non-small cell lung cancer cells and 50% of breast cancers. Overexpression of CEA has been shown to correlate with tumorigenicity and enhanced tumor invasiveness.

Claudin-18.2 is one of the two isoforms Claudin-18, a major component of tight junctions which regulate permeability, barrier function and polarity of epithelial layers. Claudin-18.2 is expressed only in short-lived differentiated cells of the gastric mucosa in healthy tissues. However, it is overexpressed in up to 80% of gastrointestinal adenocarcinomas and 60% pancreatic tumors. In addition, its expression has been observed in distinct subsets of individuals with esophageal cancer, non-small cell lung carcinoma, ovarian cancer, colon cancer, and several forms of biliary ductal carcinoma.

### SUMMARY

The invention provides multi-specific binding proteins that bind to CAIX, ANO1, mesothelin, TROP2, CEA or Claudin-18.2 on a cancer cell and to the NKG2D receptor and CD16 receptor on natural killer cells. Such proteins can engage more than one kind of NK activating receptor, and may block the binding of natural ligands to NKG2D. In certain embodiments, the proteins can agonize NK cells in humans, and in other species such as rodents and cynomolgus monkeys. Various aspects and embodiments of the invention are described in further detail below.

Accordingly, one aspect of the invention provides a protein that incorporates a first antigen-binding site that binds NKG2D; a second antigen-binding site that binds CAIX, ANO1, mesothelin, TROP2, CEA or Claudin-18.2; and an antibody Fc domain, a portion thereof sufficient to bind CD16, or a third antigen-binding site that binds CD16. The antigen-binding sites may each incorporate an antibody heavy chain variable domain and an antibody light chain variable domain (*e.g*. arranged as in an antibody, or fused together to from an scFv), or one or more of the antigen-binding sites may be a single domain antibody, such as a V_{H}H antibody like a camelid antibody or a V_{NAR} antibody like those found in cartilaginous fish.

In one embodiment, the first antigen-binding site, which binds to NKG2D, incorporates a heavy chain variable domain related to SEQ ID NO: 1, such as by having an amino acid sequence at least 90% (*e.g.,* 90%, 91%, 92%, 93%, 94%, 95%, 96%, 97%, 98%, 99%, or 100%) identical to SEQ ID NO:1, and/or incorporating amino acid sequences identical to the CDR1 (SEQ ID NO:51), CDR2 (SEQ ID NO:52), and CDR3 (SEQ ID NO:53) sequences of SEQ ID NO:1. The heavy chain variable domain related to SEQ ID NO: 1 can be coupled a variety of light chain variable domains to form a NKG2D binding site. For example, the first antigen-binding site that incorporates a heavy chain variable domain related to SEQ ID NO: 1 can further incorporate a light chain variable domain selected from any one of the sequences related to SEQ ID NOs:2, 4, 6, 8, 10, 12, 14, 16, 18, 20, 22, 24, 26, 28, 30, 32, 34, 36, and 40. For example, the first antigen-binding site incorporates a heavy chain variable domain with amino acid sequences at least 90% (*e.g.,* 90%, 91%, 92%, 93%, 94%, 95%, 96%, 97%, 98%, 99%, or 100%) identical to SEQ ID NO:1 and a light chain variable domain with amino acid sequences at least 90% (*e.g.,* 90%, 91%, 92%, 93%, 94%, 95%, 96%, 97%, 98%, 99%, or 100%) identical to any one of the sequences selected from SEQ ID NOs:2, 4, 6, 8, 10, 12, 14, 16, 18, 20, 22, 24, 26, 28, 30, 32, 34, 36, and 40. Alternatively, the first antigen-binding site can incorporate a heavy chain variable domain related to SEQ ID NO:41 and a light chain variable domain related to SEQ ID NO:42. For example, the heavy chain variable domain of the first antigen binding site can be at least 90% (*e.g.,* 90%, 91%, 92%, 93%, 94%, 95%, 96%, 97%, 98%, 99%, or 100%) identical to SEQ ID NO:41, and/or incorporate amino acid sequences identical to the CDR1 (SEQ ID NO:54), CDR2 (SEQ ID NO:55), and CDR3 (SEQ ID NO:56) sequences of SEQ ID NO:41. Similarly, the light chain variable domain of the second antigen binding site can be at least 90% (*e.g.,* 90%, 91%, 92%, 93%, 94%, 95%, 96%, 97%, 98%, 99%, or 100%) identical to SEQ ID NO:42, and/or incorporate amino acid sequences identical to the CDR1 (SEQ ID NO:57), CDR2 (SEQ ID NO:58), and CDR3 (SEQ ID NO:59) sequences of SEQ ID NO:42. In other embodiments, the first antigen-binding site can incorporate a heavy chain variable domain related to SEQ ID NO:43 and a light chain variable domain related to SEQ ID NO:44. For example, the heavy chain variable domain of the first antigen binding site can be at least 90% (*e.g.,* 90%, 91%, 92%, 93%, 94%, 95%, 96%, 97%, 98%, 99%, or 100%) identical to SEQ ID NO:43, and/or incorporate amino acid sequences identical to the CDR1 (SEQ ID NO:60), CDR2 (SEQ ID NO:61), and CDR3 (SEQ ID NO:62) sequences of SEQ ID NO:43. Similarly, the light chain variable domain of the second antigen binding site can be at least 90% (*e.g.,* 90%, 91%, 92%, 93%, 94%, 95%, 96%, 97%, 98%, 99%, or 100%) identical to SEQ ID NO:44, and/or incorporate amino acid sequences identical to the CDR1 (SEQ ID NO:63), CDR2 (SEQ ID NO:64), and CDR3 (SEQ ID NO:65) sequences of SEQ ID NO:44. In certain embodiments, the first antigen-binding site can incorporate a heavy chain variable domain related to SEQ ID NO:45 and a light chain variable domain related to SEQ ID NO:46. For example, the heavy chain variable domain of the first antigen binding site can be at least 90% (*e.g.,* 90%, 91%, 92%, 93%, 94%, 95%, 96%, 97%, 98%, 99%, or 100%) identical to SEQ ID NO:45, and/or incorporate amino acid sequences identical to the CDR1 (SEQ ID NO:66), CDR2 (SEQ ID NO:67), and CDR3 (SEQ ID NO:68) sequences of SEQ ID NO:45. Similarly, the light chain variable domain of the second antigen binding site can be at least 90% (*e.g.,* 90%, 91%, 92%, 93%, 94%, 95%, 96%, 97%, 98%, 99%, or 100%) identical to SEQ ID NO:46, and/or incorporate amino acid sequences identical to the CDR1 (SEQ ID NO:69), CDR2 (SEQ ID NO:70), and CDR3 (SEQ ID NO:71) sequences of SEQ ID NO:46.

Alternatively, the first antigen-binding site can incorporate a heavy chain variable domain related to SEQ ID NO:47 and a light chain variable domain related to SEQ ID NO:48, such as by having amino acid sequences at least 90% (*e.g.,* 90%, 91%, 92%, 93%, 94%, 95%, 96%, 97%, 98%, 99%, or 100%) identical to SEQ ID NO:47 and SEQ ID NO:48 respectively. In another embodiment, the first antigen-binding site can incorporate a heavy chain variable domain related to SEQ ID NO:49 and a light chain variable domain related to SEQ ID NO:50, such as by having amino acid sequences at least 90% *(e.g.,* 90%, 91%, 92%, 93%, 94%, 95%, 96%, 97%, 98%, 99%, or 100%) identical to SEQ ID NO:49 and SEQ ID NO:50 respectively.

In some embodiments, the first antigen-binding site can incorporate a heavy chain variable domain related to SEQ ID NO: 132 and a light chain variable domain related to SEQ ID NO: 133. For example, the heavy chain variable domain of the first antigen binding site can be at least 90% (*e.g.,* 90%, 91%, 92%, 93%, 94%, 95%, 96%, 97%, 98%, 99%, or 100%) identical to SEQ ID NO: 132, and/or incorporate amino acid sequences identical to the CDR1 (SEQ ID NO: 134), CDR2 (SEQ ID NO: 135), and CDR3 (SEQ ID NO:136) sequences of SEQ ID NO: 132. Similarly, the light chain variable domain of the second antigen binding site can be at least 90% (*e.g.,* 90%, 91%, 92%, 93%, 94%, 95%, 96%, 97%, 98%, 99%, or 100%) identical to SEQ ID NO: 133, and/or incorporate amino acid sequences identical to the CDR1 (SEQ ID NO: 137), CDR2 (SEQ ID NO: 138), and CDR3 (SEQ ID NO:139) sequences of SEQ ID NO:133.

In some embodiments, the first antigen-binding site can incorporate a heavy chain variable domain related to SEQ ID NO:140 and a light chain variable domain related to SEQ ID NO: 141. For example, the heavy chain variable domain of the first antigen binding site can be at least 90% (*e.g.,* 90%, 91%, 92%, 93%, 94%, 95%, 96%, 97%, 98%, 99%, or 100%) identical to SEQ ID NO:140, and/or incorporate amino acid sequences identical to the CDR1 (SEQ ID NO: 142), CDR2 (SEQ ID NO: 143), and CDR3 (SEQ ID NO: 144) sequences of SEQ ID NO: 140. Similarly, the light chain variable domain of the second antigen binding site can be at least 90% (*e.g.,* 90%, 91%, 92%, 93%, 94%, 95%, 96%, 97%, 98%, 99%, or 100%) identical to SEQ ID NO:141, and/or incorporate amino acid sequences identical to the CDR1 (SEQ ID NO: 145), CDR2 (SEQ ID NO: 146), and CDR3 (SEQ ID NO: 147) sequences of SEQ ID NO:141.

In some embodiments, the first antigen-binding site can incorporate a heavy chain variable domain related to SEQ ID NO:148 and a light chain variable domain related to SEQ ID NO:149. For example, the heavy chain variable domain of the first antigen binding site can be at least 90% (*e.g.,* 90%, 91%, 92%, 93%, 94%, 95%, 96%, 97%, 98%, 99%, or 100%) identical to SEQ ID NO:148, and/or incorporate amino acid sequences identical to the CDR1 (SEQ ID NO:150), CDR2 (SEQ ID NO: 151), and CDR3 (SEQ ID NO:152) sequences of SEQ ID NO: 148. Similarly, the light chain variable domain of the second antigen binding site can be at least 90% (*e.g.,* 90%, 91%, 92%, 93%, 94%, 95%, 96%, 97%, 98%, 99%, or 100%) identical to SEQ ID NO:149, and/or incorporate amino acid sequences identical to the CDR1 (SEQ ID NO:153), CDR2 (SEQ ID NO: 154), and CDR3 (SEQ ID NO: 155) sequences of SEQ ID NO:149.

In some embodiments, the first antigen-binding site can incorporate a heavy chain variable domain related to SEQ ID NO:156 and a light chain variable domain related to SEQ ID NO:157. For example, the heavy chain variable domain of the first antigen binding site can be at least 90% (*e.g.,* 90%, 91%, 92%, 93%, 94%, 95%, 96%, 97%, 98%, 99%, or 100%) identical to SEQ ID NO:156, and/or incorporate amino acid sequences identical to the CDR1 (SEQ ID NO: 158), CDR2 (SEQ ID NO: 159), and CDR3 (SEQ ID NO: 160) sequences of SEQ ID NO: 156. Similarly, the light chain variable domain of the second antigen binding site can be at least 90% (*e.g.,* 90%, 91%, 92%, 93%, 94%, 95%, 96%, 97%, 98%, 99%, or 100%) identical to SEQ ID NO:157, and/or incorporate amino acid sequences identical to the CDR1 (SEQ ID NO: 161), CDR2 (SEQ ID NO: 162), and CDR3 (SEQ ID NO: 163) sequences of SEQ ID NO:157.

In some embodiments, the first antigen-binding site can incorporate a heavy chain variable domain related to SEQ ID NO:164 and a light chain variable domain related to SEQ ID NO:165. For example, the heavy chain variable domain of the first antigen binding site can be at least 90% (*e.g.,* 90%, 91%, 92%, 93%, 94%, 95%, 96%, 97%, 98%, 99%, or 100%) identical to SEQ ID NO:164, and/or incorporate amino acid sequences identical to the CDR1 (SEQ ID NO: 166), CDR2 (SEQ ID NO: 167), and CDR3 (SEQ ID NO: 168) sequences of SEQ ID NO: 164. Similarly, the light chain variable domain of the second antigen binding site can be at least 90% (*e.g.,* 90%, 91%, 92%, 93%, 94%, 95%, 96%, 97%, 98%, 99%, or 100%) identical to SEQ ID NO:165, and/or incorporate amino acid sequences identical to the CDR1 (SEQ ID NO: 169), CDR2 (SEQ ID NO: 170), and CDR3 (SEQ ID NO: 171) sequences of SEQ ID NO:165.

The second antigen binding site can bind to CAIX, ANO1, mesothelin, TROP2, CEA or Claudin-18.2. In some embodiments, the second antigen binding site binds to CAIX and incorporates a heavy chain variable domain and light chain variable domain that can be coupled to form a CAIX binding site. For example, the second antigen binding site can incorporate a heavy chain variable domain related to SEQ ID NO:72 and a light chain variable domain related to SEQ ID NO:73. For example, the heavy chain variable domain of the second antigen binding site can be at least 90% (*e.g.,* 90%, 91%, 92%, 93%, 94%, 95%, 96%, 97%, 98%, 99%, or 100%) identical to SEQ ID NO:72, and/or incorporate amino acid sequences identical to the CDR1 (SEQ ID NO:74), CDR2 (SEQ ID NO:75), and CDR3 (SEQ ID NO:76) sequences of SEQ ID NO:72. Similarly, the light chain variable domain of the second antigen binding site can be at least 90% (*e.g.,* 90%, 91%, 92%, 93%, 94%, 95%, 96%, 97%, 98%, 99%, or 100%) identical to SEQ ID NO:73, and/or incorporate amino acid sequences identical to the CDR1 (SEQ ID NO:77), CDR2 (SEQ ID NO:78), and CDR3 (SEQ ID NO:79) sequences of SEQ ID NO:73.

Alternatively, the second antigen binding site can incorporate a heavy chain variable domain related to SEQ ID NO:80 and a light chain variable domain related to SEQ ID NO:81. For example, the heavy chain variable domain of the second antigen binding site can be at least 90% (*e.g.,* 90%, 91%, 92%, 93%, 94%, 95%, 96%, 97%, 98%, 99%, or 100%) identical to SEQ ID NO:80, and/or incorporate amino acid sequences identical to the CDR1 (SEQ ID NO:82), CDR2 (SEQ ID NO:83), and CDR3 (SEQ ID NO:84) sequences of SEQ ID NO:80. Similarly, the light chain variable domain of the second antigen binding site can be at least 90% (*e.g*., 90%, 91%, 92%, 93%, 94%, 95%, 96%, 97%, 98%, 99%, or 100%) identical to SEQ ID NO:81, and/or incorporate amino acid sequences identical to the CDR1 (SEQ ID NO:85), CDR2 (SEQ ID NO:86), and CDR3 (SEQ ID NO:87) sequences of SEQ ID NO:81.

Alternatively, the second antigen binding site can incorporate a heavy chain variable domain related to SEQ ID NO:88 and a light chain variable domain related to SEQ ID NO:89. For example, the heavy chain variable domain of the second antigen binding site can be at least 90% (*e.g.,* 90%, 91%, 92%, 93%, 94%, 95%, 96%, 97%, 98%, 99%, or 100%) identical to SEQ ID NO:88, and/or incorporate amino acid sequences identical to the CDR1 (SEQ ID NO:90), CDR2 (SEQ ID NO:91), and CDR3 (SEQ ID NO:92) sequences of SEQ ID NO:88. Similarly, the light chain variable domain of the second antigen binding site can be at least 90% (*e.g*., 90%, 91%, 92%, 93%, 94%, 95%, 96%, 97%, 98%, 99%, or 100%) identical to SEQ ID NO:89, and/or incorporate amino acid sequences identical to the CDR1 (SEQ ID NO:93), CDR2 (SEQ ID NO:94), and CDR3 (SEQ ID NO:95) sequences of SEQ ID NO:89.

In some embodiments, the second antigen binding site binds to ANO1 and incorporates a heavy chain variable domain and light chain variable domain that can be coupled to form an ANO1 binding site. For example, the second antigen binding site can incorporate a variety a heavy chain variable domain related to SEQ ID NO:97 and a light chain variable domain related to SEQ ID NO:98. For example, the heavy chain variable domain of the second antigen binding site can be at least 90% (*e.g.,* 90%, 91%, 92%, 93%, 94%, 95%, 96%, 97%, 98%, 99%, or 100%) identical to SEQ ID NO:97, and/or incorporate amino acid sequences identical to the CDR1 (SEQ ID NO:99), CDR2 (SEQ ID NO:100), and CDR3 (SEQ ID NO:101) sequences of SEQ ID NO:97. Similarly, the light chain variable domain of the second antigen binding site can be at least 90% (*e.g.,* 90%, 91%, 92%, 93%, 94%, 95%, 96%, 97%, 98%, 99%, or 100%) identical to SEQ ID NO:98, and/or incorporate amino acid sequences identical to the CDR1 (SEQ ID NO: 102), CDR2 (SEQ ID NO:103), and CDR3 (SEQ ID NO:104) sequences of SEQ ID NO:98.

In some embodiments, the second antigen binding site binds to mesothelin and incorporates a heavy chain variable domain and light chain variable domain that can be coupled to form a mesothelin binding site. For example, the second antigen binding site can incorporate a variety a heavy chain variable domain related to SEQ ID NO:106 and a light chain variable domain related to SEQ ID NO:107. For example, the heavy chain variable domain of the second antigen binding site can be at least 90% (*e.g.,* 90%, 91%, 92%, 93%, 94%, 95%, 96%, 97%, 98%, 99%, or 100%) identical to SEQ ID NO:106, and/or incorporate amino acid sequences identical to the CDR1 (SEQ ID NO:108), CDR2 (SEQ ID NO:109), and CDR3 (SEQ ID NO:110) sequences of SEQ ID NO:106. Similarly, the light chain variable domain of the second antigen binding site can be at least 90% (*e.g.,* 90%, 91%, 92%, 93%, 94%, 95%, 96%, 97%, 98%, 99%, or 100%) identical to SEQ ID NO:107, and/or incorporate amino acid sequences identical to the CDR1 (SEQ ID NO:111), CDR2 (SEQ ID NO:112), and CDR3 (SEQ ID NO:113) sequences of SEQ ID NO:107. Alternatively, the second antigen binding site can incorporate a variety a heavy chain variable domain related to SEQ ID NO:114 and a light chain variable domain related to SEQ ID NO:115. For example, the heavy chain variable domain of the second antigen binding site can be at least 90% (*e.g.,* 90%, 91%, 92%, 93%, 94%, 95%, 96%, 97%, 98%, 99%, or 100%) identical to SEQ ID NO:114, and/or incorporate amino acid sequences identical to the CDR1 (SEQ ID NO:116), CDR2 (SEQ ID NO:117), and CDR3 (SEQ ID NO:118) sequences of SEQ ID NO:115. Similarly, the light chain variable domain of the second antigen binding site can be at least 90% (*e.g.,* 90%, 91%, 92%, 93%, 94%, 95%, 96%, 97%, 98%, 99%, or 100%) identical to SEQ ID NO:115, and/or incorporate amino acid sequences identical to the CDR1 (SEQ ID NO:119), CDR2 (SEQ ID NO:120), and CDR3 (SEQ ID NO:121) sequences of SEQ ID NO:115. Alternatively, the second antigen binding site can incorporate a variety a heavy chain variable domain related to SEQ ID NO:122 and a light chain variable domain related to SEQ ID NO:123. For example, the heavy chain variable domain of the second antigen binding site can be at least 90% (*e.g.,* 90%, 91%, 92%, 93%, 94%, 95%, 96%, 97%, 98%, 99%, or 100%) identical to SEQ ID NO:122, and/or incorporate amino acid sequences identical to the CDR1 (SEQ ID NO: 124), CDR2 (SEQ ID NO: 125), and CDR3 (SEQ ID NO: 126) sequences of SEQ ID NO: 122. Similarly, the light chain variable domain of the second antigen binding site can be at least 90% (*e.g.,* 90%, 91%, 92%, 93%, 94%, 95%, 96%, 97%, 98%, 99%, or 100%) identical to SEQ ID NO: 123, and/or incorporate amino acid sequences identical to the CDR1 (SEQ ID NO: 127), CDR2 (SEQ ID NO:128), and CDR3 (SEQ ID NO: 129) sequences of SEQ ID NO: 123.

In some embodiments, the second antigen binding site binds to TROP2 and incorporates a heavy chain variable domain and light chain variable domain that can be coupled to form a TROP2 binding site. For example, the second antigen binding site can incorporate a heavy chain variable domain related to SEQ ID NO: 172 and a light chain variable domain related to SEQ ID NO: 173. For example, the heavy chain variable domain of the second antigen binding site can be at least 90% (*e.g.,* 90%, 91%, 92%, 93%, 94%, 95%, 96%, 97%, 98%, 99%, or 100%) identical to SEQ ID NO: 172, and/or incorporate amino acid sequences identical to the CDR1 (SEQ ID NO: 174), CDR2 (SEQ ID NO: 175), and CDR3 (SEQ ID NO:176) sequences of SEQ ID NO: 172. Similarly, the light chain variable domain of the second antigen binding site can be at least 90% (*e.g.,* 90%, 91%, 92%, 93%, 94%, 95%, 96%, 97%, 98%, 99%, or 100%) identical to SEQ ID NO: 173, and/or incorporate amino acid sequences identical to the CDR1 (SEQ ID NO: 177), CDR2 (SEQ ID NO: 178), and CDR3 (SEQ ID NO: 179) sequences of SEQ ID NO: 173. Alternatively, the second antigen binding site can incorporate a heavy chain variable domain related to SEQ ID NO:180 and a light chain variable domain related to SEQ ID NO:181. For example, the heavy chain variable domain of the second antigen binding site can be at least 90% (*e.g.,* 90%, 91%, 92%, 93%, 94%, 95%, 96%, 97%, 98%, 99%, or 100%) identical to SEQ ID NO: 180, and/or incorporate amino acid sequences identical to the CDR1 (SEQ ID NO: 182), CDR2 (SEQ ID NO: 183), and CDR3 (SEQ ID NO:184) sequences of SEQ ID NO:180. Similarly, the light chain variable domain of the second antigen binding site can be at least 90% (*e.g.,* 90%, 91%, 92%, 93%, 94%, 95%, 96%, 97%, 98%, 99%, or 100%) identical to SEQ ID NO:181, and/or incorporate amino acid sequences identical to the CDR1 (SEQ ID NO:185), CDR2 (SEQ ID NO:186), and CDR3 (SEQ ID NO:187) sequences of SEQ ID NO:181. Alternatively, the second antigen binding site can incorporate a heavy chain variable domain related to SEQ ID NO: 188 and a light chain variable domain related to SEQ ID NO:189. For example, the heavy chain variable domain of the second antigen binding site can be at least 90% (*e.g.,* 90%, 91%, 92%, 93%, 94%, 95%, 96%, 97%, 98%, 99%, or 100%) identical to SEQ ID NO: 189, and the light chain variable domain of the second antigen binding site can be at least 90% (*e.g.,* 90%, 91%, 92%, 93%, 94%, 95%, 96%, 97%, 98%, 99%, or 100%) identical to SEQ ID NO:189. Alternatively, the second antigen binding site can incorporate a heavy chain variable domain related to SEQ ID NO:190 and a light chain variable domain related to SEQ ID NO:191. For example, the heavy chain variable domain of the second antigen binding site can be at least 90% (*e.g.,* 90%, 91%, 92%, 93%, 94%, 95%, 96%, 97%, 98%, 99%, or 100%) identical to SEQ ID NO: 190, and the light chain variable domain of the second antigen binding site can be at least 90% (*e.g.,* 90%, 91%, 92%, 93%, 94%, 95%, 96%, 97%, 98%, 99%, or 100%) identical to SEQ ID NO:191. Alternatively, the second antigen binding site can incorporate a heavy chain variable domain related to SEQ ID NO:192 and a light chain variable domain related to SEQ ID NO:193. For example, the heavy chain variable domain of the second antigen binding site can be at least 90% (*e.g.,* 90%, 91%, 92%, 93%, 94%, 95%, 96%, 97%, 98%, 99%, or 100%) identical to SEQ ID NO: 192, and the light chain variable domain of the second antigen binding site can be at least 90% (*e.g.,* 90%, 91%, 92%, 93%, 94%, 95%, 96%, 97%, 98%, 99%, or 100%) identical to SEQ ID NO:193.

In some embodiments, the second antigen binding site binds to CEA and incorporates a heavy chain variable domain and light chain variable domain that can be coupled to form a CEA binding site. For example, the second antigen binding site can incorporate a heavy chain variable domain related to SEQ ID NO:195 and a light chain variable domain related to SEQ ID NO: 196. For example, the heavy chain variable domain of the second antigen binding site can be at least 90% (*e.g.,* 90%, 91%, 92%, 93%, 94%, 95%, 96%, 97%, 98%, 99%, or 100%) identical to SEQ ID NO:195, and/or incorporate amino acid sequences identical to the CDR1 (SEQ ID NO: 197), CDR2 (SEQ ID NO: 198), and CDR3 (SEQ ID NO: 199) sequences of SEQ ID NO:195. Similarly, the light chain variable domain of the second antigen binding site can be at least 90% (*e.g.,* 90%, 91%, 92%, 93%, 94%, 95%, 96%, 97%, 98%, 99%, or 100%) identical to SEQ ID NO:196, and/or incorporate amino acid sequences identical to the CDR1 (SEQ ID NO:200), CDR2 (SEQ ID NO:201), and CDR3 (SEQ ID NO:202) sequences of SEQ ID NO: 196. Alternatively, the second antigen binding site can incorporate a heavy chain variable domain related to SEQ ID NO:203 and a light chain variable domain related to SEQ ID NO:204. For example, the heavy chain variable domain of the second antigen binding site can be at least 90% (*e.g.,* 90%, 91%, 92%, 93%, 94%, 95%, 96%, 97%, 98%, 99%, or 100%) identical to SEQ ID NO:203, and/or incorporate amino acid sequences identical to the CDR1 (SEQ ID NO:205), CDR2 (SEQ ID NO:206), and CDR3 (SEQ ID NO:207) sequences of SEQ ID NO:203. Similarly, the light chain variable domain of the second antigen binding site can be at least 90% (*e.g.,* 90%, 91%, 92%, 93%, 94%, 95%, 96%, 97%, 98%, 99%, or 100%) identical to SEQ ID NO:204, and/or incorporate amino acid sequences identical to the CDR1 (SEQ ID NO:208), CDR2 (SEQ ID NO:209), and CDR3 (SEQ ID NO:210) sequences of SEQ ID NO:204. Alternatively, the second antigen binding site can incorporate a heavy chain variable domain related to SEQ ID NO:211 and a light chain variable domain related to SEQ ID NO:212. For example, the heavy chain variable domain of the second antigen binding site can be at least 90% (*e.g.,* 90%, 91%, 92%, 93%, 94%, 95%, 96%, 97%, 98%, 99%, or 100%) identical to SEQ ID NO:211, and/or incorporate amino acid sequences identical to the CDR1 (SEQ ID NO:213), CDR2 (SEQ ID NO:214), and CDR3 (SEQ ID NO:215) sequences of SEQ ID NO:212. Similarly, the light chain variable domain of the second antigen binding site can be at least 90% (*e.g.,* 90%, 91%, 92%, 93%, 94%, 95%, 96%, 97%, 98%, 99%, or 100%) identical to SEQ ID NO:196, and/or incorporate amino acid sequences identical to the CDR1 (SEQ ID NO:216), CDR2 (SEQ ID NO:217), and CDR3 (SEQ ID NO:218) sequences of SEQ ID NO:212.

In some embodiments, the second antigen binding site binds to Claudin-18.2 and incorporates a heavy chain variable domain and light chain variable domain that can be coupled to form a Claudin-18.2 binding site. For example, the second antigen binding site can incorporate a heavy chain variable domain related to SEQ ID NO:220 and a light chain variable domain related to SEQ ID NO:221. For example, the heavy chain variable domain of the second antigen binding site can be at least 90% (*e.g.,* 90%, 91%, 92%, 93%, 94%, 95%, 96%, 97%, 98%, 99%, or 100%) identical to SEQ ID NO:220, and/or incorporate amino acid sequences identical to the CDR1 (SEQ ID NO:222), CDR2 (SEQ ID NO:223), and CDR3 (SEQ ID NO:224) sequences of SEQ ID NO:220. Similarly, the light chain variable domain of the second antigen binding site can be at least 90% (*e.g.,* 90%, 91%, 92%, 93%, 94%, 95%, 96%, 97%, 98%, 99%, or 100%) identical to SEQ ID NO:221, and/or incorporate amino acid sequences identical to the CDR1 (SEQ ID NO:225), CDR2 (SEQ ID NO:226), and CDR3 (SEQ ID NO:227) sequences of SEQ ID NO:221. For example, the second antigen binding site can incorporate a heavy chain variable domain related to SEQ ID NO:228 and a light chain variable domain related to SEQ ID NO:229. For example, the heavy chain variable domain of the second antigen binding site can be at least 90% (*e.g.,* 90%, 91%, 92%, 93%, 94%, 95%, 96%, 97%, 98%, 99%, or 100%) identical to SEQ ID NO:228, and/or incorporate amino acid sequences identical to the CDR1 (SEQ ID NO:230), CDR2 (SEQ ID NO:231), and CDR3 (SEQ ID NO:232) sequences of SEQ ID NO:228. Similarly, the light chain variable domain of the second antigen binding site can be at least 90% (*e.g.,* 90%, 91%, 92%, 93%, 94%, 95%, 96%, 97%, 98%, 99%, or 100%) identical to SEQ ID NO:229, and/or incorporate amino acid sequences identical to the CDR1 (SEQ ID NO:233), CDR2 (SEQ ID NO:234), and CDR3 (SEQ ID NO:235) sequences of SEQ ID NO:229.

In some embodiments, the second antigen binding site incorporates a light chain variable domain having an amino acid sequence identical to the amino acid sequence of the light chain variable domain present in the first antigen binding site.

In some embodiments, the protein incorporates a portion of an antibody Fc domain sufficient to bind CD16, wherein the antibody Fc domain comprises hinge and CH2 domains, and/or amino acid sequences at least 90% identical to amino acid sequence 234-332 of a human IgG antibody.

Formulations containing one of these proteins; cells containing one or more nucleic acids expressing these proteins, and methods of enhancing tumor cell death using these proteins are also provided.

Another aspect of the invention provides a method of treating cancer in a patient. The method comprises administering to a patient in need thereof a therapeutically effective amount of the multi-specific binding proteins described herein. Cancers to be treated using CAIX-targeting multi-specific binding proteins include, for example, renal cell carcinoma, breast cancer, glioblastoma, head and neck cancer, gastric cancers, bladder cancer, ovarian cancer, tumors of the esophagus, lung, colon, kidney, cervix and non-small cell lung carcinoma. Cancers to be treated using mesothelin-targeting multi-specific binding proteins include, for example, mesothelioma, ovarian cancer, pancreatic cancer, non-small cell lung cancer, breast cancer, cholangiocarcinoma, gastric cancer, uterine serous carcinoma, thymic carcinoma, and acute myeloid leukemia. Cancers to be treated using ANO1-targeting multi-specific binding proteins include, for example, esophageal squamous cell cancer (ESCC), gastrointestinal stromal tumor (GIST), head and neck squamous cell carcinoma (HNSCC), pancreatic cancer, breast cancer, prostate cancer, and sarcoma. Cancers to be treated using TROP2-targeting multi-specific binding proteins include, for example, breast, lung, gastric, colorectal, pancreatic, prostatic, cervical, head-and-neck cancer, nasopharyngeal carcinoma, and ovarian carcinoma. Cancers to be treated using CEA-targeting multi-specific binding proteins include, for example, gastrointestinal cancer, colorectal cancer, pancreatic cancer, non-small cell lung cancer and breast cancer. Cancers to be treated using claudin-18.2-targeting multi-specific binding proteins include, for example, esophageal cancer, non-small cell lung carcinoma, ovarian cancer, colon cancer, and several forms of biliary ductal carcinoma.

### BRIEF DESCRIPTION OF THE DRAWINGS

**FIG. 1** is a representation of a heterodimeric, multi-specific antibody. Each arm can represent either the NKG2D-binding domain or one of the tumor associated antigen-binding domain selected from CAIX, ANO1, mesothelin, TROP2, CEA and Claudin-18.2 binding domains. In some embodiments, the NKG2D- and the tumor associated antigen-binding domains can share a common light chain.
**FIG. 2** is a representation of a heterodimeric, multi-specific antibody. Either the NKG2D- or the tumor associated antigen-binding domain selected from CAIX, ANO1, mesothelin, TROP2, CEA and Claudin-18.2 binding domains can take the scFv format (right arm).
**FIG. 3** are line graphs demonstrating the binding affinity of NKG2D-binding domains (listed as clones) to human recombinant NKG2D in an ELISA assay.
**FIG. 4** are line graphs demonstrating the binding affinity of NKG2D-binding domains (listed as clones) to cynomolgus recombinant NKG2D in an ELISA assay.
**FIG. 5** are line graphs demonstrating the binding affinity of NKG2D-binding domains (listed as clones) to mouse recombinant NKG2D in an ELISA assay.
**FIG. 6** are bar graphs demonstrating the binding of NKG2D-binding domains (listed as clones) to EL4 cells expressing human NKG2D by flow cytometry showing mean fluorescence intensity (MFI) fold over background.
**FIG. 7** are bar graphs demonstrating the binding of NKG2D-binding domains (listed as clones) to EL4 cells expressing mouse NKG2D by flow cytometry showing mean fluorescence intensity (MFI) fold over background.
**FIG. 8** are line graphs demonstrating specific binding affinity of NKG2D-binding domains (listed as clones) to recombinant human NKG2D-Fc by competing with natural ligand ULBP-6.
**FIG. 9** are line graphs demonstrating specific binding affinity of NKG2D-binding domains (listed as clones) to recombinant human NKG2D-Fc by competing with natural ligand MICA.
**FIG. 10** are line graphs demonstrating specific binding affinity of NKG2D-binding domains (listed as clones) to recombinant mouse NKG2D-Fc by competing with natural ligand Rae-1 delta.
**FIG. 11** are bar graphs showing activation of human NKG2D by NKG2D-binding domains (listed as clones) by quantifying the percentage of TNF-alpha positive cells, which express human NKG2D-CD3 zeta fusion proteins.
**FIG. 12** are bar graphs showing activation of mouse NKG2D by NKG2D-binding domains (listed as clones) by quantifying the percentage of TNF-alpha positive cells, which express mouse NKG2D-CD3 zeta fusion proteins.
**FIG. 13** are bar graphs showing activation of human NK cells by NKG2D-binding domains (listed as clones).
**FIG. 14** are bar graphs showing activation of human NK cells by NKG2D-binding domains (listed as clones).
**FIG. 15** are bar graphs showing activation of mouse NK cells by NKG2D-binding domains (listed as clones).
**FIG. 16** are bar graphs showing activation of mouse NK cells by NKG2D-binding domains (listed as clones).
**FIG. 17** are bar graphs showing the cytotoxic effect of NKG2D-binding domains (listed as clones) on tumor cells.
**FIG. 18** are bar graphs showing the melting temperature of NKG2D-binding domains (listed as clones) measured by differential scanning fluorimetry.
**FIGs. 19A-19C** are bar graphs of synergistic activation of NK cells using CD16 and NKG2D binding. FIG. 19A demonstrates levels of CD107a; FIG. 19B demonstrates levels of IFNγ; FIG. 19C demonstrates levels of CD107a and IFNγ. Graphs indicate the mean (n = 2) ± SD. Data are representative of five independent experiments using five different healthy donors.
**FIG. 20** is a representation of a TriNKET in the Triomab form, which is a trifunctional, bispecific antibody that maintains an IgG-like shape. This chimera consists of two half antibodies, each with one light and one heavy chain, that originate from two parental antibodies. Triomab form may be a heterodimeric construct containing ½ of rat antibody and ½ of mouse antibody.

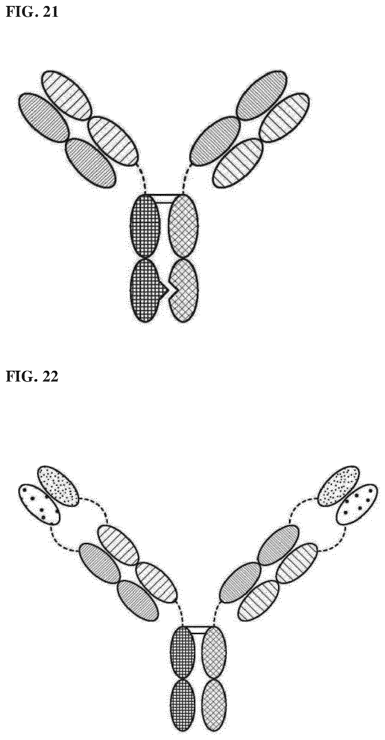

**FIG. 21** is a representation of a TriNKET in the KiH Common Light Chain (LC) form, which involves the knobs-into-holes (KIHs) technology. KiH is a heterodimer containing 2 Fabs binding to target 1 and 2, and an Fc stabilized by heterodimerization mutations. TriNKET in the KiH format may be a heterodimeric construct with 2 Fabs binding to target 1 and target 2, containing two different heavy chains and a common light chain that pairs with both heavy chains.
**FIG. 22** is a representation of a TriNKET in the dual-variable domain immunoglobulin (DVD-Ig™) form, which combines the target-binding domains of two monoclonal antibodies via flexible naturally occurring linkers, and yields a tetravalent IgG-like molecule. DVD-Ig™ is a homodimeric construct where variable domain targeting antigen 2 is fused to the N-terminus of a variable domain of Fab targeting antigen 1 Construct contains normal Fc.

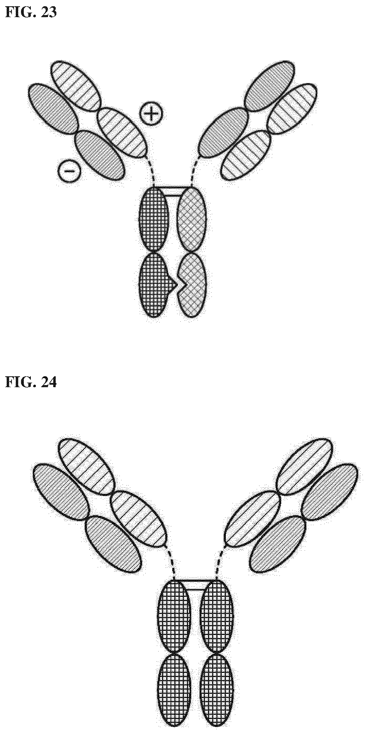

**FIG. 23** is a representation of a TriNKET in the Orthogonal Fab interface (Ortho-Fab) form, which is a heterodimeric construct that contains 2 Fabs binding to target 1 and target 2 fused to Fc. LC-HC pairing is ensured by orthogonal interface. Heterodimerization is ensured by mutations in the Fc.
**FIG. 24** is a representation of a TriNKET in the 2-in-1 Ig format.

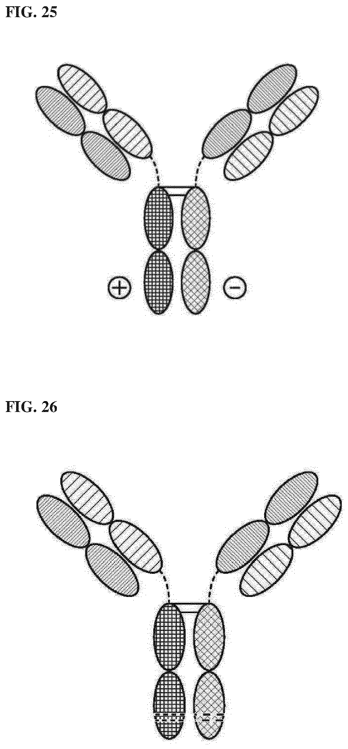

**FIG. 25** is a representation of a TriNKET in the ES form, which is a heterodimeric construct containing two different Fabs binding to target 1 and target 2 fused to the Fc. Heterodimerization is ensured by electrostatic steering mutations in the Fc.
**FIG. 26** is a representation of a TriNKET in the Fab Arm Exchange form: antibodies that exchange Fab arms by swapping a heavy chain and attached light chain (half-molecule) with a heavy-light chain pair from another molecule, resulting in bispecific antibodies. Fab Arm Exchange form (cFae) is a heterodimer containing 2 Fabs binding to target 1 and 2, and an Fc stabilized by heterodimerization mutations.

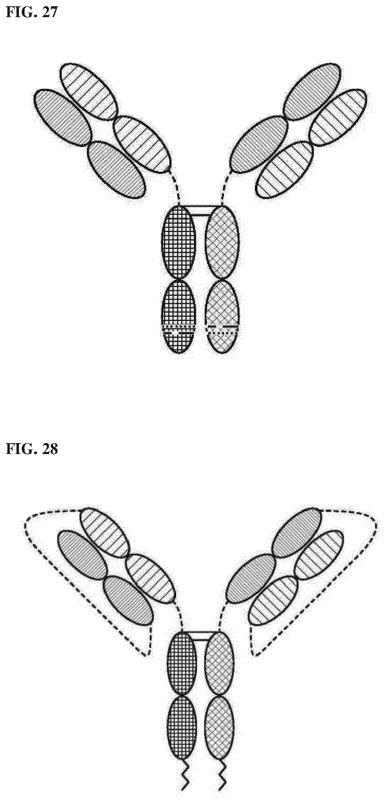

**FIG. 27** is a representation of a TriNKET in the SEED Body form, which is an heterodimer containing 2 Fabs binding to target 1 and 2, and an Fc stabilized by heterodimerization mutations.
**FIG. 28** is a representation of a TriNKET in the LuZ-Y form, in which leucine zipper is used to induce heterodimerization of two different HCs. LuZ-Y form is a heterodimer containing two different scFabs binding to target 1 and 2, fused to Fc. Heterodimerization is ensured through leucine zipper motifs fused to C-terminus of Fc.

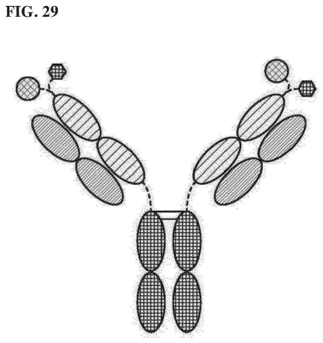

**FIG. 29** is a representation of a TriNKET in the Cov-X-Body form.
**FIGs. 30A-30B** are representations of TriNKETs in the κλ-Body forms, which are heterodimeric constructs with two different Fabs fused to Fc stabilized by heterodimerization mutations: Fab1 targeting antigen 1 contains kappa LC, while second Fab targeting antigen 2 contains lambda LC. FIG. 30A is an exemplary representation of one form of a κλ-Body; FIG. 30B is an exemplary representation of another κλ-Body.

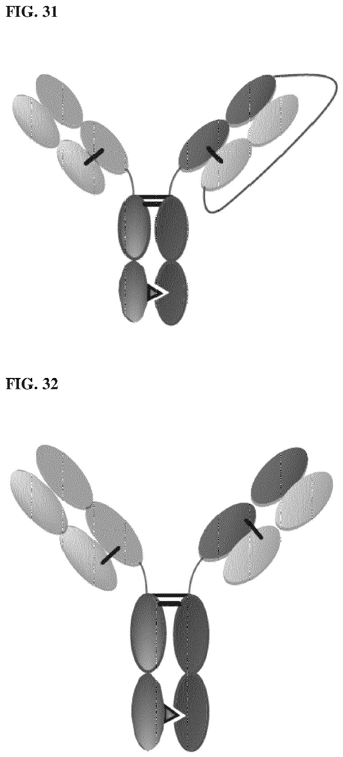

**FIG. 31** is an Oasc-Fab heterodimeric construct that includes Fab binding to target 1 and scFab binding to target 2 fused to Fc. Heterodimerization is ensured by mutations in the Fc.
**FIG. 32** is a DuetMab, which is a heterodimeric construct containing two different Fabs binding to antigens 1 and 2, and Fc stabilized by heterodimerization mutations. Fab 1 and 2 contain differential S-S bridges that ensure correct light chain (LC) and heavy chain (HC) pairing.

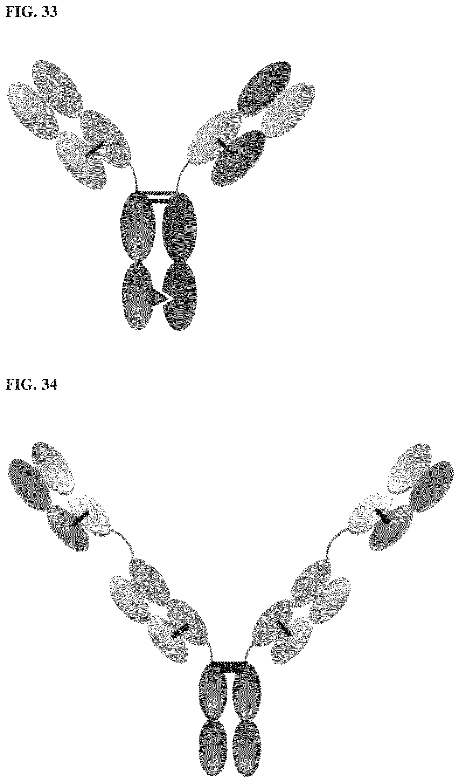

**FIG. 33** is a CrossmAb, which is a heterodimeric construct with two different Fabs binding to targets 1 and 2 fused to Fc stabilized by heterodimerization. CL and CH1 domains and VH and VL domains are switched, e.g., CH1 is fused in-line with VL, while CL is fused in-line with VH
**FIG. 34** is a Fit-Ig, which is a homodimeric construct where Fab binding to antigen 2 is fused to the N-terminus of HC of Fab that binds to antigen 1. The construct contains wild-type Fc.
**FIG. 35** shows binding of TriNKETs which include a CAIX-binding domain and a NKG2D-binding domain to NKG2D expressed on EL4 cells.
**FIG. 36** shows binding of TriNKETs that include a CAIX-binding domain and a NKG2D-binding domain, to CAIX expressed on human renal cell carcinoma cells A498.
**FIG. 37A** shows TriNKETs-mediated cytotoxicity of KHYG-1 effector cells towards CAIX-positive renal cell carcinoma A498 cells. The dotted line indicates the background killing of A498 cells during co-culture with rested KHYG-1 cells without addition of TriNKETs. **FIG. 37B** shows TriNKETs-mediated cytotoxicity of rested NK cells towards CAIX-positive renal cell carcinoma A498 cells.

### DETAILED DESCRIPTION

The invention provides multi-specific binding proteins that bind CAIX, ANO1, mesothelin, TROP2, CEA or Claudin-18.2 on a cancer cell and the NKG2D receptor and CD16 receptor on natural killer cells to activate the natural killer cells, pharmaceutical compositions comprising such multi-specific binding proteins, and therapeutic methods using such multi-specific proteins and pharmaceutical compositions, including for the treatment of cancer. Various aspects of the invention are set forth below in sections; however, aspects of the invention described in one particular section are not to be limited to any particular section.

To facilitate an understanding of the present invention, a number of terms and phrases are defined below.

The terms "a" and "an" as used herein mean "one or more" and include the plural unless the context is inappropriate. As used herein, the term "antigen-binding site" refers to the part of the immunoglobulin molecule that participates in antigen binding. In human antibodies, the antigen-binding site is formed by amino acid residues of the N-terminal variable ("V") regions of the heavy ("H") and light ("L") chains. Three highly divergent stretches within the V regions of the heavy and light chains are referred to as "hypervariable regions" which are interposed between more conserved flanking stretches known as "framework regions," or "FRs." Thus the term "FR" refers to amino acid sequences which are naturally found between and adjacent to hypervariable regions in immunoglobulins. In a human antibody molecule, the three hypervariable regions of a light chain and the three hypervariable regions of a heavy chain are disposed relative to each other in three dimensional space to form an antigen-binding surface. The antigen-binding surface is complementary to the three-dimensional surface of a bound antigen, and the three hypervariable regions of each of the heavy and light chains are referred to as "complementarity-determining regions," or "CDRs." In certain animals, such as camels and cartilaginous fish, the antigen-binding site is formed by a single antibody chain providing a "single domain antibody." Antigen-binding sites can exist in an intact antibody, in an antigen-binding fragment of an antibody that retains the antigen-binding surface, or in a recombinant polypeptide such as an scFv, using a peptide linker to connect the heavy chain variable domain to the light chain variable domain in a single polypeptide.

The term "tumor associated antigen" as used herein means any antigen including but not limited to a protein, glycoprotein, ganglioside, carbohydrate, lipid that is associated with cancer. Such antigen can be expressed on malignant cells or in the tumor microenvironment such as on tumor-associated blood vessels, extracellular matrix, mesenchymal stroma, or immune infiltrates.

As used herein, the terms "subject" and "patient" refer to an organism to be treated by the methods and compositions described herein. Such organisms preferably include, but are not limited to, mammals (*e.g*., murines, simians, equines, bovines, porcines, canines, felines, and the like), and more preferably include humans.

As used herein, the term "effective amount" refers to the amount of a compound (*e.g.,* a compound of the present invention) sufficient to effect beneficial or desired results. An effective amount can be administered in one or more administrations, applications or dosages and is not intended to be limited to a particular formulation or administration route. As used herein, the term "treating" includes any effect, *e.g*., lessening, reducing, modulating, ameliorating or eliminating, that results in the improvement of the condition, disease, disorder, and the like, or ameliorating a symptom thereof.

As used herein, the term "pharmaceutical composition" refers to the combination of an active agent with a carrier, inert or active, making the composition especially suitable for diagnostic or therapeutic use *in vivo* or *ex vivo.*

As used herein, the term "pharmaceutically acceptable carrier" refers to any of the standard pharmaceutical carriers, such as a phosphate buffered saline solution, water, emulsions (*e.g.,* such as an oil/water or water/oil emulsions), and various types of wetting agents. The compositions also can include stabilizers and preservatives. For examples of carriers, stabilizers and adjuvants, *see e.g.,* Martin, Remington's Pharmaceutical Sciences, 15th Ed., Mack Publ. Co., Easton, PA [1975].

As used herein, the term "pharmaceutically acceptable salt" refers to any pharmaceutically acceptable salt (*e.g.,* acid or base) of a compound of the present invention which, upon administration to a subject, is capable of providing a compound of this invention or an active metabolite or residue thereof. As is known to those of skill in the art, "salts" of the compounds of the present invention may be derived from inorganic or organic acids and bases. Exemplary acids include, but are not limited to, hydrochloric, hydrobromic, sulfuric, nitric, perchloric, fumaric, maleic, phosphoric, glycolic, lactic, salicylic, succinic, toluene-p-sulfonic, tartaric, acetic, citric, methanesulfonic, ethanesulfonic, formic, benzoic, malonic, naphthalene-2-sulfonic, benzenesulfonic acid, and the like. Other acids, such as oxalic, while not in themselves pharmaceutically acceptable, may be employed in the preparation of salts useful as intermediates in obtaining the compounds of the invention and their pharmaceutically acceptable acid addition salts.

Exemplary bases include, but are not limited to, alkali metal (*e.g*., sodium) hydroxides, alkaline earth metal (*e.g*., magnesium) hydroxides, ammonia, and compounds of formula NW₄⁺, wherein W is C₁₋₄ alkyl, and the like.

Exemplary salts include, but are not limited to: acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, flucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, palmoate, pectinate, persulfate, phenylpropionate, picrate, pivalate, propionate, succinate, tartrate, thiocyanate, tosylate, undecanoate, and the like. Other examples of salts include anions of the compounds of the present invention compounded with a suitable cation such as Na⁺, NH₄⁺, and NW₄⁺ (wherein W is a C₁₋₄ alkyl group), and the like.

For therapeutic use, salts of the compounds of the present invention are contemplated as being pharmaceutically acceptable. However, salts of acids and bases that are non-pharmaceutically acceptable may also find use, for example, in the preparation or purification of a pharmaceutically acceptable compound.

Throughout the description, where compositions are described as having, including, or comprising specific components, or where processes and methods are described as having, including, or comprising specific steps, it is contemplated that, additionally, there are compositions of the present invention that consist essentially of, or consist of, the recited components, and that there are processes and methods according to the present invention that consist essentially of, or consist of, the recited processing steps.

As a general matter, compositions specifying a percentage are by weight unless otherwise specified. Further, if a variable is not accompanied by a definition, then the previous definition of the variable controls.

### I. PROTEINS

The invention provides multi-specific binding proteins that bind CAIX, ANO1, mesothelin, TROP2, CEA or Claudin-18.2 on a cancer cell and the NKG2D receptor and CD16 receptor on natural killer cells to activate the natural killer cell. The multi-specific binding proteins are useful in the pharmaceutical compositions and therapeutic methods described herein. Binding of the multi-specific binding protein to the NKG2D receptor and CD16 receptor on natural killer cell enhances the activity of the natural killer cell toward destruction of a cancer cell. Binding of the multi-specific binding protein to CAIX, ANO1, mesothelin, TROP2, CEA or Claudin-18.2 on a cancer cell brings the cancer cell into proximity with the natural killer cell, which facilitates direct and indirect destruction of the cancer cell by the natural killer cell. Further description of exemplary multi-specific binding proteins is provided below.

The first component of the multi-specific binding proteins binds to NKG2D receptor-expressing cells, which can include but are not limited to NK cells, γδ T cells and CD8⁺ αβ T cells. Upon NKG2D binding, the multi-specific binding proteins may block natural ligands, such as ULBP6 and MICA, from binding to NKG2D and activating NKG2D receptors.

The second component of the multi-specific binding proteins binds to CAIX, ANO1, mesothelin, TROP2, CEA or Claudin-18.2-expressing cells. CAIX-expressing cells include, but are not limited to, renal cell carcinoma, breast cancer, glioblastoma, head and neck cancer, gastric cancers, bladder cancer, ovarian cancer, tumors of the esophagus, lung, colon, kidney, cervix and non-small cell lung carcinoma. Mesothelin-expressing cells include, but are not limited to, mesothelioma, ovarian cancer, pancreatic cancer, non-small cell lung cancer, breast cancer, cholangiocarcinoma, gastric cancer, uterine serous carcinoma, thymic carcinoma, and acute myeloid leukemia. ANO1-expressing cells include, but are not limited to, esophageal squamous cell cancer (ESCC), gastrointestinal stromal tumor (GIST), head and neck squamous cell carcinoma (HNSCC), pancreatic cancer, breast cancer, prostate cancer, and sarcoma. TROP2-expressing cells include, but are not limited to, breast, lung, gastric, colorectal, pancreatic, prostatic, cervical, head-and-neck cancer, nasopharyngeal carcinoma, and ovarian carcinoma. CEA-expressing cells include, but are not limited to, gastrointestinal cancer, colorectal cancer, pancreatic cancer, non-small cell lung cancer and breast cancer. Claudin-18.2-expressing cells include, but are not limited to, esophageal cancer, non-small cell lung carcinoma, ovarian cancer, colon cancer, and several forms of biliary ductal carcinoma.

The third component for the multi-specific binding proteins binds to cells expressing CD16, an Fc receptor on the surface of leukocytes including natural killer cells, macrophages, neutrophils, eosinophils, mast cells, and follicular dendritic cells.

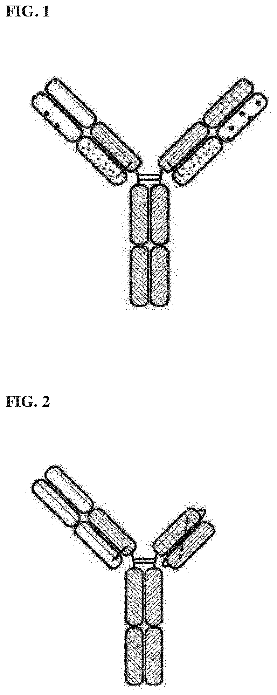

The multi-specific binding proteins described herein can take various formats. For example, one format is a heterodimeric, multi-specific antibody including a first immunoglobulin heavy chain, a first immunoglobulin light chain, a second immunoglobulin heavy chain and a second immunoglobulin light chain (FIG. 1). The first immunoglobulin heavy chain includes a first Fc (hinge-CH2-CH3) domain, a first heavy chain variable domain and optionally a first CH1 heavy chain domain. The first immunoglobulin light chain includes a first light chain variable domain and a first light chain constant domain. The first immunoglobulin light chain, together with the first immunoglobulin heavy chain, forms an antigen-binding site that binds NKG2D. The second immunoglobulin heavy chain comprises a second Fc (hinge-CH2-CH3) domain, a second heavy chain variable domain and optionally a second CH1 heavy chain domain. The second immunoglobulin light chain includes a second light chain variable domain and a second light chain constant domain. The second immunoglobulin light chain, together with the second immunoglobulin heavy chain, forms an antigen-binding site that binds CAIX, ANO1, mesothelin, TROP2, CEA or Claudin-18.2. The first Fc domain and second Fc domain together are able to bind to CD16 (FIG. 1). In some embodiments, the first immunoglobulin light chain is identical to the second immunoglobulin light chain.

Another exemplary format involves a heterodimeric, multi-specific antibody including a first immunoglobulin heavy chain, a second immunoglobulin heavy chain and an immunoglobulin light chain (FIG. 2). The first immunoglobulin heavy chain includes a first Fc (hinge-CH2-CH3) domain fused via either a linker or an antibody hinge to a single-chain variable fragment (scFv) composed of a heavy chain variable domain and light chain variable domain which pair and bind NKG2D, or bind one of the tumor associated antigen selected from CAIX, ANO1, mesothelin, TROP2, CEA and Claudin-18.2. The second immunoglobulin heavy chain includes a second Fc (hinge-CH2-CH3) domain, a second heavy chain variable domain and optionally a CH1 heavy chain domain. The immunoglobulin light chain includes a light chain variable domain and a light chain constant domain. The second immunoglobulin heavy chain pairs with the immunoglobulin light chain and binds to NKG2D or binds one of the tumor associated antigen selected from CAIX, ANO1, mesothelin, TROP2, CEA and Claudin-18.2. The first Fc domain and the second Fc domain together are able to bind to CD16 (FIG. 2).

One or more additional binding motifs may be fused to the C-terminus of the constant region CH3 domain, optionally via a linker sequence. In certain embodiments, the antigen-binding site could be a single-chain or disulfide-stabilized variable region (scFv) or could form a tetravalent or trivalent molecule.

In some embodiments, the multi-specific binding protein is in the Triomab form, which is a trifunctional, bispecific antibody that maintains an IgG-like shape. This chimera consists of two half antibodies, each with one light and one heavy chain, that originate from two parental antibodies.

In some embodiments, the multi-specific binding protein is the KiH Common Light Chain (LC) form, which involves the knobs-into-holes (KIHs) technology. The KIH involves engineering C_{H}3 domains to create either a "knob" or a "hole" in each heavy chain to promote heterodimerization. The concept behind the "Knobs-into-Holes (KiH)" Fc technology was to introduce a "knob" in one CH3 domain (CH3A) by substitution of a small residue with a bulky one (*e.g*., T366W_{CH3A} in EU numbering). To accommodate the "knob," a complementary "hole" surface was created on the other CH3 domain (CH3B) by replacing the closest neighboring residues to the knob with smaller ones (*e.g*., T366S/L368A/Y407V_{CH3B}). The "hole" mutation was optimized by structured-guided phage library screening (Atwell S, Ridgway JB, Wells JA, Carter P., Stable heterodimers from remodeling the domain interface of a homodimer using a phage display library, J. Mol. Biol. (1997) 270(1):26-35). X-ray crystal structures of KiH Fc variants (Elliott JM, Ultsch M, Lee J, Tong R, Takeda K, Spiess C, et al., Antiparallel conformation of knob and hole aglycosylated half-antibody homodimers is mediated by a CH2-CH3 hydrophobic interaction. J. Mol. Biol. (2014) 426(9):1947-57; Mimoto F, Kadono S, Katada H, Igawa T, Kamikawa T, Hattori K. Crystal structure of a novel asymmetrically engineered Fc variant with improved affinity for FcgammaRs. Mol. Immunol. (2014) 58(1):132-8) demonstrated that heterodimerization is thermodynamically favored by hydrophobic interactions driven by steric complementarity at the inter-CH3 domain core interface, whereas the knob-knob and the hole-hole interfaces do not favor homodimerization owing to steric hindrance and disruption of the favorable interactions, respectively.

In some embodiments, the multi-specific binding protein is in the dual-variable domain immunoglobulin (DVD-Ig™) form, which combines the target binding domains of two monoclonal antibodies via flexible naturally occurring linkers, and yields a tetravalent IgG-like molecule.

In some embodiments, the multi-specific binding protein is in the Orthogonal Fab interface (Ortho-Fab) form. In the ortho-Fab IgG approach (Lewis SM, Wu X, Pustilnik A, Sereno A, Huang F, Rick HL, et al., Generation of bispecific IgG antibodies by structure-based design of an orthogonal Fab interface. Nat. Biotechnol. (2014) 32(2):191-8), structure-based regional design introduces complementary mutations at the LC and HC_{VH-CH1} interface in only one Fab, without any changes being made to the other Fab.

In some embodiments, the multi-specific binding protein is in the 2-in-1 Ig format. In some embodiments, the multi-specific binding protein is in the ES form, which is a heterodimeric construct containing two different Fabs binding to targets 1 and target 2 fused to the Fc. Heterodimerization is ensured by electrostatic steering mutations in the Fc.

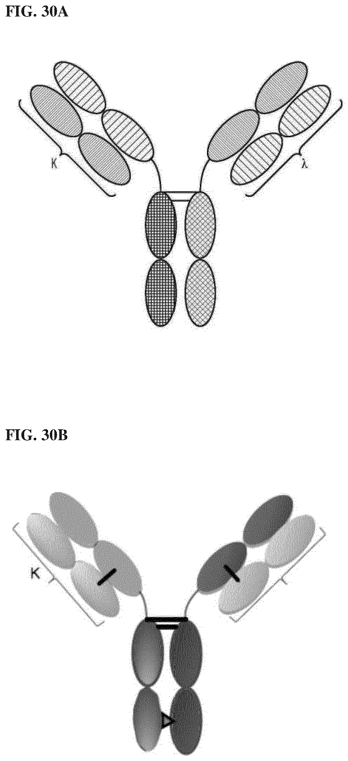

In some embodiments, the multi-specific binding protein is in the κλ-Body form, which is a heterodimeric construct with two different Fabs fused to Fc stabilized by heterodimerization mutations: Fab1 targeting antigen 1 contains kappa LC, while second Fab targeting antigen 2 contains lambda LC. FIG. 30A is an exemplary representation of one form of a κλ-Body; FIG. 30B is an exemplary representation of another κλ-Body.

In some embodiments, the multi-specific binding protein is in Fab Arm Exchange form (antibodies that exchange Fab arms by swapping a heavy chain and attached light chain (half-molecule) with a heavy-light chain pair from another molecule, which results in bispecific antibodies).

In some embodiments, the multi-specific binding protein is in the SEED Body form. The strand-exchange engineered domain (SEED) platform was designed to generate asymmetric and bispecific antibody-like molecules, a capability that expands therapeutic applications of natural antibodies. This protein engineered platform is based on exchanging structurally related sequences of immunoglobulin within the conserved CH3 domains. The SEED design allows efficient generation of AG/GA heterodimers, while disfavoring homodimerization of AG and GA SEED CH3 domains. (Muda M. et al., Protein Eng. Des. Sel. (2011, 24(5):447-54)).

In some embodiments, the multi-specific binding protein is in the LuZ-Y form, in which a leucine zipper is used to induce heterodimerization of two different HCs. (Wranik, BJ. et al., J. Biol. Chem. (2012), 287:43331-9).

In some embodiments, the multi-specific binding protein is in the Cov-X-Body form. In bispecific CovX-Bodies, two different peptides are joined together using a branched azetidinone linker and fused to the scaffold antibody under mild conditions in a site-specific manner. Whereas the pharmacophores are responsible for functional activities, the antibody scaffold imparts long half-life and Ig-like distribution. The pharmacophores can be chemically optimized or replaced with other pharmacophores to generate optimized or unique bispecific antibodies. (Doppalapudi VR et al., PNAS (2010), 107(52);22611-22616).

In some embodiments, the multi-specific binding protein is in an Oasc-Fab heterodimeric form that includes Fab binding to target 1, and scFab binding to target 2 fused to Fc. Heterodimerization is ensured by mutations in the Fc.

In some embodiments, the multi-specific binding protein is in a DuetMab form, which is an heterodimeric construct containing two different Fabs binding to antigens 1 and 2, and Fc stabilized by heterodimerization mutations. Fab 1 and 2 contain differential S-S bridges that ensure correct LC and HC pairing.

In some embodiments, the multi-specific binding protein is in a CrossmAb form, which is an heterodimeric construct with two different Fabs binding to targets 1 and 2, fused to Fc stabilized by heterodimerization. CL and CH1 domains and VH and VL domains are switched, *e.g*., CH1 is fused in-line with VL, while CL is fused in-line with VH.

In some embodiments, the multi-specific binding protein is in a Fit-Ig form, which is a homodimeric construct where Fab binding to antigen 2 is fused to the N terminus of HC of Fab that binds to antigen 1. The construct contains wild-type Fc.

Table 1 lists peptide sequences of heavy chain variable domains and light chain variable domains that, in combination, can bind to NKG2D. The NKG2D-binding domains can vary in their binding affinities to NKG2D, nevertheless, they all activate human NKG2D and NK cells.

| Table 1 | | |
|---|---|---|
| Clones | Heavy chain variable region amino acid sequence | Light chain variable region amino acid sequence |
| ADI-27705 | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS (SEQ ID NO:1) | DIQMTQSPSTLSASVGDRVTITCR ASQSISSWLAWYQQKPGKAPKLL IYKASSLESGVPSRFSGSGSGTEFT LTISSLQPDDFATYYCQQYNSYPI TFGGGTKVEIK (SEQ ID NO:2) |
| | CDR1 (SEQ ID NO:51) - | |
| | GSFSGYYWS CDR2 (SEQ ID NO:52) - | |
| | EIDHSGSTNYNPSLKS CDR3 (SEQ ID NO:53) - ARARGPWSFDP | |
| ADI-27724 | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS (SEQ ID NO:3) | EIVLTQSPGTLSLSPGERATLSCRA SQSVSSSYLAWYQQKPGQAPRLL IYGASSRATGIPDRFSGSGSGTDFT LTISRLEPEDFAVYYCQQYGSSPIT FGGGTKVEIK (SEQ ID NO:4) |
| ADI-27740 | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS (SEQ ID NO:5) | DIQMTQSPSTLSASVGDRVTITCR ASQSIGSWLAWYQQKPGKAPKLL IYKASSLESGVPSRFSGSGSGTEFT LTISSLQPDDFATYYCQQYHSFYT FGGGTKVEIK (SEQ ID NO:6) |
| ADI-27741 | QVQLQQWGAGLLKPSETLSLTCAV YGGSFSGYYWSWIRQPPGKGLEWI GEIDHSGSTNYNPSLKSRVTISVDTS | DIQMTQSPSTLSASVGDRVTITCR ASQSIGSWLAWYQQKPGKAPKLL IYKASSLESGVPSRFSGSGSGTEFT |
| | KNQFSLKLSSVTAADTAVYYCARA RGPWSFDPWGQGTLVTVSS (SEQ ID NO:7) | LTISSLQPDDFATYYCQQSNSYYT FGGGTKVEIK (SEQ ID NO:8) |
| ADI-27743 | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS (SEQ ID NO:9) | DIQMTQSPSTLSASVGDRVTITCR ASQSISSWLAWYQQKPGKAPKLL IYKASSLESGVPSRFSGSGSGTEFT LTISSLQPDDFATYYCQQYNSYPT FGGGTKVEIK (SEQ ID NO:10) |
| ADI-28153 | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWGFDPWGQGTLVTVSS (SEQ ID NO:11) | ELQMTQSPSSLSASVGDRVTITCR TSQSISSYLNWYQQKPGQPPKLLI YWASTRESGVPDRFSGSGSGTDF TLTISSLQPEDSATYYCQQSYDIPY TFGQGTKLEIK (SEQ ID NO:12) |
| ADI-28226 | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS (SEQ ID NO:13) | DIQMTQSPSTLSASVGDRVTITCR ASQSISSWLAWYQQKPGKAPKLL IYKASSLESGVPSRFSGSGSGTEFT LTISSLQPDDFATYYCQQYGSFPIT FGGGTKVEIK (SEQ ID NO:14) |
| ADI-28154 | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS (SEQ ID NO:15) | DIQMTQSPSTLSASVGDRVTITCR ASQSISSWLAWYQQKPGKAPKLL IYKASSLESGVPSRFSGSGSGTDFT LTISSLQPDDFATYYCQQSKEVPW TFGQGTKVEIK (SEQ ID NO:16) |
| ADI-29399 | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS (SEQ ID NO:17) | DIQMTQSPSTLSASVGDRVTITCR ASQSISSWLAWYQQKPGKAPKLL IYKASSLESGVPSRFSGSGSGTEFT LTISSLQPDDFATYYCQQYNSFPT FGGGTKVEIK (SEQ ID NO:18) |
| ADI-29401 | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS (SEQ ID NO:19) | DIQMTQSPSTLSASVGDRVTITCR ASQSIGSWLAWYQQKPGKAPKLL IYKASSLESGVPSRFSGSGSGTEFT LTISSLQPDDFATYYCQQYDIYPT FGGGTKVEIK (SEQ ID NO:20) |
| ADI-29403 | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS (SEQ ID NO:21) | DIQMTQSPSTLSASVGDRVTITCR ASQSISSWLAWYQQKPGKAPKLL IYKASSLESGVPSRFSGSGSGTEFT LTISSLQPDDFATYYCQQYDSYPT FGGGTKVEIK (SEQ ID NO:22) |
| ADI-29405 | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS (SEQ ID NO:23) | DIQMTQSPSTLSASVGDRVTITCR ASQSISSWLAWYQQKPGKAPKLL IYKASSLESGVPSRFSGSGSGTEFT LTISSLQPDDFATYYCQQYGSFPT FGGGTKVEIK (SEQ ID NO:24) |
| ADI-29407 | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS (SEQ ID NO:25) | DIQMTQSPSTLSASVGDRVTITCR ASQSISSWLAWYQQKPGKAPKLL IYKASSLESGVPSRFSGSGSGTEFT LTISSLQPDDFATYYCQQYQSFPT FGGGTKVEIK (SEQ ID NO:26) |
| ADI-29419 | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS (SEQ ID NO:27) | DIQMTQSPSTLSASVGDRVTITCR ASQSISSWLAWYQQKPGKAPKLL IYKASSLESGVPSRFSGSGSGTEFT LTISSLQPDDFATYYCQQYSSFSTF GGGTKVEIK (SEQ ID NO:28) |
| ADI-29421 | QVQLQQWGAGLLKPSETLSLTCAV YGGSFSGYYWSWIRQPPGKGLEWI GEIDHSGSTNYNPSLKSRVTISVDTS | DIQMTQSPSTLSASVGDRVTITCR ASQSISSWLAWYQQKPGKAPKLL IYKASSLESGVPSRFSGSGSGTEFT |
| | KNQFSLKLSSVTAADTAVYYCARA RGPWSFDPWGQGTLVTVSS (SEQ ID NO:29) | LTISSLQPDDFATYYCQQYESYST FGGGTKVEIK (SEQ ID NO:30) |
| ADI-29424 | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS (SEQ ID NO:31) | DIQMTQSPSTLSASVGDRVTITCR ASQSISSWLAWYQQKPGKAPKLL IYKASSLESGVPSRFSGSGSGTEFT LTISSLQPDDFATYYCQQYDSFITF GGGTKVEIK (SEQ ID NO:32) |
| ADI-29425 | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS (SEQ ID NO:33) | DIQMTQSPSTLSASVGDRVTITCR ASQSISSWLAWYQQKPGKAPKLL IYKASSLESGVPSRFSGSGSGTEFT LTISSLQPDDFATYYCQQYQSYPT FGGGTKVEIK (SEQ ID NO:34) |
| ADI-29426 | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS (SEQ ID NO:35) | DIQMTQSPSTLSASVGDRVTITCR ASQSIGSWLAWYQQKPGKAPKLL IYKASSLESGVPSRFSGSGSGTEFT LTISSLQPDDFATYYCQQYHSFPT FGGGTKVEIK (SEQ ID NO:36) |
| ADI-29429 | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS (SEQ ID NO:37) | DIQMTQSPSTLSASVGDRVTITCR ASQSIGSWLAWYQQKPGKAPKLL IYKASSLESGVPSRFSGSGSGTEFT LTISSLQPDDFATYYCQQYELYSY TFGGGTKVEIK (SEQ ID NO:38) |
| ADI-29447 | QVQLQQWGAGLLKPSETLSLTCAV YGGSFSGYYWSWIRQPPGKGLEWI GEIDHSGSTNYNPSLKSRVTISVDTS KNQFSLKLSSVTAADTAVYYCARA RGPWSFDPWGQGTLVTVSS (SEQ ID NO:39) | DIQMTQSPSTLSASVGDRVTITCR ASQSISSWLAWYQQKPGKAPKLL IYKASSLESGVPSRFSGSGSGTEFT LTISSLQPDDFATYYCQQYDTFITF GGGTKVEIK (SEQ ID NO:40) |
| ADI-27727 | QVQLVQSGAEVKKPGSSVKVSCKA SGGTFSSYAISWVRQAPGQGLEWM GGIIPIFGTANYAQKFQGRVTITADE STSTAYMELSSLRSEDTAVYYCAR GDSSIRHAYYYYGMDVWGQGTTV TVSS (SEQ ID NO:41) | DIVMTQSPDSLAVSLGERATINCK SSQSVLYSSNNKNYLAWYQQKP GQPPKLLIYWASTRESGVPDRFSG SGSGTDFTLTISSLQAEDVAVYYC QQYYSTPITFGGGTKVEIK (SEQ ID NO:42) |
| | CDR1 (SEQ ID NO:54) - | CDR1 (SEQ ID NO:57) - |
| | GTFSSYAISCDR2 (SEQ ID NO:55) - | KSSQSVLYSSNNKNYLA CDR2 (SEQ IDNO:58) |
| | GIIPIFGTANYAQKFQGCDR3 (SEQ ID NO:56) - ARGDSSIRHAYYYYGMDV | WASTRES CDR3 (SEQ IDNO:59)-QQYYSTPIT |
| ADI-29443 | QLQLQESGPGLVKPSETLSLTCTVS GGSISSSSYYWGWIRQPPGKGLEWI GSIYYSGSTYYNPSLKSRVTISVDTS KNQFSLKLSSVTAADTAVYYCARG SDRFHPYFDYWGQGTLVTVSS (SEQ ID NO:43) | EIVLTQSPATLSLSPGERATLSCRA SQSVSRYLAWYQQKPGQAPRLLI YDASNRATGIPARFSGSGSGTDFT LTISSLEPEDFAVYYCQQFDTWPP TFGGGTKVEIK (SEQ ID NO:44) |
| | CDR1 (SEQ ID NO:60) - GSISSSSYYWG | CDR1 (SEQ ID NO:63) - RASQSVSRYLA |
| | CDR2 (SEQ ID NO:61) - SIYYSGSTYYNPSLKS | CDR2 (SEQ ID NO:64) - DASNRAT |
| | CDR3 (SEQ ID NO:62) - ARGSDRFHPYFDY | CDR3 (SEQ ID NO:65) - QQFDTWPPT |
| ADI-28200 | QVQLVQSGAEVKKPGSSVKVSCKA SGGTFSSYAISWVRQAPGQGLEWM GGIIPIFGTANYAQKFQGRVTITADE STSTAYMELSSLRSEDTAVYYCAR RGRKASGSFYYYYGMDVWGQGTT VTVSS |  (SEQ ID NO:46) |
| | (SEQ ID NO:45) | CDR1 (SEQ ID NO:69) - ESSQSLLNSGNQKNYLT |
| | CDR1 (SEQ ID NO:66) - GTFSSYAIS | CDR2 (SEQ IDNO:70)-WASTRES |
| | CDR2 (SEQ ID N0:67) - GIIPIFGTANYAQKFQG | CDR3 (SEQ ID NO:71) - QNDYSYPYT |
| | CDR3 (SEQ ID NO:68) - ARRGRKASGSFYYYYGMDV | |
| ADI-27744 (A44) | EVQLLESGGGLVQPGGSLRLSCAA SGFTFSSYAMSWVRQAPGKGLEW VSAISGSGGSTYYADSVKGRFTISR DNSKNTLYLQMNSLRAEDTAVYY CAKDGGYYDSGAGDYWGQGTLV TVSS (SEQ ID NO:132) | DIQMTQSPSSVSASVGDRVTITCR ASQGIDSWLAWYQQKPGKAPKL LIYAASSLQSGVPSRFSGSGSGTD FTLTISSLQPEDFATYYCQQGVSY PRTFGGGTKVEIK (SEQ ID NO:133) |
| | CDR1 (SEQ ID NO:134) - FTFSSYAMS | CDR1 (SEQ ID NO : 137 - RASQGIDSWLA |
| | CDR2 (SEQ ID NO:135) - AISGSGGSTYYADSVKG | AASSLQS CDR3 (SEQ ID NO:139) -QQGVSYPRT |
| | CDR3 (SEQ ID NO:136) - AKDGGYYDSGAGDY | |
| ADI-27749 (A49) | EVQLVESGGGLVKPGGSLRLSCAA SGFTFSSYSMNWVRQAPGKGLEW VSSISSSSSYIYYADSVKGRFTISRD NAKNSLYLQMNSLRAEDTAVYYC ARGAPMGAAAGWFDPWGQGTLV TVSS (SEQ ID NO:140) | DIQMTQSPSSVSASVGDRVTITCR ASQGISSWLAWYQQKPGKAPKLL IYAASSLQSGVPSRFSGSGSGTDF TLTISSLQPEDFATYYCQQGVSFP RTFGGGTKVEIK (SEQ ID NO:141) CDR1 (SEQ ID NO:145)-RASQGISSWLA |
| | CDR1 (SEQ ID NO:142) - FTFSSYSMN | CDR2 (SEQ ID NO:146) - AASSLQS |
| | CDR2 (SEQ ID NO:143) - SISSSSSYIYYADSVKG | CDR3 (SEQ ID NO:147) - QQGVSFPRT |
| | CDR3 (SEQ ID NO:144) - ARGAPMGAAAGWFDP | |
| ADI-29463 (F63) | QVQLVQSGAEVKKPGASVKVSCK ASGYTFTGYYMHWVRQAPGQGLE WMGWINPNSGGTNYAQKFQGRVT MTRDTSISTAYMELSRLRSDDTAV YYCARDTGEYYDTDDHGMDVWG QGTTVTVSS (SEQ ID NO:148) | EIVLTQSPGTLSLSPGERATLSCRA SQSVSSNLAWYQQKPGQAPRLLI YGASTRATGIPARFSGSGSGTEFT LTISSLQSEDFAVYYCQQDDYWP PTFGGGTKVEIK (SEQ ID NO:149) CDR1 (SEQ ID NO:153) - |
| | CDR1 (SEQ ID NO:150) - YTFTGYYMH | RASQSVSSNLA CDR2 (SEQ ID NO:154) - GASTRAT |
| | CDR2 (SEQ ID NO:151) - WINPNSGGTNYAQKFQG | CDR3 (SEQ ID NO:155) - QQDDYWPPT |
| | CDR3 (SEQ ID NO:152) - ARDTGEYYDTDDHGMDV | |
| ADI-29379 (E79) | QVQLVQSGAEVKKPGASVKVSCK ASGYTFTSYYMHWVRQAPGQGLE WMGIINPSGGSTSYAQKFQGRVTM TRDTSTSTVYMELSSLRSEDTAVY YCARGAPNYGDTTHDYYYMDVW GKGTTVTVSS (SEQ ID NO:156) | EIVMTQSPATLSVSPGERATLSCR ASQSVSSNLAWYQQKPGQAPRLL IYGASTRATGIPARFSGSGSGTEFT LTISSLQSEDFAVYYCQQYDDWP FTFGGGTKVEIK (SEQ ID NO:157) |
| | CDR1 (SEQ ID NO:158) - YTFTSYYMH | CDR1 (SEQ ID NO:161)-RASQSVSSNLA CDR2 (SEQ ID NO:162) - GASTRAT |
| | CDR2 (SEQ ID NO:159) - IINPSGGSTSYAQKFQG | CDR3 (SEQ ID NO:163) - QQYDDWPFT |
| | CDR3 (SEQ ID NO:160) - ARGAPNYGDTTHDYYYMDV | |
| ADI-29447 | QVQLQQWGAGLLKPSETLSLTCAV YGGSFSGYYWSWIRQPPGKGLEWI | DIQMTQSPSTLSASVGDRVTITCR ASQSISSWLAWYQQKPGKAPKLL |
| (F47) | GEIDHSGSTNYNPSLKSRVTISVDTS KNQFSLKLSSVTAADTAVYYCARA RGPWSFDPWGQGTLVTVSS (SEQ ID NO:164) | IYKASSLESGVPSRFSGSGSGTEFT LTISSLQPDDFATYYCQQYDTFITF GGGTKVEIK (SEQ ID NO:165) |
| | CDR1 (SEQ ID NO:166) - GSFSGYYWS | CDR1 (SEQ ID NO:169) - RASQSISSWLA |
| | CDR2 (SEQ ID NO:167) - EIDHSGSTNYNPSLKS | CDR2 (SEQ ID NO:170) - KASSLES |
| | CDR3 (SEQ ID NO:168) - ARARGPWSFDP | CDR3 (SEQ ID NO:171) - QQYDTFIT |

Alternatively, a heavy chain variable domain defined by SEQ ID NO:47 can be paired with a light chain variable domain defined by SEQ ID NO:48 to form an antigen-binding site that can bind to NKG2D, as illustrated in US 9,273,136.

SEQ ID NO:47
QVQLVESGGGLVKPGGSLRLSCAASGFTFSSYGMHWVRQAPGKGLEWVAFIRYDGSN
KYYADSVKGRFTISRDNSKNTLYLQMNSLRAEDTAVYYCAKDRGLGDGTYFDYWGQG
TTVTVSS

SEQ ID NO:48
QSALTQPASVSGSPGQSITISCSGSSSNIGNNAVNWYQQLPGKAPKLLIYYDDLLPSGVS
DRFSGSKSGTSAFLAISGLQSEDEADYYCAAWDDSLNGPVFGGGTKLTVL

Alternatively, a heavy chain variable domain defined by SEQ ID NO:49 can be paired with a light chain variable domain defined by SEQ ID NO:50 to form an antigen-binding site that can bind to NKG2D, as illustrated in US 7,879,985.

SEQ ID NO:49
QVHLQESGPGLVKPSETLSLTCTVSDDSISSYYWSWIRQPPGKGLEWIGHISYSGSANYN
PSLKSRVTISVDTSKNQFSLKLSSVTAADTAVYYCANWDDAFNIWGQGTMVTVSS

SEQ ID NO:50
EIVLTQSPGTLSLSPGERATLSCRASQSVSSSYLAWYQQKPGQAPRLLIYGASSRATGIPD
RFSGSGSGTDFTLTISRLEPEDFAVYYCQQYGSSPWTFGQGTKVEIK

Table 2 lists peptide sequences of heavy chain variable domains and light chain variable domains that, in combination, can bind to CAIX.

| Table 2 | | |
|---|---|---|
| Clones | Heavy chain variable domain amino acid sequence | Light chain variable domain amino acid sequence |
| Girentuximab (PCT/EP2002/ 001283) | DVKLVESGGGLVKLGGSLKLSC AASGFTFSNYYMSWVRQTPEKR LELVAAINSDGGITYYLDTVKG RFTISRDNAKNTLYLQMSSLKSE DTALFYCARHRSGYFSMDYWG QGTSVTVSS (SEQ ID NO:72) | DIVMTQSQRFMSTTVGDRVSIT CKASQNVVSAVAWYQQKPGQS PKLLIYSASNRYTGVPDRFTGSG SGTDFTLTISNMQSEDLADFFCQ QYSNYPWTFGGGTKLEIKR (SEQ ID NO:73) |
| | CDR1 (SEQ ID NO:74) - GFTFSNY | CDR1(SEQ ID NO:77) - QNVVSAVA |
| | CDR2 (SEQ ID NO:75) - NSDGGI | CDR2 (SEQ ID NO:78) -SASNRYT |
| | CDR3 (SEQ ID NO:76) - HRSGYFSMDY | CDR3 (SEQ ID NO:79) - QQYSNYPWT |
| BAY79-4620 (US 8,168,758) | QVELVESGGGLVQPGGSLRLSC AASGFTFSSYGMSWVRQAPGK GLEWVSGISSLGSTTYYADSVK GRFTISRDNSKNTLYLQMNSLR AEDTAVYYCARTGSPGTFMHG DHWGQGTLVTVSS (SEQ ID NO:80) | DIQMTQSPSSLSASVGDRVTITC RASQDINNYLSWYQQKPGKAP KLLIYGASNLQSGVPSRFSGSGS GTDFTLTISSLQPEDFAVYYCQQ YYGRPTTFGQGTKVEIK (SEQ ID NO:81) |
| | CDR1 (SEQ ID NO:82) - GFTFSSYGMS | CDR1 (SEQ ID NO:85) - RASQDINNYLS |
| | CDR2 (SEQ ID NO:83) - GISSLGSTTYYADSVKG | CDR2 (SEQ ID NO:86) - YGASNLQS |
| | CDR3 (SEQ ID NO:84) - TGSPGTFMHGDH | CDR3 (SEQ ID NO:87) - QQYYGRPT |
| Anti-CAIX | EVQLVESGGGLVQPGGSLRLSC AASGFTFSNYYMSWVRQAPGQ | DIVMTQSPSSLSASVGDRVTITC RASQNVVSAVAWYLQKPGKSP |
| (US 12/016,884) | RLELVSAINSDGGITYYLDSVKG RFTISRDNAKNTLYLQMGSLRA EDMAVYYCARHRSGYFSMDY WGQGTLVTVSS (SEQ ID NO:88) | KLLIYSASNRYTGVPDRFTGSGS GTDFTLTISSLQPEDFATYFCQQ YSNYPWTFGGGTKLEIK (SEQ ID NO:89) |
| | CDR1 (SEQ ID NO:90) - GFTFSNY | CDR1 (SEQ ID NO:93) - QNVVSAVA |
| | CDR2 (SEQ ID NO:91) - NSDGGI | CDR2 (SEQ ID NO:94) - SASNRYT |
| | CDR3 (SEQ ID NO:92) - HRSGYFSMDY | CDR3 (SEQ ID NO:95) - QQYSNYPWT |

Alternatively, novel antigen-binding sites that bind to CAIX can be identified by screening for binding to the amino acid sequence defined by SEQ ID NO:96.

SEQ ID NO:96
MAPLCPSPWLPLLIPAPAPGLTVQLLLSLLLLVPVHPQRLPRMQEDSPLGGGSSGEDDPL
GEEDLPSEEDSPREEDPPGEEDLPGEEDLPGEEDLPEVKPKSEEEGSLKLEDLPTVEAPGD
PQEPQNNAHRDKEGDDQSHWRYGGDPPWPRVSPACAGRFQSPVDIRPQLAAFCPALRP
LELLGFQLPPLPELRLRNNGHSVQLTLPPGLEMALGPGREYRALQLHLHWGAAGRPGSE
HTVEGHRFPAEIHVVHLSTAFARVDEALGRPGGLAVLAAFLEEGPEENSAYEQLLSRLE
EIAEEGSETQVPGLDISALLPSDFSRYFQYEGSLTTPPCAQGVIWTVFNQTVMLSAKQLH
TLSDTLWGPGDSRLQLNFRATQPLNGRVIEASFPAGVDSSPRAAEPVQLNSCLAAGDILA
LVFGLLFAVTSVAFLVQMRRQHRRGTKGGVSYRPAEVAETGA

Table 3 lists peptide sequences of heavy chain variable domains and light chain variable domains that, in combination, can bind to ANO1.

| Table 3 | | |
|---|---|---|
| Clones | Heavy chain variable domain amino acid sequence | Light chain variable domain amino acid sequence |
| ANO1 antibody (PCT/US20 12/067430) | QVQLKESGPGLVQPSQTLTLTCTV SGFSLSNYDMHWVRQSPGKGLD WMGVIWGNGKTQYNSGLTSRLSI SRDTSKSQVFLKMNSLQTEDTAIY | DVVLTQTPPTLSATIGQSVSISCR SSQSLLHSNGNTYLNWLLQRPG QPPQLLIYLVSRLESGVPNRFSGS GSGTDFTLKISGVEAEDLGVYYC |
| | FCTRSGYYYDGSYYSLFDYWGQG VMVTVSS (SEQ ID NO:97) | VQSTHAPAFGGGTKLELK (SEQ ID NO:98) |
| | CDR1 (SEQ ID NO:99) - NYDMH | CDR1(SEQ ID NO:102) - RSSQSLLHSNGNTYLN |
| | CDR2 (SEQ ID NO:100) - VIWGNGKTQYNSGLTS | CDR2 (SEQ ID NO:103) - LVSRLES |
| | CDR3 (SEQ ID NO:101) - SGYYYDGSYYSLFDY | CDR3 (SEQ ID NO:104) - VQSTHAPA |

Alternatively, novel antigen-binding sites that bind to ANO1 can be identified by screening for binding to the amino acid sequence defined by SEQ ID NO:105.

SEQ ID NO:105
MRVNEKYSTLPAEDRSVHIINICAIEDIGYLPSEGTLLNSLSVDPDAECKYGLYFRDGRR
KVDYILVYHHKRPSGNRTLVRRVQHSDTPSGARSVKQDHPLPGKGASLDAGSGEPPMD
YHEDDKRFRREEYEGNLLEAGLELERDEDTKIHGVGFVKIHAPWNVLCREAEFLKLKM
PTKKMYHINETRGLLKKINSVLQKITDPIQPKVAEHRPQTMKRLSYPFSREKQHLFDLSD
KDSFFDSKTRSTIVYEILKRTTCTKAKYSMGITSLLANGVYAAAYPLHDGDYNGENVEF
NDRKLLYEEWARYGVFYKYQPIDLVRKYFGEKIGLYFAWLGVYTQMLIPASIVGIIVFL
YGCATMDENIPSMEMCDQRHNITMCPLCDKTCSYWKMSSACATARASHLFDNPATVFF
SVFMALWAATFMEHWKRKQMRLNYRWDLTGFEEEEEAVKDHPRAEYEARVLEKSLK
KESRNKEKRRHIPEESTNKWKQRVKTAMAGVKLTDKVKLTWRDRFPAYLTNLVSIIFMI
AVTFAIVLGVIIYRISMAAALAMNSSPSVRSNIRVTVTATAVIINLVVIILLDEVYGCIARW
LTKIEVPKTEKSFEERLIFKAFLLKFVNSYTPIFYVAFFKGRFVGRPGDYVYIFRSFRMEE
CAPGGCLMELCIQLSIIMLGKQLIQNNLFEIGIPKMKKLIRYLKLKQQSPPDHEECVKRKQ
RYEVDYNLEPFAGLTPEYMEMIIQFGFVTLFVASFPLAPLFALLNNIIEIRLDAKKFVTEL
RRPVAVRAKDIGIWYNILRGIGKLAVIINAFVISFTSDFIPRLVYLYMYSKNGTMHGFVN
HTLSSFNVSDFQNGTAPNDPLDLGYEVQICRYKDYREPPWSENKYDISKDFWAVLAAR
LAFVIVFQNLVMFMSDFVDWVIPDIPKDISQQIHKEKVLMVELFMREEQDKQQLLETW
MEKERQKDEPPCNHHNTKACPDSLGSPAPSHAYHGGVL

Table 4 lists peptide sequences of heavy chain variable domains and light chain variable domains that, in combination, can bind to mesothelin.

| Table 4 | | |
|---|---|---|
| Clones | Heavy chain variable domain amino acid sequence | Light chain variable domain amino acid sequence |
| Am atuximab (US 7,592,426) | QVQLQQSGPELEKPGASVKISCK ASGYSFTGYTMNWVKQSHGKSL EWIGLITPYNGASSYNQKFRGKA TLTVDKSSSTAYMDLLSLTSEDSA VYFCARGGYDGRGFDYWGSGTP VTVSSA (SEQ ID NO:106) | DIELTQSPAIMSASPGEKVTMTC SASSSVSYMHWYQQKSGTSPKR WIYDTSKLASGVPGRFSGSGSG NSYSLTISSVEAEDDATYYCQQ WSKHPLTFGSGTKVEIKR (SEQ ID NO:107) |
| | CDR1 (SEQ ID NO:108) - GYSFTGY | CDR1(SEQ ID NO:111) - SSVSYMH |
| | CDR2 (SEQ ID NO:109) - TPYNGA | CDR2 (SEQ ID NO:112) - DTSKLAS |
| | CDR3 (SEQ ID NO:110) - GGYDGRGFDY | CDR3 (SEQ ID NO:113) - QQWSKHPLT |
| Antimesothelin (US 8,425,904) | EVHLVESGGGLVQPGGSLRLSCA ASGFTFSRYWMSWVRQAQGKGL EWVASIKQAGSEKTYVDSVKGRF TISRDNAKNSLSLQMNSLRAEDT AVYYCAREGAYYYDSASYYPYY YYYSMDVWGQGTTVTVSS (SEQ ID NO:114) | EIVLTQSPGTLSLSPGERATLSCR ASQSVSSSYLAWYQQKPGQAPR LLIYGASSRATGIPDRFSGSGSGT DFTLTISRLEPEDFAVYYCQQYG SSQYTFGQGTKLEIK (SEQ ID NO:115) |
| | CDR1 (SEQ ID NO:116) - GFTFSRY | CDR1 (SEQ ID NO:119) - QSVSSSYLA |
| | CDR2 (SEQ ID NO:117) - KQAGSE | CDR2 (SEQ ID NO:120) - GASSRAT |
| | CDR3 (SEQ ID NO:118) - EGAYYYDSASYYPYYYYYSMDV | CDR3 (SEQ ID NO:121)-QQYGSSQYT |
| Anetumab (US 9,023,351) | QVELVQSGAEVKKPGESLKISCK GSGYSFTSYWIGWVRQAPGKGLE WMGIIDPGDSRTRYSPSFQGQVTI SADKSISTAYLQWSSLKASDTAM | DIALTQPASVSGSPGQSITISCTG TSSDIGGYNSVSWYQQHPGKAP KLMIYGVNNRPSGVSNRFSGSK |
| | YYCARGQLYGGTYMDGWGQGT LVTVSSA (SEQ ID NO:122) | SGNTASLTISGLQAEDEADYYCS SYDIESATPVFGGGTKLTVLG (SEQ ID NO:123) |
| | CDR1 (SEQ ID NO:124) - GYSFTSY | CDR1 (SEQ ID NO:127) - SSDIGGYNSVS |
| | CDR2 (SEQ ID NO:125) - DPGDSR | CDR2 (SEQ ID NO:128) - GVNNRPS |
| | CDR3 (SEQ ID NO:126) - GQLYGGTYMDG | CDR3 (SEQ ID NO:129) - SSYDIESATPV |

Alternatively, novel antigen-binding sites that bind to mesothelin can be identified by screening for binding to the amino acid sequence defined by SEQ ID NO:130.

SEQ ID NO:130
MALPTARPLLGSCGTPALGSLLFLLFSLGWVQPSRTLAGETGQEAAPLDGVLANPPNISS
LSPRQLLGFPCAEVSGLSTERVRELAVALAQKNVKLSTEQLRCLAHRLSEPPEDLDALPL
DLLLFLNPDAFSGPQACTRFFSRITKANVDLLPRGAPERQRLLPAALACWGVRGSLLSEA
DVRALGGLACDLPGRFVAESAEVLLPRLVSCPGPLDQDQQEAARAALQGGGPPYGPPS
TWSVSTMDALRGLLPVLGQPIIRSIPQGIVAAWRQRSSRDPSWRQPERTILRPRFRREVE
KTACPSGKKAREIDESLIFYKKWELEACVDAALLATQMDRVNAIPFTYEQLDVLKHKL
DELYPQGYPESVIQHLGYLFLKMSPEDIRKWNVTSLETLKALLEVNKGHEMSPQAPRRP
LPQVATLIDRFVKGRGQLDKDTLDTLTAFYPGYLCSLSPEELSSVPPSSIWAVRPQDLDT
CDPRQLDVLYPKARLAFQNMNGSEYFVKIQSFLGGAPTEDLKALSQQNVSMDLATFMK
LRTDAVLPLTVAEVQKLLGPHVEGLKAEERHRPVRDWILRQRQDDLDTLGLGLQGGIP
NGYLVLDLSMQEALSGTPCLLGPGPVLTVLALLLASTLA

Table 5 lists peptide sequences of heavy chain variable domains and light chain variable domains that, in combination, can bind to TROP2.

| Table 5 | | |
|---|---|---|
| Clones | Heavy chain variable domain amino acid sequence | Light chain variable domain amino acid sequence |
| IMMU-132 (US 7,238,785) | SVQLQQSGSELKKPGASVKVSCK ASGYTFTNYGMNWVKQAPGQGL KWMGWINTYTGEPTYTDDFKGR | DIQLTQSPSSLSASVGDRVSITCKA SQDVSIAVAWYQQKPGKAPKLLIY SASYRYTGVPDRFSGSGSGTDFTL |
| | FAFSLDTSVSTAYLQISSLKADDT AVYFCARGGFGSSYWYFDVWGQ GSLVTVSS (SEQ ID NO:172) | TISSLQPEDFAVYYCQQHYITPLTF GAGTKVEIKR (SEQ ID NO:173) |
| | CDR1 (SEQ ID NO:174) - GYTFTNY | CDR1(SEQ ID NO:177) - QDVSIAVA |
| | CDR2 (SEQ ID NO:175) - NTYTGE | CDR2 (SEQ ID NO: 178) - SASYRYT |
| | CDR3 (SEQ ID NO:176)-GGFGSSYWYFDV | CDR3 (SEQ ID NO: 179) - QQHYITPLT |
| Anti-TROP2 antibody (PCT/CA08/000 979) | QIQLVQSGHEVKKPGASVKVSCK ASGYTFTNYGMNWVRQAPGQGL EWMGWINTKTGEPTYAEEFKGRF VFSLETSASTAYLQISSLKAEDTA MYFCGRGGYGSSYWYFDVWGQ GTTVTVSS (SEQ ID NO:180) | DIQMTQSPSSLSASVGDRVTITCKA SQDVSIAVAWYQQKPGKAPKVLI YSASYRYTGVPDRFSGSGSGTDFT FTISSLQPEDIAVYYCQQHYITPLTF GGGTKVEIK (SEQ ID NO:181) |
| | CDR1 (SEQ ID NO:182) - GYTFTNY | CDR1 (SEQ ID NO:185) - QDVSIAVA |
| | CDR2 (SEQ ID NO:183) - NTKTGE | CDR2 (SEQ ID NO:186) - SASYRYT |
| | CDR3 (SEQ ID NO:184) - GGYGSSYWYFDV | CDR3 (SEQ ID NO:187) - QQHYITPLT |
| Anti-TROP2 antibody (US 15/187, 179) | QVQLVQSGAEVKKPGASVKVSC KASGYTFTTAGMQWVRQAPGQG LEWMGWINTHSGVPKYAEDFKG RVTISADTSTSTAYLQLSSLKSED TAVYYCARSGFGSSYWYFDVWG QGTLVTVSS (SEQ ID NO:188) | DIQMTQSPSSLSASVGDRVTITCKA SQDVSTAVAWYQQKPGKAPKLLI YSASYRYTGVPSRFSGSGSGTDFT LTISSLQPEDFAVYYCQQHYITPLT FGQGTKLEIKRT (SEQ ID NO:189) |
| | QVQLVQSGAEVKKPGASVKVSC KASGYTFTTAGMQWVRQAPGQG LEWMGWINTHSGVPKYAEDFKG RVTISLDTSTSTAYLQLSSLKSEDT | DIVMTQSPSSLSASVGDRVTITCKA SQDVSTAVAWYQQKPGKAPKLLI YSASYRYTGVPSRFSGSGSGTDFT LTISSLQPEDFAVYYCQQHYITPLT FGQGTKLEIKRT |
| | AVYYCARSGFGSSYWYFDVWGQ GTLVTVSS (SEQ ID NO:190) | (SEQ ID NO:191) |
| | QIQLVQSGAEVKKPGESVKVSCK ASGYTFTTAGMQWVQQMPGKGL EWMGWINTHSGVPKYAEDFKGR VTFSLDTSTSTAYLQLSSLKSEDT AVYYCARSGFGSSYWYFDVWGQ GTLVTVSS (SEQ ID NO:192) | DIVMTQSPSSLSASVGDRVTITCKA SQDVSTAVAWYQQKPGKQPKLLI YSASYRYTGVPSRFSGSGSGTDFT LTISSLQPEDFAVYYCQQHYITPLT FGQGTKLEIKR (SEQ ID NO:193) |

Alternatively, novel antigen-binding sites that bind to TROP2 can be identified by screening for binding to the amino acid sequence defined by SEQ ID NO:194.

SEQ ID NO:194
MARGPGLAPPPLRLPLLLLVLAAVTGHTAAQDNCTCPTNKMTVCSPDGPGGRCQCRAL
GSGMAVDCSTLTSKCLLLKARMSAPKNARTLVRPSEHALVDNDGLYDPDCDPEGRFKA
RQCNQTSVCWCVNSVGVRRTDKGDLSLRCDELVRTHHILIDLRHRPTAGAFNHSDLDA
ELRRLFRERYRLHPKFVAAVHYEQPTIQIELRQNTSQKAAGDVDIGDAAYYFERDIKGES
LFQGRGGLDLRVRGEPLQVERTLIYYLDEIPPKFSMKRLTAGLIAVIVVVVVALVAGMA
VLVITNRRKSGKYKKVEIKELGELRKEPSL

Table 6 lists peptide sequences of heavy chain variable domains and light chain variable domains that, in combination, can bind to CEA.

| Table 6 | | |
|---|---|---|
| Clones | Heavy chain variable domain amino acid sequence | Light chain variable domain amino acid sequence |
| labetuzumab | EVQLVESGGGVVQPGRSLRLSCSAS GFDFTTYWMSWVRQAPGKGLEWI GEIHPDSSTINYAPSLKDRFTISRDN AKNTLFLQMDSLRPEDTGVYFCAS LYFGFPWFAYWGQGTPVTVSS | DIQLTQSPSSLSASVGDRVTITCKA SQDVGTSVAWYQQKPGKAPKLLI YWTSTRHTGVPSRFSGSGSGTDFT FTISSLQPEDIATYYCQQYSLYRSF GQGTKVEIK |
| | (SEQ ID NO:195) | (SEQ ID NO:196) |
| | CDR1 (SEQ ID NO:197) - GFDFTTY | CDR1(SEQ ID N0:200) - QDVGTSVA |
| | CDR2 (SEQ ID NO:198) - HPDSST | CDR2 (SEQ ID N0:201) - WTSTRHT |
| | CDR3 (SEQ ID NO:199) - LYFGFPWFAY | CDR3 (SEQ ID NO:202) - QQYSLYRS |
| cergutuzumab | QVQLVQSGAEVKKPGASVKVSCK ASGYTFTEFGMNWVRQAPGQGLE WMGWINTKTGEATYVEEFKGRVT FTTDTSTSTAYMELRSLRSDDTAVY YCARWDFAYYVEAMDYWGQGTT VTVSSA (SEQ ID NO:203) | DIQMTQSPSSLSASVGDRVTITCKA SAAVGTYVAWYQQKPGKAPKLLI YSASYRKRGVPSRFSGSGSGTDFT LTISSLQPEDFATYYCHQYYTYPLF TFGQGTKLEIKR (SEQ ID NO:204) |
| | CDR1 (SEQ ID NO:205) - GYTFTEF | CDR1 (SEQ ID NO:208) - AAVGTYVA |
| | CDR2 (SEQ ID NO:206) - NTKTGE | CDR2 (SEQ ID NO:209) - SASYRKR |
| | CDR3 (SEQ ID NO:207) - WDFAYYVEAMDY | CDR3 (SEQ ID NO:210) - HQYYTYPLFT |
| anti-CEA (US 9,056,911) | QVQLQQSGAELVRSGASIKLSCTAS GFNIKHYYMHWVKQRPEQGLEWI GWINPENVDTEYAPKFQGKATMTA DTSSNTAYLQLSSLTSEDTAVYYCN HYRYAGGGALDYWGQGTTVTVSS A (SEQ ID NO:211) | DIQLTQSPAIMSASPGEKVTMTCS ASSSVSYIHWYQQKSGTSPKRWV YDTSKLASGVPARFSGSGSGTSYS LTISTMEAEDAATYYCQQWNNNP YSFGGGTKVVIKTV (SEQ ID NO:212) |
| | CDR1 (SEQ ID NO:213) - GFNIKHY | CDR1 (SEQ ID NO:216) - SSVSYIH |
| | CDR2 (SEQ ID NO:214) - NPENVD | CDR2 (SEQ ID NO:217) - DTSKLAS |
| | CDR3 (SEQ ID NO:215) - YRYAGGGALDY | CDR3 (SEQ ID NO:218) - QQWNNNPYS |

Alternatively, novel antigen-binding sites that bind to CEA can be identified by screening for binding to the amino acid sequence defined by SEQ ID NO:219.

SEQ ID NO:219

MESPSAPPHRWCIPWQRLLLTASLLTFWNPPTTAKLTIESTPFNVAEGKEVLLLVHNLPQ
HLFGYSWYKGERVDGNRQIIGYVIGTQQATPGPAYSGREIIYPNASLLIQNIIQNDTGFYT
LHVIKSDLVNEEATGQFRVYPELPKPSISSNNSKPVEDKDAVAFTCEPETQDATYLWWV
NNQSLPVSPRLQLSNGNRTLTLFNVTRNDTASYKCETQNPVSARRSDSVILNVLYGPDA
PTISPLNTSYRSGENLNLSCHAASNPPAQYSWFVNGTFQQSTQELFIPNITVNNSGSYTCQ
AHNSDTGLNRTTVTTITVYAEPPKPFITSNNSNPVEDEDAVALTCEPEIQNTTYLWWVN
NQSLPVSPRLQLSNDNRTLTLLSVTRNDVGPYECGIQNKLSVDHSDPVILNVLYGPDDPT
ISPSYTYYRPGVNLSLSCHAASNPPAQYSWLIDGNIQQHTQELFISNITEKNSGLYTCQAN
NSASGHSRTTVKTITVSAELPKPSISSNNSKPVEDKDAVAFTCEPEAQNTTYLWWVNGQ
SLPVSPRLQLSNGNRTLTLFNVTRNDARAYVCGIQNSVSANRSDPVTLDVLYGPDTPIISP
PDSSYLSGANLNLSCHSASNPSPQYSWRINGIPQQHTQVLFIAKITPNNNGTYACFVSNL
ATGRNNSIVKSITVSASGTSPGLSAGATVGIMIGVLVGVALI

Table 7 lists peptide sequences of heavy chain variable domains and light chain variable domains that, in combination, can bind to Claudin-18.2.

| Table 7 | | |
|---|---|---|
| Clones | Heavy chain variable domain amino acid sequence | Light chain variable domain amino acid sequence |
| 1 (PCT/EP2015/ 058206) | QIQLVQSGPELKKPGETVKISCKAS GYTFTNYGMNWVKQAPGKGLKW MGWINTNTGEPTY AEEFKGRFAFSLETSASTAYLQINN LKNEDTATYFCARLGFGNAMDYW GQGTSVTVSS (SEQ ID NO:220) | DIVMTQSPSSLTVTAGEKVTMSCK SSQSLLNSGNQKNYLTWYQQKPG QPPKLLIYWASTR ESGVPDRFTGSGSGTDFTLTISSVQ AEDLAVYYCQNDYSYPLTFGAGT KLELK (SEQ ID NO:221) |
| | CDR1 (SEQ ID NO:222) - GYTFTNYG | CDR1(SEQ ID NO:225) - QSLLNSGNQKNY |
| | CDR2 (SEQ ID NO:223) - INTNTGEP | CDR2 (SEQ ID NO:226) - WAS |
| | CDR3 (SEQ ID NO:224) - ARLGFGNAMDY | CDR3 (SEQ ID NO:227) - QNDYSYPLT |
| 2 (PCT/EP2015/ 058206) | QVQLQQPGAELVRPGASVKLSCKA SGYTFTSYWINWVKQRPGQGLEWI GNIYPSDSYTNY | DIVMTQSPSSLTVTAGEKVTMSCK SSQSLLNSGNQKNYLTWYQQKPG QPPKLLIYWASTR |
| | NQKFKDKATLTVDKSSSTAYMQLS SPTSEDSAVYYCTRSWRGNSFDYW GQGTTLTVSS (SEQ ID NO:228) | ESGVPDRFTGSGSGTDFTLTISSVQ AEDLAVYYCQNDYSYPFTFGSGT KLEIK (SEQ ID NO:229) |
| | CDR1 (SEQ ID NO:230) - GYTFTSYW | CDR1 (SEQ ID NO:233) - QSLLNSGNQKNY |
| | CDR2 (SEQ ID NO:231) - IYPSDSYT | CDR2 (SEQ ID NO:234) - WAS |
| | CDR3 (SEQ ID NO:232) - TRSWRGNSFDY | CDR3 (SEQ ID NO:235) - QNDYSYPFT |

Alternatively, novel antigen-binding sites that bind to Claudin-18.2 can be identified by screening for binding to the amino acid sequence defined by SEQ ID NO:236.

SEQ ID NO:236
MAVTACQGLGFVVSLIGIAGIIAATCMDQWSTQDLYNNPVTAVFNYQGLWRSCVRESS
GFTECRGYFTLLGLPAMLQAVRALMIVGIVLGAIGLLVSIFALKCIRIGSMEDSAKANMT
LTSGIMFIVSGLCAIAGVSVFANMLVTNFWMSTANMYTGMGGMVQTVQTRYTFGAAL
FVGWVAGGLTLIGGVMMCIACRGLAPEETNYKAVSYHASGHSVAYKPGGFKASTGFG
SNTKNKKIYDGGARTEDEVQSYPSKHDYV

Within the Fc domain, CD16 binding is mediated by the hinge region and the CH2 domain. For example, within human IgG1, the interaction with CD16 is primarily focused on amino acid residues Asp 265 - Glu 269, Asn 297 - Thr 299, Ala 327 - Ile 332, Leu 234 - Ser 239, and carbohydrate residue N-acetyl-D-glucosamine in the CH2 domain (*see,* Sondermann et al, Nature, 406 (6793):267-273). Based on the known domains, mutations can be selected to enhance or reduce the binding affinity to CD16, such as by using phage-displayed libraries or yeast surface-displayed cDNA libraries, or can be designed based on the known three-dimensional structure of the interaction.

The assembly of heterodimeric antibody heavy chains can be accomplished by expressing two different antibody heavy chain sequences in the same cell, which may lead to the assembly of homodimers of each antibody heavy chain as well as assembly of heterodimers. Promoting the preferential assembly of heterodimers can be accomplished by incorporating different mutations in the CH3 domain of each antibody heavy chain constant region as shown in US13/494870, US16/028850, US11/533709, US12/875015, US13/289934, US14/773418, US12/811207, US13/866756, US14/647480, and US14/830336. For example, mutations can be made in the CH3 domain based on human IgG1 and incorporating distinct pairs of amino acid substitutions within a first polypeptide and a second polypeptide that allow these two chains to selectively heterodimerize with each other. The positions of amino acid substitutions illustrated below are all numbered according to the EU index as in Kabat.

In one scenario, an amino acid substitution in the first polypeptide replaces the original amino acid with a larger amino acid, selected from arginine (R), phenylalanine (F), tyrosine (Y) or tryptophan (W), and at least one amino acid substitution in the second polypeptide replaces the original amino acid(s) with a smaller amino acid(s), chosen from alanine (A), serine (S), threonine (T), or valine (V), such that the larger amino acid substitution (a protuberance) fits into the surface of the smaller amino acid substitutions (a cavity). For example, one polypeptide can incorporate a T366W substitution, and the other can incorporate three substitutions including T366S, L368A, and Y407V.

An antibody heavy chain variable domain of the invention can optionally be coupled to an amino acid sequence at least 90% identical to an antibody constant region, such as an IgG constant region including hinge, CH2 and CH3 domains with or without CH1 domain. In some embodiments, the amino acid sequence of the constant region is at least 90% identical to a human antibody constant region, such as an human IgG1 constant region, an IgG2 constant region, IgG3 constant region, or IgG4 constant region. In some other embodiments, the amino acid sequence of the constant region is at least 90% identical to an antibody constant region from another mammal, such as rabbit, dog, cat, mouse, or horse. One or more mutations can be incorporated into the constant region as compared to human IgG1 constant region, for example at Q347, Y349, L351, S354, E356, E357, K360, Q362, S364, T366, L368, K370, N390, K392, T394, D399, S400, D401, F405, Y407, K409, T411 and/or K439. Exemplary substitutions include, for example, Q347E, Q347R, Y349S, Y349K, Y349T, Y349D, Y349E, Y349C, T350V, L351K, L351D, L351Y, S354C, E356K, E357Q, E357L, E357W, K360E, K360W, Q362E, S364K, S364E, S364H, S364D, T366V, T366I, T366L, T366M, T366K, T366W, T366S, L368E, L368A, L368D, K370S, N390D, N390E, K392L, K392M, K392V, K392F, K392D, K392E, T394F, T394W, D399R, D399K, D399V, S400K, S400R, D401K, F405A, F405T, Y407A, Y407I, Y407V, K409F, K409W, K409D, T411D, T411E, K439D, and K439E.

In certain embodiments, mutations that can be incorporated into the CH1 of a human IgG1 constant region may be at amino acid V125, F126, P127, T135, T139, A140, F170, P171, and/or V173. In certain embodiments, mutations that can be incorporated into the Cκ of a human IgG1 constant region may be at amino acid E123, F116, S176, V163, S174, and/or T164.

Alternatively, amino acid substitutions could be selected from the following sets of substitutions shown in Table 8.

| Table 8 | | |
|---|---|---|
| | First Polypeptide | Second Polypeptide |
| Set 1 | S364E/F405A | Y349K/T394F |
| Set 2 | S364H/D401K | Y349T/T411E |
| Set 3 | S364H/T394F | Y349T/F405A |
| Set 4 | S364E/T394F | Y349K/F405A |
| Set 5 | S364E/T411E | Y349K/D401K |
| Set 6 | S364D/T394F | Y349K/F405A |
| Set 7 | S364H/F405A | Y349T/T394F |
| Set 8 | S364K/E357Q | L368D/K370S |
| Set 9 | L368D/K370S | S364K |
| Set 10 | L368E/K370S | S364K |
| Set 11 | K360E/Q362E | D401K |
| Set 12 | L368D/K370S | S364K/E357L |
| Set 13 | K370S | S364K/E357Q |
| Set 14 | F405L | K409R |
| Set 15 | K409R | F405L |

Alternatively, amino acid substitutions could be selected from the following sets of substitutions shown in Table 9.

| Table 9 | | |
|---|---|---|
| | First Polypeptide | Second Polypeptide |
| Set 1 | K409W | D399V/F405T |
| Set 2 | Y349S | E357W |
| Set 3 | K360E | Q347R |
| Set 4 | K360E/K409W | Q347R/D399V/F405T |
| Set 5 | Q347E/K360E/K409W | Q347R/D399V/F405T |
| Set 6 | Y349S/K409W | E357W/D399V/F405T |

Alternatively, amino acid substitutions could be selected from the following set of substitutions shown in Table 10.

| Table 10 | | |
|---|---|---|
| | First Polypeptide | Second Polypeptide |
| Set 1 | T366K/L351K | L351D/L368E |
| Set 2 | T366K/L351K | L351D/Y349E |
| Set 3 | T366K/L351K | L351D/Y349D |
| Set 4 | T366K/L351K | L351D/Y349E/L368E |
| Set 5 | T366K/L351K | L351D/Y349D/L368E |
| Set 6 | E356K/D399K | K392D/K409D |

Alternatively, at least one amino acid substitution in each polypeptide chain could be selected from Table 11.

| Table 11 | |
|---|---|
| First Polypeptide | Second Polypeptide |
| L351Y, D399R, D399K, S400K, S400R, Y407A, Y407I, Y407V | T366V, T366I, T366L, T366M, N390D, N390E, K392L, K392M, K392V, K392F K392D, K392E, K409F, K409W, T411D and T411E |

Alternatively, at least one amino acid substitutions could be selected from the following set of substitutions in Table 12, where the position(s) indicated in the First Polypeptide column is replaced by any known negatively-charged amino acid, and the position(s) indicated in the Second Polypeptide Column is replaced by any known positively-charged amino acid.

| Table 12 | |
|---|---|
| First Polypeptide | Second Polypeptide |
| K392, K370, K409, or K439 | D399, E356, or E357 |

Alternatively, at least one amino acid substitutions could be selected from the following set of in Table 13, where the position(s) indicated in the First Polypeptide column is replaced by any known positively-charged amino acid, and the position(s) indicated in the Second Polypeptide Column is replaced by any known negatively-charged amino acid.

| Table 13 | |
|---|---|
| First Polypeptide | Second Polypeptide |
| D399, E356, or E357 | K409, K439, K370, or K392 |

Alternatively, amino acid substitutions could be selected from the following set in Table 14.

| Table 14 | |
|---|---|
| First Polypeptide | Second Polypeptide |
| T350V, L351Y, F405A, and Y407V | T350V, T366L, K392L, and T394W |

Alternatively, or in addition, the structural stability of a heteromultimer protein may be increased by introducing S354C on either of the first or second polypeptide chain, and Y349C on the opposing polypeptide chain, which forms an artificial disulfide bridge within the interface of the two polypeptides.

The multi-specific proteins described above can be made using recombinant DNA technology well known to a skilled person in the art. For example, a first nucleic acid sequence encoding the first immunoglobulin heavy chain can be cloned into a first expression vector; a second nucleic acid sequence encoding the second immunoglobulin heavy chain can be cloned into a second expression vector; a third nucleic acid sequence encoding the immunoglobulin light chain can be cloned into a third expression vector; the first, second, and third expression vectors can be stably transfected together into host cells to produce the multimeric proteins.

To achieve the highest yield of the multi-specific protein, different ratios of the first, second, and third expression vector can be explored to determine the optimal ratio for transfection into the host cells. After transfection, single clones can be isolated for cell bank generation using methods known in the art, such as limited dilution, ELISA, FACS, microscopy, or Clonepix.

Clones can be cultured under conditions suitable for bio-reactor scale-up and maintained expression of the multi-specific protein. The multispecific proteins can be isolated and purified using methods known in the art including centrifugation, depth filtration, cell lysis, homogenization, freeze-thawing, affinity purification, gel filtration, ion exchange chromatography, hydrophobic interaction exchange chromatography, and mixed-mode chromatography.

### II. Characteristics of the multi-specific proteins

In certain embodiments, the multi-specific proteins described herein, which include an NKG2D-binding domain and a binding domain for CAIX, ANO1, mesothelin, TROP2, CEA or Claudin-18.2, bind to cells expressing human NKG2D. In certain embodiments, the multi-specific proteins bind to the tumor associated antigen CAIX, ANO1, mesothelin, TROP2, CEA or Claudin-18.2 at a comparable level to that of a monoclonal antibody having the same respective CAIX, ANO1, mesothelin, TROP2, CEA or Claudin-18.2-binding domain. However, the multi-specific proteins described herein may be more effective in reducing tumor growth and killing cancer cells expressing CAIX, ANO1, mesothelin, TROP2, CEA or Claudin-18.2 than the corresponding CAIX, ANO1, mesothelin, TROP2, CEA or Claudin-18.2 monoclonal antibodies.

In certain embodiments, the multi-specific proteins described herein, which include an NKG2D-binding domain and a binding domain for CAIX, ANO1, mesothelin, TROP2, CEA or Claudin-18.2, can activate primary human NK cells when culturing with tumor cells expressing the antigen CAIX, ANO1, mesothelin, TROP2, CEA or Claudin-18.2. NK cell activation is marked by the increase in CD107a degranulation and IFNγ cytokine production. Furthermore, compared to a monoclonal antibody that includes the same CAIX, ANO1, mesothelin, TROP2, CEA or Claudin-18.2-binding domain, the multi-specific proteins show superior activation of human NK cells in the presence of tumor cells expressing the antigen CAIX, ANO1, mesothelin, TROP2, CEA or Claudin-18.2.

In certain embodiments, the multi-specific proteins described herein, which include an NKG2D-binding domain and a binding domain for CAIX, ANO1, mesothelin, TROP2, CEA or Claudin-18.2, can enhance the activity of rested and IL-2-activated human NK cells in the presence of tumor cells expressing the antigen CAIX, ANO1, mesothelin, TROP2, CEA or Claudin-18.2.

In certain embodiments, the multi-specific proteins described herein, which include an NKG2D-binding domain and a binding domain for tumor-associated antigen CAIX, ANO1, mesothelin, TROP2, CEA or Claudin-18.2, can enhance the cytotoxic activity of rested and IL-2-activated human NK cells in the presence of tumor cells expressing the antigen CAIX, ANO1, mesothelin, TROP2, CEA or Claudin-18.2. In certain embodiments, compared to the corresponding monoclonal antibodies, the multi-specific proteins can offer an advantage against tumor cells expressing medium and low CAIX, ANO1, mesothelin, TROP2, CEA or Claudin-18.2.

In certain embodiments, the multi-specific proteins described herein can be advantageous in treating cancers with high expression of Fc receptor (FcR), or cancers residing in a tumor microenvironment with high levels of FcR, compared to the corresponding CAIX, ANO1, mesothelin, TROP2, CEA or Claudin-18.2 monoclonal antibodies. Monoclonal antibodies exert their effects on tumor growth through multiple mechanisms including ADCC, CDC, phagocytosis, and signal blockade amongst others. Amongst FcγRs, CD16 has the lowest affinity for IgG Fc; FcγRI (CD64) is the high-affinity FcR, which binds about 1000 times more strongly to IgG Fc than CD16. CD64 is normally expressed on many hematopoietic lineages such as the myeloid lineage, and can be expressed on tumors derived from these cell types, such as acute myeloid leukemia (AML). Immune cells infiltrating into the tumor, such as MDSCs and monocytes, also express CD64 and are known to infiltrate the tumor microenvironment. Expression of CD64 by the tumor or in the tumor microenvironment can have a detrimental effect on monoclonal antibody therapy. Expression of CD64 in the tumor microenvironment makes it difficult for these antibodies to engage CD16 on the surface of NK cells, as the antibodies prefer to bind the high-affinity receptor. The multi-specific proteins, through targeting two activating receptors on the surface of NK cells, can overcome the detrimental effect of CD64 expression (either on tumor or tumor microenvironment) on monoclonal antibody therapy. Regardless of CD64 expression on the tumor cells, the multi-specific proteins are able to mediate human NK cell responses against all tumor cells, because dual targeting of two activating receptors on NK cells provides stronger specific binding to NK cells.

In some embodiments, the multi-specific proteins described herein can provide a better safety profile through reduced on-target off-tumor side effects. Natural killer cells and CD8 T cells are both able to directly lyse tumor cells, although the mechanisms through which NK cells and CD8 T cells recognize normal self from tumor cells differ. The activity of NK cells is regulated by the balance of signals from activating (NCRs, NKG2D, CD16, *etc.*) and inhibitory (KIRs, NKG2A, etc.) receptors. The balance of these activating and inhibitory signals allow NK cells to determine healthy self-cells from stressed, virally infected, or transformed self-cells. This "built-in" mechanism of self-tolerance will help protect normal heathy tissue from NK cell responses. To extend this principle, the self-tolerance of NK cells will allow TriNKETs to target antigens expressed both on self and tumor without off-tumor side effects, or with an increased therapeutic window. Unlike natural killer cells, T cells require recognition of a specific peptide presented by MHC molecules for activation and effector functions. T cells have been the primary target of immunotherapy, and many strategies have been developed to redirect T cell responses against the tumor. T cell bispecifics, checkpoint inhibitors, and CAR-T cells have all been approved by the FDA, but often suffer from dose-limiting toxicities. T cell bispecifics and CAR-T cells work around the TCR-MHC recognition system by using binding domains to target antigens on the surface of tumor cells, and using engineered signaling domains to transduce the activation signals into the effector cell. Although effective at eliciting an antitumor immune response these therapies are often coupled with cytokine release syndrome (CRS), and on-target off-tumor side effects. The multi-specific proteins are unique in this context as they will not "override" the natural systems of NK cell activation and inhibition. Instead, the multi-specific proteins are designed to sway the balance, and provide additional activation signals to the NK cells, while maintaining NK tolerance to healthy self.

In some embodiments, the multi-specific proteins described herein can delay progression of the tumor more effectively than the corresponding CAIX, ANO1, mesothelin, TROP2, CEA or Claudin-18.2 monoclonal antibodies that include the same antigen-binding domain. In some embodiments, the multi-specific proteins described herein can be more effective against cancer metastases than the corresponding CAIX, ANO1, mesothelin, TROP2, CEA or Claudin-18.2 monoclonal antibodies that include the same antigen-binding domain.

### III. THERAPEUTIC APPLICATIONS

The invention provides methods for treating cancer using a multi-specific binding protein described herein and/or a pharmaceutical composition described herein. The methods may be used to treat a variety of cancers which express CAIX, ANO1, mesothelin, TROP2, CEA or Claudin-18.2 by administering to a patient in need thereof a therapeutically effective amount of a multi-specific binding protein described herein.

The therapeutic method can be characterized according to the cancer to be treated. Cancers to be treated using CAIX-targeting multi-specific binding proteins include, for example, renal cell carcinoma, breast cancer, glioblastoma, head and neck cancer, gastric cancers, bladder cancer, ovarian cancer, tumors of the esophagus, lung, colon, kidney, cervix and non-small cell lung carcinoma. Cancers to be treated using mesothelin-targeting multi-specific binding proteins include, for example, mesothelioma, ovarian cancer, pancreatic cancer, non-small cell lung cancer, breast cancer, cholangiocarcinoma, gastric cancer, uterine serous carcinoma, thymic carcinoma, and acute myeloid leukemia. Cancers to be treated using ANO1-targeting multi-specific binding proteins include, for example, esophageal squamous cell cancer (ESCC), gastrointestinal stromal tumor (GIST), head and neck squamous cell carcinoma (HNSCC), pancreatic cancer, breast cancer, prostate cancer, and sarcoma. Cancers to be treated using TROP2-targeting multi-specific binding proteins include, for example, breast, lung, gastric, colorectal, pancreatic, prostatic, cervical, head-and-neck cancer, nasopharyngeal carcinoma, and ovarian carcinoma. Cancers to be treated using CEA-targeting multi-specific binding proteins include, for example, gastrointestinal cancer, colorectal cancer, pancreatic cancer, non-small cell lung cancer and breast cancer. Cancers to be treated using Claudin-18.2-targeting multi-specific binding proteins include, for example, esophageal cancer, non-small cell lung carcinoma, ovarian cancer, colon cancer, and several forms of biliary ductal carcinoma.

In certain other embodiments, the cancer is brain cancer, cervical cancer, colon cancer, colorectal cancer, endometrial cancer, esophageal cancer, leukemia, lung cancer, liver cancer, melanoma, ovarian cancer, pancreatic cancer, rectal cancer, renal cancer, stomach cancer, testicular cancer, or uterine cancer. In yet other embodiments, the cancer is a squamous cell carcinoma, adenocarcinoma, small cell carcinoma, melanoma, neuroblastoma, sarcoma (e.g., an angiosarcoma or chondrosarcoma), larynx cancer, parotid cancer, bilary tract cancer, thyroid cancer, acral lentiginous melanoma, actinic keratoses, acute lymphocytic leukemia, acute myeloid leukemia, adenoid cystic carcinoma, adenomas, adenosarcoma, adenosquamous carcinoma, anal canal cancer, anal cancer, anorectum cancer, astrocytic tumor, bartholin gland carcinoma, basal cell carcinoma, biliary cancer, bone cancer, bone marrow cancer, bronchial cancer, bronchial gland carcinoma, carcinoid, cholangiocarcinoma, chondosarcoma, choriod plexus papilloma/carcinoma, chronic lymphocytic leukemia, chronic myeloid leukemia, clear cell carcinoma, connective tissue cancer, cystadenoma, digestive system cancer, duodenum cancer, endocrine system cancer, endodermal sinus tumor, endometrial hyperplasia, endometrial stromal sarcoma, endometrioid adenocarcinoma, endothelial cell cancer, ependymal cancer, epithelial cell cancer, Ewing's sarcoma, eye and orbit cancer, female genital cancer, focal nodular hyperplasia, gallbladder cancer, gastric antrum cancer, gastric fundus cancer, gastrinoma, glioblastoma, glucagonoma, heart cancer, hemangiblastomas, hemangioendothelioma, hemangiomas, hepatic adenoma, hepatic adenomatosis, hepatobiliary cancer, hepatocellular carcinoma, Hodgkin's disease, ileum cancer, insulinoma, intaepithelial neoplasia, interepithelial squamous cell neoplasia, intrahepatic bile duct cancer, invasive squamous cell carcinoma, jejunum cancer, joint cancer, Kaposi's sarcoma, pelvic cancer, large cell carcinoma, large intestine cancer, leiomyosarcoma, lentigo maligna melanomas, lymphoma, male genital cancer, malignant melanoma, malignant mesothelial tumors, medulloblastoma, medulloepithelioma, meningeal cancer, mesothelial cancer, metastatic carcinoma, mouth cancer, mucoepidermoid carcinoma, multiple myeloma, muscle cancer, nasal tract cancer, nervous system cancer, neuroepithelial adenocarcinoma nodular melanoma, non-epithelial skin cancer, non-Hodgkin's lymphoma, oat cell carcinoma, oligodendroglial cancer, oral cavity cancer, osteosarcoma, papillary serous adenocarcinoma, penile cancer, pharynx cancer, pituitary tumors, plasmacytoma, pseudosarcoma, pulmonary blastoma, rectal cancer, renal cell carcinoma, respiratory system cancer, retinoblastoma, rhabdomyosarcoma, sarcoma, serous carcinoma, sinus cancer, skin cancer, small cell carcinoma, small intestine cancer, smooth muscle cancer, soft tissue cancer, somatostatin-secreting tumor, spine cancer, squamous cell carcinoma, striated muscle cancer, submesothelial cancer, superficial spreading melanoma, T cell leukemia, tongue cancer, undifferentiated carcinoma, ureter cancer, urethra cancer, urinary bladder cancer, urinary system cancer, uterine cervix cancer, uterine corpus cancer, uveal melanoma, vaginal cancer, verrucous carcinoma, VIPoma, vulva cancer, well differentiated carcinoma, or Wilms tumor.

In certain other embodiments, the cancer is non-Hodgkin's lymphoma, such as a B-cell lymphoma or a T-cell lymphoma. In certain embodiments, the non-Hodgkin's lymphoma is a B-cell lymphoma, such as a diffuse large B-cell lymphoma, primary mediastinal B-cell lymphoma, follicular lymphoma, small lymphocytic lymphoma, mantle cell lymphoma, marginal zone B-cell lymphoma, extranodal marginal zone B-cell lymphoma, nodal marginal zone B-cell lymphoma, splenic marginal zone B-cell lymphoma, Burkitt lymphoma, lymphoplasmacytic lymphoma, hairy cell leukemia, or primary central nervous system (CNS) lymphoma. In certain other embodiments, the non-Hodgkin's lymphoma is a T-cell lymphoma, such as a precursor T-lymphoblastic lymphoma, peripheral T-cell lymphoma, cutaneous T-cell lymphoma, angioimmunoblastic T-cell lymphoma, extranodal natural killer/T-cell lymphoma, enteropathy type T-cell lymphoma, subcutaneous panniculitis-like T-cell lymphoma, anaplastic large cell lymphoma, or peripheral T-cell lymphoma.

The cancer to be treated can be characterized according to the presence of a particular antigen expressed on the surface of the cancer cell. In certain embodiments, the cancer cell can express one or more of the following: CD2, CD19, CD20, CD30, CD38, CD40, CD52, CD70, EGFR/ERBB1, IGF1R, HER3/ERBB3, HER4/ERBB4, MUC1, cMET, SLAMF7, PSCA, MICA, MICB, TRAILR1, TRAILR2, MAGE-A3, B7.1, B7.2, CTLA4, and PD1.

### IV. COMBINATION THERAPY

Another aspect of the invention provides for combination therapy. Multi-specific binding proteins described herein be used in combination with additional therapeutic agents to treat the cancer.

Exemplary therapeutic agents that may be used as part of a combination therapy in treating cancer, include, for example, radiation, mitomycin, tretinoin, ribomustin, gemcitabine, vincristine, etoposide, cladribine, mitobronitol, methotrexate, doxorubicin, carboquone, pentostatin, nitracrine, zinostatin, cetrorelix, letrozole, raltitrexed, daunorubicin, fadrozole, fotemustine, thymalfasin, sobuzoxane, nedaplatin, cytarabine, bicalutamide, vinorelbine, vesnarinone, aminoglutethimide, amsacrine, proglumide, elliptinium acetate, ketanserin, doxifluridine, etretinate, isotretinoin, streptozocin, nimustine, vindesine, flutamide, drogenil, butocin, carmofur, razoxane, sizofilan, carboplatin, mitolactol, tegafur, ifosfamide, prednimustine, picibanil, levamisole, teniposide, improsulfan, enocitabine, lisuride, oxymetholone, tamoxifen, progesterone, mepitiostane, epitiostanol, formestane, interferon-alpha, interferon-2 alpha, interferon-beta, interferon-gamma, colony stimulating factor-1, colony stimulating factor-2, denileukin diftitox, interleukin-2, luteinizing hormone releasing factor and variations of the aforementioned agents that may exhibit differential binding to its cognate receptor, and increased or decreased serum half-life.

An additional class of agents that may be used as part of a combination therapy in treating cancer is immune checkpoint inhibitors. Exemplary immune checkpoint inhibitors include agents that inhibit one or more of (i) cytotoxic T-lymphocyte-associated antigen 4 (CTLA4), (ii) programmed cell death protein 1 (PD1), (iii) PDL1, (iv) LAG3, (v) B7-H3, (vi) B7-H4, and (vii) TIM3. The CTLA4 inhibitor ipilimumab has been approved by the United States Food and Drug Administration for treating melanoma.

Yet other agents that may be used as part of a combination therapy in treating cancer are monoclonal antibody agents that target non-checkpoint targets (*e.g.,* herceptin) and non-cytotoxic agents (*e.g.,* tyrosine-kinase inhibitors).

Yet other categories of anti-cancer agents include, for example: (i) an inhibitor selected from an ALK Inhibitor, an ATR Inhibitor, an A2A Antagonist, a Base Excision Repair Inhibitor, a Bcr-Abl Tyrosine Kinase Inhibitor, a Bruton's Tyrosine Kinase Inhibitor, a CDC7 Inhibitor, a CHK1 Inhibitor, a Cyclin-Dependent Kinase Inhibitor, a DNA-PK Inhibitor, an Inhibitor of both DNA-PK and mTOR, a DNMT1 Inhibitor, a DNMT1 Inhibitor plus 2-chloro-deoxyadenosine, an HDAC Inhibitor, a Hedgehog Signaling Pathway Inhibitor, an IDO Inhibitor, a JAK Inhibitor, a mTOR Inhibitor, a MEK Inhibitor, a MELK Inhibitor, a MTH1 Inhibitor, a PARP Inhibitor, a Phosphoinositide 3-Kinase Inhibitor, an Inhibitor of both PARP1 and DHODH, a Proteasome Inhibitor, a Topoisomerase-II Inhibitor, a Tyrosine Kinase Inhibitor, a VEGFR Inhibitor, and a WEE1 Inhibitor; (ii) an agonist of OX40, CD137, CD40, GITR, CD27, HVEM, TNFRSF25, or ICOS; and (iii) a cytokine selected from IL-12, IL-15, GM-CSF, and G-CSF.

Proteins of the invention can also be used as an adjunct to surgical removal of the primary lesion.

The amount of multi-specific binding protein and additional therapeutic agent and the relative timing of administration may be selected in order to achieve a desired combined therapeutic effect. For example, when administering a combination therapy to a patient in need of such administration, the therapeutic agents in the combination, or a pharmaceutical composition or compositions comprising the therapeutic agents, may be administered in any order such as, for example, sequentially, concurrently, together, simultaneously and the like. Further, for example, a multi-specific binding protein may be administered during a time when the additional therapeutic agent(s) exerts its prophylactic or therapeutic effect, or *vice versa.*

### V. PHARMACEUTICAL COMPOSITIONS

The present disclosure also features pharmaceutical compositions that contain a therapeutically effective amount of a protein described herein. The composition can be formulated for use in a variety of drug delivery systems. One or more physiologically acceptable excipients or carriers can also be included in the composition for proper formulation. Suitable formulations for use in the present disclosure are found in Remington's Pharmaceutical Sciences, Mack Publishing Company, Philadelphia, Pa., 17th ed., 1985. For a brief review of methods for drug delivery, *see, e.g.,* Langer (Science 249:1527-1533, 1990).

The intravenous drug delivery formulation of the present disclosure may be contained in a bag, a pen, or a syringe. In certain embodiments, the bag may be connected to a channel comprising a tube and/or a needle. In certain embodiments, the formulation may be a lyophilized formulation or a liquid formulation. In certain embodiments, the formulation may be freeze-dried (lyophilized) and contained in about 12-60 vials. In certain embodiments, the formulation may be freeze-dried and 45 mg of the freeze-dried formulation may be contained in one vial. In certain embodiments, the about 40 mg - about 100 mg of freeze-dried formulation may be contained in one vial. In certain embodiments, freeze-dried formulations from 12, 27, or 45 vials are combined to obtained a therapeutic dose of the protein in the intravenous drug formulation. In certain embodiments, the formulation may be a liquid formulation and stored as about 250 mg/vial to about 1000 mg/vial. In certain embodiments, the formulation may be a liquid formulation and stored as about 600 mg/vial. In certain embodiments, the formulation may be a liquid formulation and stored as about 250 mg/vial.

This present disclosure could exist in a liquid aqueous pharmaceutical formulation including a therapeutically effective amount of the protein in a buffered solution forming a formulation.

These compositions may be sterilized by conventional sterilization techniques, or may be sterile filtered. The resulting aqueous solutions may be packaged for use as-is, or lyophilized, the lyophilized preparation being combined with a sterile aqueous carrier prior to administration. The pH of the preparations typically will be between 3 and 11, more preferably between 5 and 9 or between 6 and 8, and most preferably between 7 and 8, such as 7 to 7.5. The resulting compositions in solid form may be packaged in multiple single dose units, each containing a fixed amount of the above-mentioned agent or agents. The composition in solid form can also be packaged in a container for a flexible quantity.

In certain embodiments, the present disclosure provides a formulation with an extended shelf life including the protein of the present disclosure, in combination with mannitol, citric acid monohydrate, sodium citrate, disodium phosphate dihydrate, sodium dihydrogen phosphate dihydrate, sodium chloride, polysorbate 80, water, and sodium hydroxide.

In certain embodiments, an aqueous formulation is prepared including the protein of the present disclosure in a pH-buffered solution. The buffer of this invention may have a pH ranging from about 4 to about 8, *e.g.,* from about 4.5 to about 6.0, or from about 4.8 to about 5.5, or may have a pH of about 5.0 to about 5.2. Ranges intermediate to the above recited pH's are also intended to be part of this disclosure. For example, ranges of values using a combination of any of the above recited values as upper and/or lower limits are intended to be included. Examples of buffers that will control the pH within this range include acetate (e.g. sodium acetate), succinate (such as sodium succinate), gluconate, histidine, citrate and other organic acid buffers.

In certain embodiments, the formulation includes a buffer system which contains citrate and phosphate to maintain the pH in a range of about 4 to about 8. In certain embodiments the pH range may be from about 4.5 to about 6.0, or from about pH 4.8 to about 5.5, or in a pH range of about 5.0 to about 5.2. In certain embodiments, the buffer system includes citric acid monohydrate, sodium citrate, disodium phosphate dihydrate, and/or sodium dihydrogen phosphate dihydrate. In certain embodiments, the buffer system includes about 1.3 mg/ml of citric acid (*e.g.,* 1.305 mg/ml), about 0.3 mg/ml of sodium citrate (*e.g.,* 0.305 mg/ml), about 1.5 mg/ml of disodium phosphate dihydrate (*e.g.,* 1.53 mg/ml), about 0.9 mg/ml of sodium dihydrogen phosphate dihydrate (*e.g.,* 0.86), and about 6.2 mg/ml of sodium chloride (*e.g.,* 6.165 mg/ml). In certain embodiments, the buffer system includes 1-1.5 mg/ml of citric acid, 0.25 to 0.5 mg/ml of sodium citrate, 1.25 to 1.75 mg/ml of disodium phosphate dihydrate, 0.7 to 1.1 mg/ml of sodium dihydrogen phosphate dihydrate, and 6.0 to 6.4 mg/ml of sodium chloride. In certain embodiments, the pH of the formulation is adjusted with sodium hydroxide.

A polyol, which acts as a tonicifier and may stabilize the antibody, may also be included in the formulation. The polyol is added to the formulation in an amount which may vary with respect to the desired isotonicity of the formulation. In certain embodiments, the aqueous formulation may be isotonic. The amount of polyol added may also be altered with respect to the molecular weight of the polyol. For example, a lower amount of a monosaccharide (e.g., mannitol) may be added, compared to a disaccharide (such as trehalose). In certain embodiments, the polyol which may be used in the formulation as a tonicity agent is mannitol. In certain embodiments, the mannitol concentration may be about 5 to about 20 mg/ml. In certain embodiments, the concentration of mannitol may be about 7.5 to 15 mg/ml. In certain embodiments, the concentration of mannitol may be about 10-14 mg/ml. In certain embodiments, the concentration of mannitol may be about 12 mg/ml. In certain embodiments, the polyol sorbitol may be included in the formulation.

A detergent or surfactant may also be added to the formulation. Exemplary detergents include nonionic detergents such as polysorbates (*e.g.,* polysorbates 20, 80 etc.) or poloxamers (e.g., poloxamer 188). The amount of detergent added is such that it reduces aggregation of the formulated antibody and/or minimizes the formation of particulates in the formulation and/or reduces adsorption. In certain embodiments, the formulation may include a surfactant which is a polysorbate. In certain embodiments, the formulation may contain the detergent polysorbate 80 or Tween 80. Tween 80 is a term used to describe polyoxyethylene (20) sorbitanmonooleate (*see* Fiedler, Lexikon der Hifsstoffe, Editio Cantor Verlag Aulendorf, 4th ed., 1996). In certain embodiments, the formulation may contain between about 0.1 mg/mL and about 10 mg/mL of polysorbate 80, or between about 0.5 mg/mL and about 5 mg/mL. In certain embodiments, about 0.1% polysorbate 80 may be added in the formulation.

In embodiments, the protein product of the present disclosure is formulated as a liquid formulation. The liquid formulation may be presented at a 10 mg/mL concentration in either a USP / Ph Eur type I 50R vial closed with a rubber stopper and sealed with an aluminum crimp seal closure. The stopper may be made of elastomer complying with USP and Ph Eur. In certain embodiments vials may be filled with 61.2 mL of the protein product solution in order to allow an extractable volume of 60 mL. In certain embodiments, the liquid formulation may be diluted with 0.9% saline solution.

In certain embodiments, the liquid formulation of the disclosure may be prepared as a 10 mg/mL concentration solution in combination with a sugar at stabilizing levels. In certain embodiments the liquid formulation may be prepared in an aqueous carrier. In certain embodiments, a stabilizer may be added in an amount no greater than that which may result in a viscosity undesirable or unsuitable for intravenous administration. In certain embodiments, the sugar may be disaccharides, *e.g.,* sucrose. In certain embodiments, the liquid formulation may also include one or more of a buffering agent, a surfactant, and a preservative.

In certain embodiments, the pH of the liquid formulation may be set by addition of a pharmaceutically acceptable acid and/or base. In certain embodiments, the pharmaceutically acceptable acid may be hydrochloric acid. In certain embodiments, the base may be sodium hydroxide.

In addition to aggregation, deamidation is a common product variant of peptides and proteins that may occur during fermentation, harvest/cell clarification, purification, drug substance/drug product storage and during sample analysis. Deamidation is the loss of NH₃ from a protein forming a succinimide intermediate that can undergo hydrolysis. The succinimide intermediate results in a 17 dalton mass decrease of the parent peptide. The subsequent hydrolysis results in an 18 dalton mass increase. Isolation of the succinimide intermediate is difficult due to instability under aqueous conditions. As such, deamidation is typically detectable as 1 dalton mass increase. Deamidation of an asparagine results in either aspartic or isoaspartic acid. The parameters affecting the rate of deamidation include pH, temperature, solvent dielectric constant, ionic strength, primary sequence, local polypeptide conformation and tertiary structure. The amino acid residues adjacent to Asn in the peptide chain affect deamidation rates. Gly and Ser following an Asn in protein sequences results in a higher susceptibility to deamidation.

In certain embodiments, the liquid formulation of the present disclosure may be preserved under conditions of pH and humidity to prevent deamination of the protein product.

The aqueous carrier of interest herein is one which is pharmaceutically acceptable (safe and non-toxic for administration to a human) and is useful for the preparation of a liquid formulation. Illustrative carriers include sterile water for injection (SWFI), bacteriostatic water for injection (BWFI), a pH buffered solution (*e.g.,* phosphate-buffered saline), sterile saline solution, Ringer's solution or dextrose solution.

A preservative may be optionally added to the formulations herein to reduce bacterial action. The addition of a preservative may, for example, facilitate the production of a multi-use (multiple-dose) formulation.

Intravenous (IV) formulations may be the preferred administration route in particular instances, such as when a patient is in the hospital after transplantation receiving all drugs via the IV route. In certain embodiments, the liquid formulation is diluted with 0.9% Sodium Chloride solution before administration. In certain embodiments, the diluted drug product for injection is isotonic and suitable for administration by intravenous infusion.

In certain embodiments, a salt or buffer components may be added in an amount of 10 mM - 200 mM. The salts and/or buffers are pharmaceutically acceptable and are derived from various known acids (inorganic and organic) with "base forming" metals or amines. In certain embodiments, the buffer may be phosphate buffer. In certain embodiments, the buffer may be glycinate, carbonate, citrate buffers, in which case, sodium, potassium or ammonium ions can serve as counterion.

A preservative may be optionally added to the formulations herein to reduce bacterial action. The addition of a preservative may, for example, facilitate the production of a multi-use (multiple-dose) formulation.

The aqueous carrier of interest herein is one which is pharmaceutically acceptable (safe and non-toxic for administration to a human) and is useful for the preparation of a liquid formulation. Illustrative carriers include sterile water for injection (SWFI), bacteriostatic water for injection (BWFI), a pH buffered solution (*e.g.,* phosphate-buffered saline), sterile saline solution, Ringer's solution or dextrose solution.

This present disclosure could exist in a lyophilized formulation including the proteins and a lyoprotectant. The lyoprotectant may be sugar, *e.g.,* disaccharides. In certain embodiments, the lyoprotectant may be sucrose or maltose. The lyophilized formulation may also include one or more of a buffering agent, a surfactant, a bulking agent, and/or a preservative.

The amount of sucrose or maltose useful for stabilization of the lyophilized drug product may be in a weight ratio of at least 1:2 protein to sucrose or maltose. In certain embodiments, the protein to sucrose or maltose weight ratio may be of from 1:2 to 1:5.

In certain embodiments, the pH of the formulation, prior to lyophilization, may be set by addition of a pharmaceutically acceptable acid and/or base. In certain embodiments the pharmaceutically acceptable acid may be hydrochloric acid. In certain embodiments, the pharmaceutically acceptable base may be sodium hydroxide.

Before lyophilization, the pH of the solution containing the protein of the present disclosure may be adjusted between 6 to 8. In certain embodiments, the pH range for the lyophilized drug product may be from 7 to 8.

In certain embodiments, a salt or buffer components may be added in an amount of 10 mM - 200 mM. The salts and/or buffers are pharmaceutically acceptable and are derived from various known acids (inorganic and organic) with "base forming" metals or amines. In certain embodiments, the buffer may be phosphate buffer. In certain embodiments, the buffer may be glycinate, carbonate, citrate buffers, in which case, sodium, potassium or ammonium ions can serve as counterion.

In certain embodiments, a "bulking agent" may be added. A "bulking agent" is a compound which adds mass to a lyophilized mixture and contributes to the physical structure of the lyophilized cake (*e.g.,* facilitates the production of an essentially uniform lyophilized cake which maintains an open pore structure). Illustrative bulking agents include mannitol, glycine, polyethylene glycol and sorbitol. The lyophilized formulations of the present invention may contain such bulking agents.

A preservative may be optionally added to the formulations herein to reduce bacterial action. The addition of a preservative may, for example, facilitate the production of a multi-use (multiple-dose) formulation.

In certain embodiments, the lyophilized drug product may be constituted with an aqueous carrier. The aqueous carrier of interest herein is one which is pharmaceutically acceptable (*e.g.,* safe and non-toxic for administration to a human) and is useful for the preparation of a liquid formulation, after lyophilization. Illustrative diluents include sterile water for injection (SWFI), bacteriostatic water for injection (BWFI), a pH buffered solution (*e.g.,* phosphate-buffered saline), sterile saline solution, Ringer's solution or dextrose solution.

In certain embodiments, the lyophilized drug product of the current disclosure is reconstituted with either Sterile Water for Injection, USP (SWFI) or 0.9% Sodium Chloride Injection, USP. During reconstitution, the lyophilized powder dissolves into a solution.

In certain embodiments, the lyophilized protein product of the instant disclosure is constituted to about 4.5 mL water for injection and diluted with 0.9% saline solution (sodium chloride solution).

Actual dosage levels of the active ingredients in the pharmaceutical compositions of this invention may be varied so as to obtain an amount of the active ingredient which is effective to achieve the desired therapeutic response for a particular patient, composition, and mode of administration, without being toxic to the patient.

The specific dose can be a uniform dose for each patient, for example, 50-5000 mg of protein. Alternatively, a patient's dose can be tailored to the approximate body weight or surface area of the patient. Other factors in determining the appropriate dosage can include the disease or condition to be treated or prevented, the severity of the disease, the route of administration, and the age, sex and medical condition of the patient. Further refinement of the calculations necessary to determine the appropriate dosage for treatment is routinely made by those skilled in the art, especially in light of the dosage information and assays disclosed herein. The dosage can also be determined through the use of known assays for determining dosages used in conjunction with appropriate dose-response data. An individual patient's dosage can be adjusted as the progress of the disease is monitored. Blood levels of the targetable construct or complex in a patient can be measured to see if the dosage needs to be adjusted to reach or maintain an effective concentration. Pharmacogenomics may be used to determine which targetable constructs and/or complexes, and dosages thereof, are most likely to be effective for a given individual (Schmitz et al., Clinica Chimica Acta 308: 43-53, 2001; Steimer et al., Clinica Chimica Acta 308: 33-41, 2001).

In general, dosages based on body weight are from about 0.01 µg to about 100 mg per kg of body weight, such as about 0.01 µg to about 100 mg/kg of body weight, about 0.01 µg to about 50 mg/kg of body weight, about 0.01 µg to about 10 mg/kg of body weight, about 0.01 µg to about 1 mg/kg of body weight, about 0.01 µg to about 100 µg/kg of body weight, about 0.01 µg to about 50 µg/kg of body weight, about 0.01 µg to about 10 µg/kg of body weight, about 0.01 µg to about 1 µg/kg of body weight, about 0.01 µg to about 0.1 µg/kg of body weight, about 0.1 µg to about 100 mg/kg of body weight, about 0.1 µg to about 50 mg/kg of body weight, about 0.1 µg to about 10 mg/kg of body weight, about 0.1 µg to about 1 mg/kg of body weight, about 0.1 µg to about 100 µg/kg of body weight, about 0.1 µg to about 10 µg/kg of body weight, about 0.1 µg to about 1 µg/kg of body weight, about 1 µg to about 100 mg/kg of body weight, about 1 µg to about 50 mg/kg of body weight, about 1 µg to about 10 mg/kg of body weight, about 1 µg to about 1 mg/kg of body weight, about 1 µg to about 100 µg/kg of body weight, about 1 µg to about 50 µg/kg of body weight, about 1 µg to about 10 µg/kg of body weight, about 10 µg to about 100 mg/kg of body weight, about 10 µg to about 50 mg/kg of body weight, about 10 µg to about 10 mg/kg of body weight, about 10 µg to about 1 mg/kg of body weight, about 10 µg to about 100 µg/kg of body weight, about 10 µg to about 50 µg/kg of body weight, about 50 µg to about 100 mg/kg of body weight, about 50µg to about 50 mg/kg of body weight, about 50 µg to about 10 mg/kg of body weight, about 50 µg to about 1 mg/kg of body weight, about 50 µg to about 100 µg/kg of body weight, about 100 µg to about 100 mg/kg of body weight, about 100 µg to about 50 mg/kg of body weight, about 100 µg to about 10 mg/kg of body weight, about 100 µg to about 1 mg/kg of body weight, about 1 mg to about 100 mg/kg of body weight, about 1 mg to about 50 mg/kg of body weight, about 1 mg to about 10 mg/kg of body weight, about 10 mg to about 100 mg/kg of body weight, about 10 mg to about 50 mg/kg of body weight, about 50 mg to about 100 mg/kg of body weight.

Doses may be given once or more times daily, weekly, monthly or yearly, or even once every 2 to 20 years. Persons of ordinary skill in the art can easily estimate repetition rates for dosing based on measured residence times and concentrations of the targetable construct or complex in bodily fluids or tissues. Administration of the present invention could be intravenous, intraarterial, intraperitoneal, intramuscular, subcutaneous, intrapleural, intrathecal, intracavitary, by perfusion through a catheter or by direct intralesional injection. This may be administered once or more times daily, once or more times weekly, once or more times monthly, and once or more times annually.

The description above describes multiple aspects and embodiments of the invention. The patent application specifically contemplates all combinations and permutations of the aspects and embodiments.

### EXAMPLES

The invention now being generally described, will be more readily understood by reference to the following examples, which are included merely for purposes of illustration of certain aspects and embodiments of the present invention, and is not intended to limit the invention.

### Example 1 - NKG2D binding domains bind to NKG2D

### NKG2D binding domains bind to purified recombinant NKG2D

The nucleic acid sequences of human, mouse or cynomolgus NKG2D ectodomains were fused with nucleic acid sequences encoding human IgG1 Fc domains and introduced into mammalian cells to be expressed. After purification, NKG2D-Fc fusion proteins were adsorbed to wells of microplates. After blocking the wells with bovine serum albumin to prevent non-specific binding, NKG2D-binding domains were titrated and added to the wells pre-adsorbed with NKG2D-Fc fusion proteins. Primary antibody binding was detected using a secondary antibody which was conjugated to horseradish peroxidase and specifically recognizes a human kappa light chain to avoid Fc cross-reactivity. 3,3',5,5'-Tetramethylbenzidine (TMB), a substrate for horseradish peroxidase, was added to the wells to visualize the binding signal, whose absorbance was measured at 450 nM and corrected at 540 nM. An NKG2D-binding domain clone, an isotype control or a positive control (selected from SEQ ID NOs:47-50, or anti-mouse NKG2D clones MI-6 and CX-5 available at eBioscience) was added to each well.

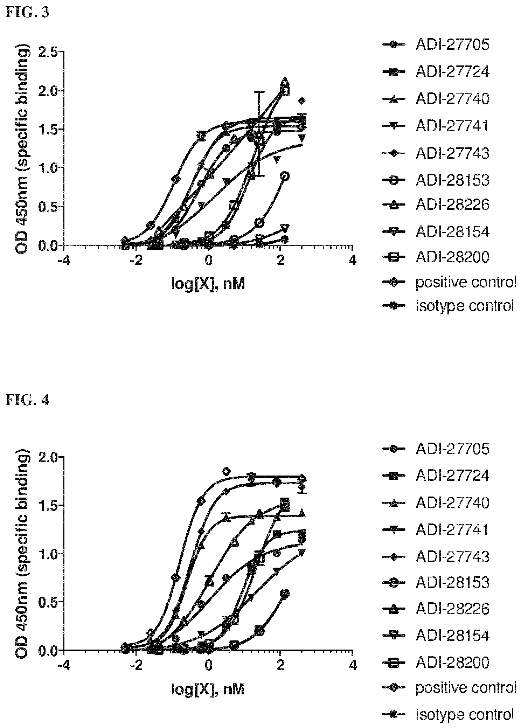

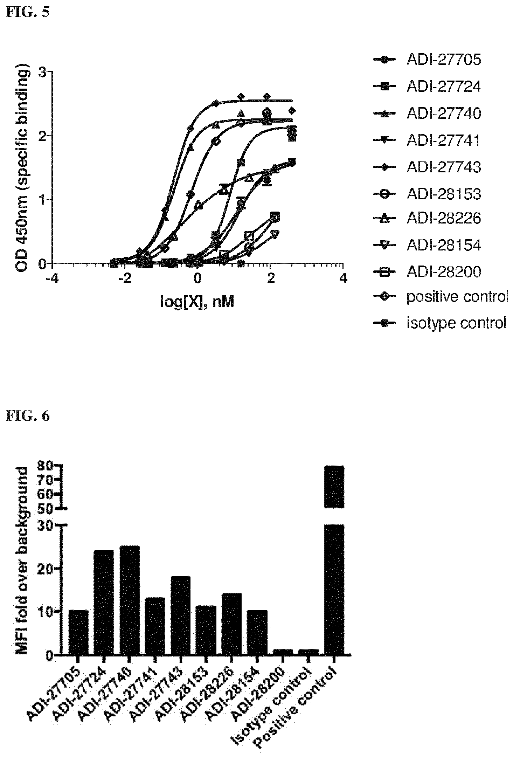

The isotype control showed minimal binding to recombinant NKG2D-Fc proteins, while the positive control bound strongest to the recombinant antigens. NKG2D-binding domains produced by all clones demonstrated binding across human, mouse, and cynomolgus recombinant NKG2D-Fc proteins, although with varying affinities from clone to clone. Generally, each anti-NKG2D clone bound to human (FIG. 3) and cynomolgus (FIG. 4) recombinant NKG2D-Fc with similar affinity, but with lower affinity to mouse (FIG. 5) recombinant NKG2D-Fc.

### NKG2D-binding domains bind to cells expressing NKG2D

EL4 mouse lymphoma cell lines were engineered to express human or mouse NKG2D - CD3 zeta signaling domain chimeric antigen receptors. An NKG2D-binding clone, an isotype control or a positive control was used at a 100 nM concentration to stain extracellular NKG2D expressed on the EL4 cells. The antibody binding was detected using fluorophore-conjugated anti-human IgG secondary antibodies. Cells were analyzed by flow cytometry, and fold-over-background (FOB) was calculated using the mean fluorescence intensity (MFI) of NKG2D expressing cells compared to parental EL4 cells.

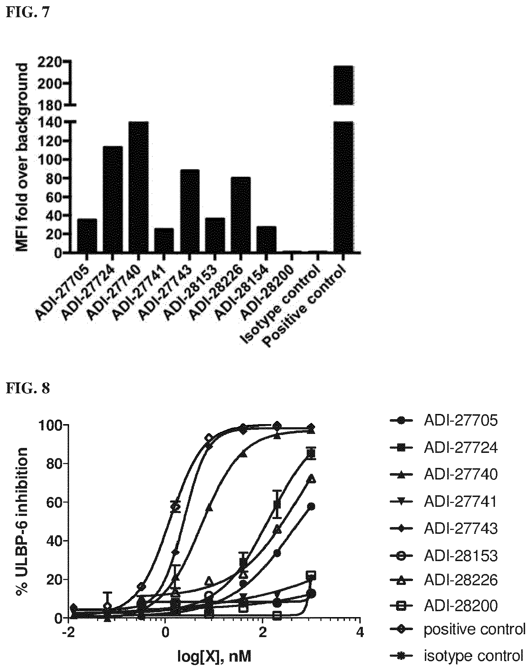

NKG2D-binding domains produced by all clones bound to EL4 cells expressing human and mouse NKG2D. Positive control antibodies (selected from SEQ ID NO: 45-48, or anti-mouse NKG2D clones MI-6 and CX-5 available at eBioscience) gave the best FOB binding signal. The NKG2D-binding affinity for each clone was similar between cells expressing human NKG2D (FIG. 6) and mouse (FIG. 7) NKG2D.

### Example 2 - NKG2D-binding domains block natural ligand binding to NKG2D

### Competition With ULBP-6

Recombinant human NKG2D-Fc proteins were adsorbed to wells of a microplate, and the wells were blocked with bovine serum albumin reduce non-specific binding. A saturating concentration of ULBP-6-His-biotin was added to the wells, followed by addition of the NKG2D-binding domain clones. After a 2-hour incubation, wells were washed and ULBP-6-His-biotin that remained bound to the NKG2D-Fc coated wells was detected by streptavidin-conjugated to horseradish peroxidase and TMB substrate. Absorbance was measured at 450 nM and corrected at 540 nM. After subtracting background, specific binding of NKG2D-binding domains to the NKG2D-Fc proteins was calculated from the percentage of ULBP-6-His-biotin that was blocked from binding to the NKG2D-Fc proteins in wells. The positive control antibody (selected from SEQ ID NOs:47-50) and various NKG2D-binding domains blocked ULBP-6 binding to NKG2D, while isotype control showed little competition with ULBP-6 (FIG. 8).

ULBP-6 sequence is represented by SEQ ID NO:131

MAAAAIPALLLCLPLLFLLFGWSRARRDDPHSLCYDITVIPKFRPGPRWCAVQGQVDEK
TFLHYDCGNKTVTPVSPLGKKLNVTMAWKAQNPVLREVVDILTEQLLDIQLENYTPKE
PLTLQARMSCEQKAEGHSSGSWQFSIDGQTFLLFDSEKRMWTTVHPGARKMKEKWEN

DKDVAMSFHYISMGDCIGWLEDFLMGMDSTLEPSAGAPLAMSSGTTQLRATATTLILC
CLLIILPCFILPGI (SEQ ID NO:131)

### Competition With MICA

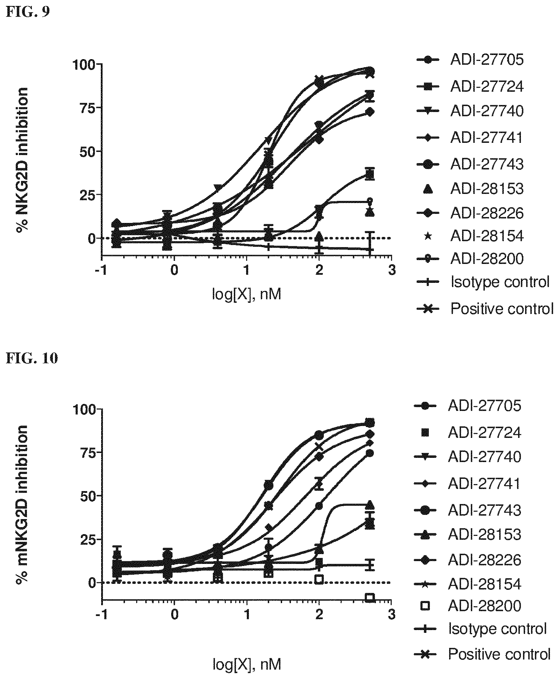

Recombinant human MICA-Fc proteins were adsorbed to wells of a microplate, and the wells were blocked with bovine serum albumin to reduce non-specific binding. NKG2D-Fc-biotin was added to wells followed by NKG2D-binding domains. After incubation and washing, NKG2D-Fc-biotin that remained bound to MICA-Fc coated wells was detected using streptavidin-HRP and TMB substrate. Absorbance was measured at 450 nM and corrected at 540 nM. After subtracting background, specific binding of NKG2D-binding domains to the NKG2D-Fc proteins was calculated from the percentage of NKG2D-Fc-biotin that was blocked from binding to the MICA-Fc coated wells. The positive control antibody (selected from SEQ ID NOs:47-50) and various NKG2D-binding domains blocked MICA binding to NKG2D, while isotype control showed little competition with MICA (FIG. 9).

### Competition With Rae-1 delta

Recombinant mouse Rae-1 delta-Fc (purchased from R&D Systems) was adsorbed to wells of a microplate, and the wells were blocked with bovine serum albumin to reduce non-specific binding. Mouse NKG2D-Fc-biotin was added to the wells followed by NKG2D-binding domains. After incubation and washing, NKG2D-Fc-biotin that remained bound to Rae-1 delta-Fc coated wells was detected using streptavidin-HRP and TMB substrate. Absorbance was measured at 450 nM and corrected at 540 nM. After subtracting background, specific binding of NKG2D-binding domains to the NKG2D-Fc proteins was calculated from the percentage of NKG2D-Fc-biotin that was blocked from binding to the Rae-1 delta-Fc coated wells. The positive control (selected from SEQ ID NOs:47-50, or anti-mouse NKG2D clones MI-6 and CX-5 available at eBioscience) and various NKG2D-binding domain clones blocked Rae-1 delta binding to mouse NKG2D, while the isotype control antibody showed little competition with Rae-1 delta (FIG. 10).

### Example 3 - NKG2D-binding domain clones activate NKG2D

Nucleic acid sequences of human and mouse NKG2D were fused to nucleic acid sequences encoding a CD3 zeta signaling domain to obtain chimeric antigen receptor (CAR) constructs. The NKG2D-CAR constructs were then cloned into a retrovirus vector using Gibson assembly and transfected into expi293 cells for retrovirus production. EL4 cells were infected with viruses containing NKG2D-CAR together with 8 µg/mL polybrene. 24 hours after infection, the expression levels of NKG2D-CAR in the EL4 cells were analyzed by flow cytometry, and clones which express high levels of the NKG2D-CAR on the cell surface were selected.

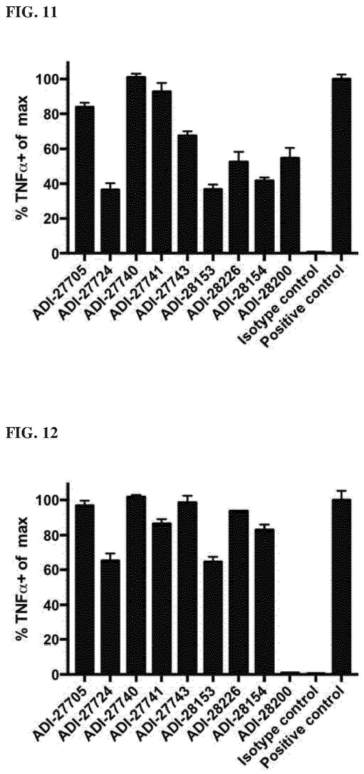

To determine whether NKG2D-binding domains activate NKG2D, they were adsorbed to wells of a microplate, and NKG2D-CAR EL4 cells were cultured on the antibody fragment-coated wells for 4 hours in the presence of brefeldin-A and monensin. Intracellular TNF-alpha production, an indicator for NKG2D activation, was assayed by flow cytometry. The percentage of TNF-alpha positive cells was normalized to the cells treated with the positive control. All NKG2D-binding domains activated both human NKG2D (FIG. 11) and mouse NKG2D (FIG. 12).

### Example 4 - NKG2D-binding domains activate NK cells

### Primary human NK cells

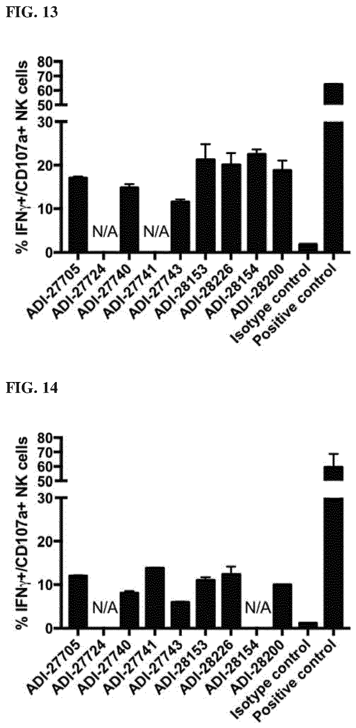

Peripheral blood mononuclear cells (PBMCs) were isolated from human peripheral blood buffy coats using density gradient centrifugation. NK cells (CD3⁻ CD56⁺) were isolated using negative selection with magnetic beads from PBMCs, and the purity of the isolated NK cells was typically >95%. Isolated NK cells were then cultured in media containing 100 ng/mL IL-2 for 24-48 hours before they were transferred to the wells of a microplate to which the NKG2D-binding domains were adsorbed, and cultured in the media containing fluorophore-conjugated anti-CD107a antibody, brefeldin-A, and monensin. Following culture, NK cells were assayed by flow cytometry using fluorophore-conjugated antibodies against CD3, CD56 and IFN-γ. CD107a and IFN-γ staining were analyzed in CD3⁻ CD56⁺ cells to assess NK cell activation. The increase in CD107a/IFN-γ double-positive cells is indicative of better NK cell activation through engagement of two activating receptors rather than one receptor. NKG2D-binding domains and the positive control (selected from SEQ ID NOs:47-50) showed a higher percentage of NK cells becoming CD107a⁺ and IFN-γ⁺ than the isotype control (FIG. 13 & FIG. 14 represent data from two independent experiments, each using a different donor's PBMC for NK cell preparation).

### Primary mouse NK cells

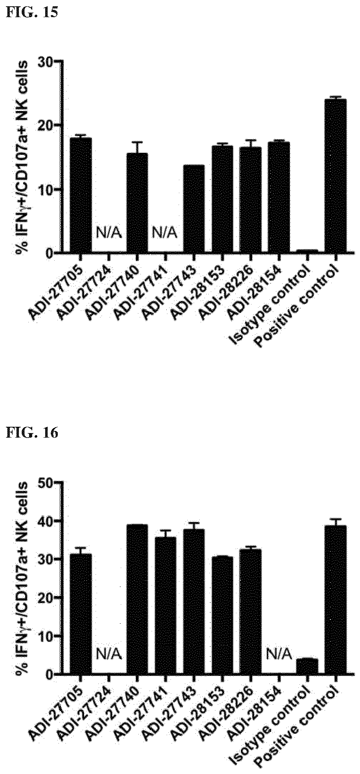

Spleens were obtained from C57B1/6 mice and crushed through a 70 µm cell strainer to obtain single cell suspension. Cells were pelleted and resuspended in ACK lysis buffer (purchased from Thermo Fisher Scientific #A1049201; 155 mM ammonium chloride, 10 mM potassium bicarbonate, 0.01 mM EDTA) to remove red blood cells. The remaining cells were cultured with 100 ng/mL hIL-2 for 72 hours before being harvested and prepared for NK cell isolation. NK cells (CD3⁻NK1.1⁺) were then isolated from spleen cells using a negative depletion technique with magnetic beads with typically >90% purity. Purified NK cells were cultured in media containing 100 ng/mL mIL-15 for 48 hours before they were transferred to the wells of a microplate to which the NKG2D-binding domains were adsorbed, and cultured in the media containing fluorophore-conjugated anti-CD107a antibody, brefeldin-A, and monensin. Following culture in NKG2D-binding domain-coated wells, NK cells were assayed by flow cytometry using fluorophore-conjugated antibodies against CD3, NK1.1 and IFN-γ. CD107a and IFN-γ staining were analyzed in CD3⁻NK1.1⁺ cells to assess NK cell activation. The increase in CD107a/IFN-γ double-positive cells is indicative of better NK cell activation through engagement of two activating receptors rather than one receptor. NKG2D-binding domains and the positive control (selected from anti-mouse NKG2D clones MI-6 and CX-5 available at eBioscience) showed a higher percentage of NK cells becoming CD107a⁺ and IFN-γ⁺ than the isotype control (FIG. 15 & FIG. 16 represent data from two independent experiments, each using a different mouse for NK cell preparation).

### Example 5 - NKG2D-binding domains enable cytotoxicity of target tumor cells

Human and mouse primary NK cell activation assays demonstrate increased cytotoxicity markers on NK cells after incubation with NKG2D-binding domains. To address whether this translates into increased tumor cell lysis, a cell-based assay was utilized where each NKG2D-binding domain was developed into a monospecific antibody. The Fc region was used as one targeting arm, while the Fab region (NKG2D-binding domain) acted as another targeting arm to activate NK cells. THP-1 cells, which are of human origin and express high levels of Fc receptors, were used as a tumor target and a Perkin Elmer DELFIA Cytotoxicity Kit was used. THP-1 cells were labeled with BATDA reagent, and resuspended at 10⁵/mL in culture media. Labeled THP-1 cells were then combined with NKG2D antibodies and isolated mouse NK cells in wells of a microtiter plate at 37 °C for 3 hours. After incubation, 20 µl of the culture supernatant was removed, mixed with 200 µl of Europium solution and incubated with shaking for 15 minutes in the dark. Fluorescence was measured over time by a PheraStar plate reader equipped with a time-resolved fluorescence module (Excitation 337 nm, Emission 620nm) and specific lysis was calculated according to the kit instructions.

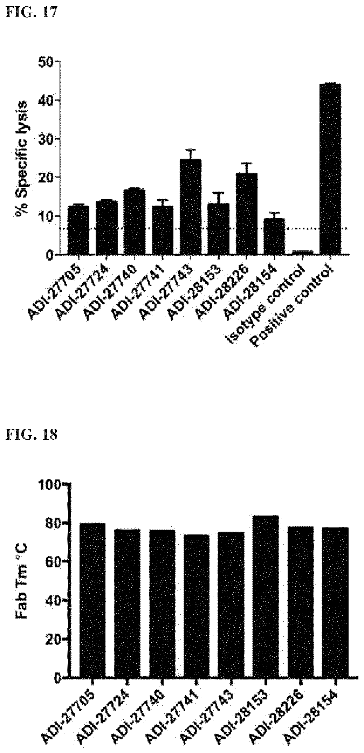

The positive control, ULBP-6 - a natural ligand for NKG2D, showed increased specific lysis of THP-1 target cells by mouse NK cells. NKG2D antibodies also increased specific lysis of THP-1 target cells, while isotype control antibody showed reduced specific lysis. The dotted line indicates specific lysis of THP-1 cells by mouse NK cells without antibody added (FIG. 17).

### Example 6 - NKG2D antibodies show high thermostability

Melting temperatures of NKG2D-binding domains were assayed using differential scanning fluorimetry. The extrapolated apparent melting temperatures are high relative to typical IgG1 antibodies (FIG. 18).

### Example 7 - Synergistic activation of human NK cells by cross-linking NKG2D and CD16

### Primary human NK cell activation assay

Peripheral blood mononuclear cells (PBMCs) were isolated from peripheral human blood buffy coats using density gradient centrifugation. NK cells were purified from PBMCs using negative magnetic beads (StemCell # 17955). NK cells were >90% CD3⁻CD56⁺ as determined by flow cytometry. Cells were then expanded 48 hours in media containing 100 ng/mL hIL-2 (Peprotech #200-02) before use in activation assays. Antibodies were coated onto a 96-well flat-bottom plate at a concentration of 2 µg/ml (anti-CD16, Biolegend # 302013) and 5 µg/mL (anti-NKG2D, R&D #MAB139) in 100 µl sterile PBS overnight at 4 °C followed by washing the wells thoroughly to remove excess antibody. For the assessment of degranulation IL-2-activated NK cells were resuspended at 5×10⁵ cells/ml in culture media supplemented with 100 ng/mL hIL2 and 1 µg/mL APC-conjugated anti-CD107a mAb (Biolegend # 328619). 1×10⁵ cells/well were then added onto antibody coated plates. The protein transport inhibitors Brefeldin A (BFA, Biolegend # 420601) and Monensin (Biolegend # 420701) were added at a final dilution of 1:1000 and 1:270 respectively. Plated cells were incubated for 4 hours at 37 °C in 5% CO₂. For intracellular staining of IFN-γ NK cells were labeled with anti-CD3 (Biolegend #300452) and anti-CD56 mAb (Biolegend # 318328) and subsequently fixed and permeabilized and labeled with anti-IFN-y mAb (Biolegend # 506507). NK cells were analyzed for expression of CD107a and IFN-γ by flow cytometry after gating on live CD56⁺CD3⁻cells.

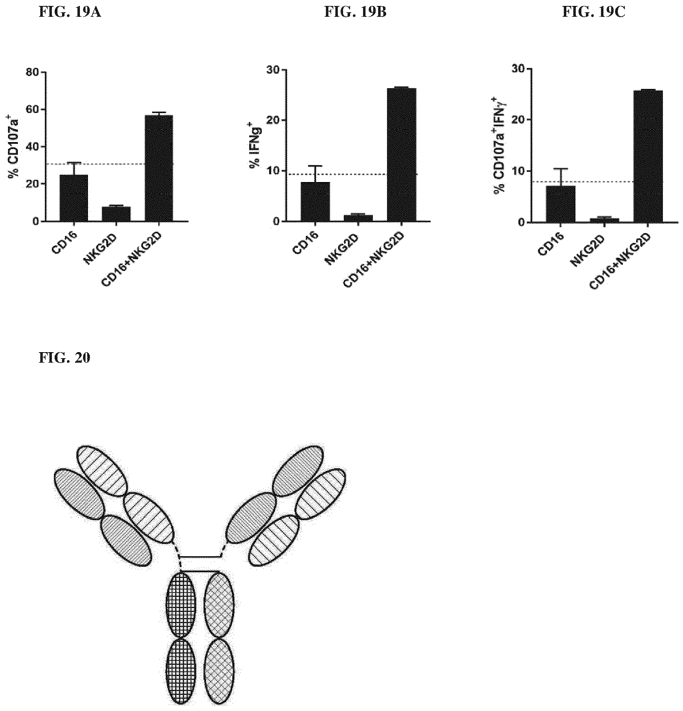

To investigate the relative potency of receptor combination, crosslinking of NKG2D or CD16 and co-crosslinking of both receptors by plate-bound stimulation was performed. As shown in Figure 19 (FIGs. 19A-19C), combined stimulation of CD16 and NKG2D resulted in highly elevated levels of CD107a (degranulation) (FIG. 19A) and/or IFN-γ production (FIG. 19B). Dotted lines represent an additive effect of individual stimulations of each receptor.

CD107a levels and intracellular IFN-γ production of IL-2-activated NK cells were analyzed after 4 hours of plate-bound stimulation with anti-CD16, anti-NKG2D or a combination of both monoclonal antibodies. Graphs indicate the mean (n = 2) ± SD. FIG. 19A demonstrates levels of CD107a; FIG. 19B demonstrates levels of IFNγ; FIG. 19C demonstrates levels of CD107a and IFNγ. Data shown in FIGs. 19A-19C are representative of five independent experiments using five different healthy donors.

### Example 8 - Multi-specific binding proteins bind to NKG2D

EL4 mouse lymphoma cell lines were engineered to express human NKG2D. Trispecific binding proteins (TriNKETs) that each contain an NKG2D-binding domain, a tumor-associated antigen-binding domain (CAIX-binding domain), and an Fc domain that binds to CD 16 as shown in FIG. 1, were tested for their affinity to extracellular NKG2D expressed on EL4 cells. The binding of the multi-specific binding proteins to NKG2D was detected using fluorophore-conjugated anti-human IgG secondary antibodies. Cells were analyzed by flow cytometry, and fold-over-background (FOB) was calculated using the mean fluorescence intensity (MFI) of NKG2D-expressing cells compared to parental EL4 cells.

TriNKETs tested include A44-TriNKET-CAIX (ADI-27744 and a CAIX-binding domain), F63-TriNKET-CAIX (ADI-29404 and a CD33-binding domain), E79-TriNKET-CAIX (ADI-29379 and a CAIX-binding domain), F47-TriNKET-CAIX (ADI-29447 and a CAIX-binding domain), and A49-TriNKET-CAIX (ADI-27749 and a CAIX-binding domain). The CAIX-binding domain was composed of a heavy chain variable domain of SEQ ID NO:80, and light chain variable domain of SEQ ID NO:81.

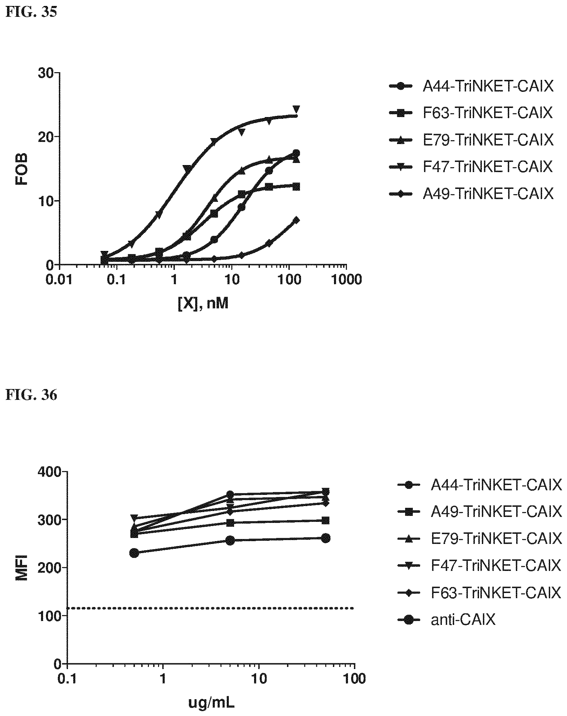

FIG. 35 shows that TriNKETs, each of which includes a CAIX-binding domain and a distinct NKG2D-binding domain, bind to NKG2D expressed on EL4 cells.

### Example 9 - Multi-specific binding proteins bind to human tumor antigens

### Trispecific binding proteins bind to CAIX

Human renal cell carcinoma line A498 expressing CAIX was used to assay the binding of TriNKETs to the tumor associated antigen CAIX. TriNKETs and optionally the parental anti-CAIX monoclonal antibody were incubated with the cells, and the binding was detected using fluorophore-conjugated anti-human IgG secondary antibodies. Cells were analyzed by flow cytometry, and fold-over-background (FOB) was calculated using the mean fluorescence intensity (MFI) from the TriNKETs and the parental monoclonal anti-CAIX antibody normalized to secondary antibody controls. CAIX-targeting TriNKETs show comparable levels of binding to CAIX on A498 cells as compared with the parental anti-CAIX antibody (FIG. 36). The overall binding signal is comparable among TriNKETs as they contain the same CAIX binding domain.

### Example 10 - Trispecific binding proteins enable cytotoxicity of target cancer cells

Peripheral blood mononuclear cells (PBMCs) were isolated from human peripheral blood buffy coats using density gradient centrifugation. NK cells (CD3⁻ CD56⁺) were isolated using negative selection with magnetic beads from PBMCs, and the purity of the isolated NK cells was typically >90%. Isolated NK cells were cultured in media containing 100 ng/mL IL-2 for activation or rested overnight without cytokine. IL-2-activated or rested NK cells were used the following day in cytotoxicity assays.

NK/NK T-cell lines KHYG-1 cells were maintained in 10% HI-FBS-RPMI-1640 with 10 ng/mL IL-2. The day before use as effector cells, KHYG-1 cells were harvested from culture, and cells were washed out of the IL-2 containing media. After washing KHYG-1 cells were resuspended in 10% HI-FBS-RPMI-1640, and were rested overnight without cytokine.

### DELFIA cytotoxicity assay:

Human A498 cancer cells expressing CAIX were harvested from culture; cells were washed with PBS and were resuspended in growth media at 10⁶/mL for labeling with BATDA reagent (Perkin Elmer AD0116). Manufacturer instructions were followed for labeling of the target cells. After labeling cells were washed 3x with PBS, and were resuspended at 0.5-1.0x10⁵/mL in culture media. To prepare the background wells an aliquot of the labeled cells was put aside, and the cells were spun out of the media. 100 µl of the media was carefully added to wells in triplicate to avoid disturbing the pelleted cells. 100 µl of BATDA-labeled cells were added to each well of the 96-well plate. Wells were saved for spontaneous release from target cells, and wells were prepared for max lysis of target cells by addition of 1% Triton-X. TriNKETs against the tumor target of interest were diluted in culture media, 50 µl of diluted TriNKET was added to each well. Rested NK cells, activated NK cells or KHYG-1 cells were harvested from culture, washed, and resuspended at 10⁵-2.0x10⁶/mL in culture media depending on the desired effector to target cell ratio (E:T). 50 µl of NK cells was added to each well of the plate to make a total of 200 µl culture volume. The plate was incubated at 37 °C with 5% CO2 for 2-3 hours before developing the assay.

After culturing for 2-3 hours, the plate was removed from the incubator and the cells were pelleted by centrifugation at 200g for 5 minutes. 20 µl of culture supernatant was transferred to a clean microplate provided from the manufacturer and 200 µl of room temperature europium solution was added to each well. The plate was protected from the light and incubated on a plate shaker at 250 rpm for 15 minutes. The plate was read using either Victor 3 or SpectraMax i3X instruments. % Specific lysis was calculated as follows: % Specific lysis = ((Experimental release - Spontaneous release) / (Maximum release - Spontaneous release)) ^{∗} 100%.

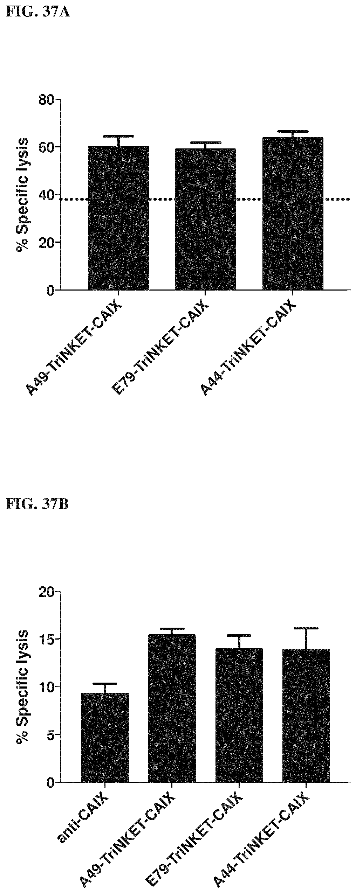

TriNKETs mediated cytotoxicity of human NK cells towards the CAIX-positive human cancer cells. Co-culture of rested KHYG-1 cells with A498 target cells resulted in ∼38% specific lysis tumor lysis (shown by the dotted line in FIG. 37A). Addition of three different TriNKETs targeting CAIX (A49-TriNKET-CAIX; E79-TriNKET-CAIX; and A44-TriNKET-CAIX) to the co-culture enhanced specific lysis of tumor cells by about 20% for all three NKG2D binding domains (FIG. 37A).

Rested human NK cells were mixed with A498 cancer cells. Rested human NK cells had no cytotoxic activity against A498 cells without CAIX mAb or TriNKETs added. A monoclonal antibody against CAIX (BAY79-4620 variable domains with hIgG1 as mAb (homodimer) was able to increase specific lysis to about 9%. TriNKETs (e.g., A49-TriNKET-CAIX, E79-TriNKET-CAIX and A44-TriNKET-CAIX) enhanced cytotoxic activity of rested human NK cells towards the cancer cells compared to the monoclonal antibody (FIG. 37B).

### INCORPORATION BY REFERENCE

The entire disclosure of each of the patent documents and scientific articles referred to herein is incorporated by reference for all purposes.

### EQUIVALENTS

The invention may be embodied in other specific forms without departing from the spirit or essential characteristics thereof. The foregoing embodiments are therefore to be considered in all respects illustrative rather than limiting the invention described herein. Scope of the invention is thus indicated by the appended claims rather than by the foregoing description, and all changes that come within the meaning and range of equivalency of the claims are intended to be embraced therein.

### Embodiments of the invention

1. A protein comprising:
   (a) a first antigen-binding site that binds NKG2D;
   (b) a second antigen-binding site that binds CAIX, ANO1, mesothelin, TROP2, CEA or Claudin-18.2; and
   (c) an antibody Fc domain or a portion thereof sufficient to bind CD16, or a third antigen-binding site that binds CD16.
2. The protein of embodiment 1, wherein the first antigen-binding site binds to NKG2D in humans, non-human primates, and rodents.
3. The protein of embodiment 1 or 2, wherein the first antigen-binding site comprises a heavy chain variable domain and a light chain variable domain.
4. The protein according to embodiment 3, wherein the heavy chain variable domain and the light chain variable domain are present on the same polypeptide.
5. The protein according to embodiments 3 or 4, wherein the second antigen-binding site comprises a heavy chain variable domain and a light chain variable domain.
6. The protein according to embodiment 5, wherein the heavy chain variable domain and the light chain variable domain of the second antigen-binding site are present on the same polypeptide.
7. The protein according to embodiment 5 or 6, wherein the light chain variable domain of the first antigen-binding site has an amino acid sequence identical to the amino acid sequence of the light chain variable domain of the second antigen-binding site.
8. A protein according to any one of the preceding embodiments, wherein the first antigen-binding site comprises a heavy chain variable domain at least 90% identical to SEQ ID NO:1.
9. The protein according to any one of embodiments 1-7, wherein the first antigen-binding site comprises a heavy chain variable domain at least 90% identical to SEQ ID NO:41 and a light chain variable domain at least 90% identical to SEQ ID NO:42.
10. The protein according to any one of embodiments 1-7, wherein the first antigen-binding site comprises a heavy chain variable domain at least 90% identical to SEQ ID NO:43 and a light chain variable domain at least 90% identical to SEQ ID NO:44.
11. The protein according to any one of embodiments 1-7, wherein the first antigen-binding site comprises a heavy chain variable domain at least 90% identical to SEQ ID NO:45 and a light chain variable domain at least 90% identical to SEQ ID NO:46.
12. The protein according to any one of embodiments 1-7, wherein the first antigen-binding site comprises a heavy chain variable domain at least 90% identical to SEQ ID NO:47 and a light chain variable domain at least 90% identical to SEQ ID NO:48.
13. The protein according to any one of embodiments 1-7, wherein the first antigen-binding site comprises a heavy chain variable domain at least 90% identical to SEQ ID NO:49 and a light chain variable domain at least 90% identical to SEQ ID NO:50.
14. The protein according to any one of embodiments 1-7, wherein the first antigen-binding site comprises a heavy chain variable domain at least 90% identical to SEQ ID NO:132 and a light chain variable domain at least 90% identical to SEQ ID NO:133.
15. The protein according to any one of embodiments 1-7, wherein the first antigen-binding site comprises a heavy chain variable domain at least 90% identical to SEQ ID NO:140 and a light chain variable domain at least 90% identical to SEQ ID NO:141.
16. The protein according to any one of embodiments 1-7, wherein the first antigen-binding site comprises a heavy chain variable domain at least 90% identical to SEQ ID NO:148 and a light chain variable domain at least 90% identical to SEQ ID NO:149.
17. The protein according to any one of embodiments 1-7, wherein the first antigen-binding site comprises a heavy chain variable domain at least 90% identical to SEQ ID NO:156 and a light chain variable domain at least 90% identical to SEQ ID NO:157.
18. The protein according to any one of embodiments 1-7, wherein the first antigen-binding site comprises a heavy chain variable domain at least 90% identical to SEQ ID NO:164 and a light chain variable domain at least 90% identical to SEQ ID NO:165.
19. The protein of embodiment 1 or 2, wherein the first antigen-binding site is a single-domain antibody.
20. The protein of embodiment 19, wherein the single-domain antibody is a V_{H}H fragment or a V_{NAR} fragment.
21. The protein according to any one of embodiments 1-2 or 19-20, wherein the second antigen-binding site comprises a heavy chain variable domain and a light chain variable domain.
22. The protein according to embodiment 21, wherein the heavy chain variable domain and the light chain variable domain of the second antigen-binding site are present on the same polypeptide.
23. The protein according to any of the preceding embodiments, wherein the second antigen-binding site binds CAIX, the heavy chain variable domain of the second antigen-binding site comprises an amino acid sequence at least 90% identical to SEQ ID NO:72 and the light chain variable domain of the second antigen-binding site comprises an amino acid sequence at least 90% identical to SEQ ID NO:73.
24. The protein according to any of the preceding embodiments, wherein the second antigen-binding site binds CAIX, the heavy chain variable domain of the second antigen-binding site comprises an amino acid sequence including:
   a heavy chain CDR1 sequence identical to the amino acid sequence of SEQ ID NO:74;
   a heavy chain CDR2 sequence identical to the amino acid sequence of SEQ ID NO:75; and
   a heavy chain CDR3 sequence identical to the amino acid sequence of SEQ ID NO:76.
25. The protein according to embodiment 24, wherein the light chain variable domain of the second antigen-binding site comprises an amino acid sequence including:
   a light chain CDR1 sequence identical to the amino acid sequence of SEQ ID NO:77;
   a light chain CDR2 sequence identical to the amino acid sequence of SEQ ID NO:78; and
   a light chain CDR3 sequence identical to the amino acid sequence of SEQ ID NO:79.
26. The protein according to any one of embodiments 1-22, wherein the second antigen-binding site binds CAIX, the heavy chain variable domain of the second antigen-binding site comprises an amino acid sequence at least 90% identical to SEQ ID NO:80 and the light chain variable domain of the second antigen-binding site comprises an amino acid sequence at least 90% identical to SEQ ID NO:81.
27. The protein according to any one of embodiments 1-22 or 26, wherein the second antigen-binding site binds CAIX, the heavy chain variable domain of the second antigen-binding site comprises an amino acid sequence including:
   a heavy chain CDR1 sequence identical to the amino acid sequence of SEQ ID NO:82;
   a heavy chain CDR2 sequence identical to the amino acid sequence of SEQ ID NO:83; and
   a heavy chain CDR3 sequence identical to the amino acid sequence of SEQ ID NO:84.
28. The protein according to embodiment 27, wherein the light chain variable domain of the second antigen-binding site comprises an amino acid sequence including:
   a light chain CDR1 sequence identical to the amino acid sequence of SEQ ID NO:85;
   a light chain CDR2 sequence identical to the amino acid sequence of SEQ ID NO:86; and
   a light chain CDR3 sequence identical to the amino acid sequence of SEQ ID NO:87.
29. The protein according to any one of embodiments 1-22, wherein the second antigen-binding site binds CAIX, the heavy chain variable domain of the second antigen-binding site comprises an amino acid sequence at least 90% identical to SEQ ID NO:88 and the light chain variable domain of the second antigen-binding site comprises an amino acid sequence at least 90% identical to SEQ ID NO:89.
30. The protein according to any one of embodiments 1-22 or 29, wherein the second antigen-binding site binds CAIX, the heavy chain variable domain of the second antigen-binding site comprises an amino acid sequence including:
   a heavy chain CDR1 sequence identical to the amino acid sequence of SEQ ID NO:90;
   a heavy chain CDR2 sequence identical to the amino acid sequence of SEQ ID NO:91; and
   a heavy chain CDR3 sequence identical to the amino acid sequence of SEQ ID NO:92.
31. The protein according to embodiment 30, wherein the light chain variable domain of the second antigen-binding site comprises an amino acid sequence including:
   a light chain CDR1 sequence identical to the amino acid sequence of SEQ ID NO:93;
   a light chain CDR2 sequence identical to the amino acid sequence of SEQ ID NO:94; and
   a light chain CDR3 sequence identical to the amino acid sequence of SEQ ID NO:95.
32. The protein according to any one of embodiments 1-22, wherein the second antigen-binding site binds ANO1, the heavy chain variable domain of the second antigen-binding site comprises an amino acid sequence at least 90% identical to SEQ ID NO:97 and the light chain variable domain of the second antigen-binding site comprises an amino acid sequence at least 90% identical to SEQ ID NO:98.
33. The protein according to any one of embodiments 1-22, wherein the second antigen-binding site binds mesothelin, the heavy chain variable domain of the second antigen-binding site comprises an amino acid sequence at least 90% identical to SEQ ID NO:106 and the light chain variable domain of the second antigen-binding site comprises an amino acid sequence at least 90% identical to SEQ ID NO:107.
34. The protein according to any one of embodiments 1-22, wherein the second antigen-binding site binds mesothelin, the heavy chain variable domain of the second antigen-binding site comprises an amino acid sequence at least 90% identical to SEQ ID NO:114 and the light chain variable domain of the second antigen-binding site comprises an amino acid sequence at least 90% identical to SEQ ID NO:115.
35. The protein according to any one of embodiments 1-22, wherein the second antigen-binding site binds mesothelin, the heavy chain variable domain of the second antigen-binding site comprises an amino acid sequence at least 90% identical to SEQ ID NO:122 and the light chain variable domain of the second antigen-binding site comprises an amino acid sequence at least 90% identical to SEQ ID NO:123.
36. The protein according to any one of embodiments 1-22, wherein the second antigen-binding site binds TROP2, the heavy chain variable domain of the second antigen-binding site comprises an amino acid sequence at least 90% identical to SEQ ID NO:172 and the light chain variable domain of the second antigen-binding site comprises an amino acid sequence at least 90% identical to SEQ ID NO:173
37. The protein according to any one of embodiments 1-22, wherein the second antigen-binding site binds TROP2, the heavy chain variable domain of the second antigen-binding site comprises an amino acid sequence at least 90% identical to SEQ ID NO:180 and the light chain variable domain of the second antigen-binding site comprises an amino acid sequence at least 90% identical to SEQ ID NO:181.
38. The protein according to any one of embodiments 1-22, wherein the second antigen-binding site binds TROP2, the heavy chain variable domain of the second antigen-binding site comprises an amino acid sequence at least 90% identical to SEQ ID NO:188 and the light chain variable domain of the second antigen-binding site comprises an amino acid sequence at least 90% identical to SEQ ID NO:189.
39. The protein according to any one of embodiments 1-22, wherein the second antigen-binding site binds TROP2, the heavy chain variable domain of the second antigen-binding site comprises an amino acid sequence at least 90% identical to SEQ ID NO:190 and the light chain variable domain of the second antigen-binding site comprises an amino acid sequence at least 90% identical to SEQ ID NO:191.
40. The protein according to any one of embodiments 1-22, wherein the second antigen-binding site binds TROP2, the heavy chain variable domain of the second antigen-binding site comprises an amino acid sequence at least 90% identical to SEQ ID NO:192 and the light chain variable domain of the second antigen-binding site comprises an amino acid sequence at least 90% identical to SEQ ID NO:193.
41. The protein according to any one of embodiments 1-22, wherein the second antigen-binding site binds CEA, the heavy chain variable domain of the second antigen-binding site comprises an amino acid sequence at least 90% identical to SEQ ID NO:195 and the light chain variable domain of the second antigen-binding site comprises an amino acid sequence at least 90% identical to SEQ ID NO:196.
42. The protein according to any one of embodiments 1-22, wherein the second antigen-binding site binds CEA, the heavy chain variable domain of the second antigen-binding site comprises an amino acid sequence at least 90% identical to SEQ ID NO:203 and the light chain variable domain of the second antigen-binding site comprises an amino acid sequence at least 90% identical to SEQ ID NO:204.
43. The protein according to any one of embodiments 1-22, wherein the second antigen-binding site binds CEA, the heavy chain variable domain of the second antigen-binding site comprises an amino acid sequence at least 90% identical to SEQ ID NO:211 and the light chain variable domain of the second antigen-binding site comprises an amino acid sequence at least 90% identical to SEQ ID NO:212.
44. The protein according to any one of embodiments 1-22, wherein the second antigen-binding site binds claudin-18.2, the heavy chain variable domain of the second antigen-binding site comprises an amino acid sequence at least 90% identical to SEQ ID NO:220 and the light chain variable domain of the second antigen-binding site comprises an amino acid sequence at least 90% identical to SEQ ID NO:221.
45. The protein according to any one of embodiments 1-22, wherein the second antigen-binding site binds claudin-18.2, the heavy chain variable domain of the second antigen-binding site comprises an amino acid sequence at least 90% identical to SEQ ID NO:228 and the light chain variable domain of the second antigen-binding site comprises an amino acid sequence at least 90% identical to SEQ ID NO:229.
46. A protein according to any one of embodiments 1-4 or 8-20, wherein the second antigen-binding site is a single-domain antibody.
47. The protein of embodiment 46, wherein the second antigen-binding site is a V_{H}H fragment or a V_{NAR} fragment.
48. A protein according to any one of the preceding embodiments, wherein the protein comprises a portion of an antibody Fc domain sufficient to bind CD16, wherein the antibody Fc domain comprises hinge and CH2 domains.
49. A protein according to embodiment 48, wherein the antibody Fc domain comprises hinge and CH2 domains of a human IgG1 antibody.
50. A protein according to embodiment 48 or 49, wherein the Fc domain comprises an amino acid sequence at least 90% identical to amino acids 234-332 of a human IgG1 antibody.
51. A protein according to any one of embodiments 48-50, wherein the Fc domain comprises amino acid sequence at least 90% identical to the Fc domain of human IgG1 and differs at one or more positions selected from the group consisting of Q347, Y349, L351, S354, E356, E357, K360, Q362, S364, T366, L368, K370, N390, K392, T394, D399, S400, D401, F405, Y407, K409, T411, K439.
52. A formulation comprising a protein according to any one of the preceding embodiments and a pharmaceutically acceptable carrier.
53. A cell comprising one or more nucleic acids expressing a protein according to any one of embodiments 1-51.
54. A method of directly and/or indirectly enhancing tumor cell death, the method comprising exposing a tumor and natural killer cells to a protein according to any one of embodiments 1-51.
55. A method of treating cancer, wherein the method comprises administering a protein according to any one of embodiments 1-51 or a formulation according to embodiment 52 to a patient.
56. The method of embodiment 55, wherein the second antigen binding site of the protein binds CAIX, the cancer to be treated is selected from the group consisting of renal cell carcinoma, breast cancer, glioblastoma, head and neck cancer, gastric cancers, bladder cancer, ovarian cancer, tumors of the esophagus, lung, colon, kidney, cervix and non-small cell lung carcinoma.
57. The method of embodiment 55, wherein the second antigen binding site of the protein binds mesothelin, the cancer to be treated is selected from the group consisting of mesothelioma, ovarian cancer, pancreatic cancer, non-small cell lung cancer, breast cancer, cholangiocarcinoma, gastric cancer, uterine serous carcinoma, thymic carcinoma, and acute myeloid leukemia.
58. The method of embodiment 55, wherein the second antigen binding site of the protein binds ANO1, the cancer to be treated is selected from the group consisting of esophageal squamous cell cancer (ESCC), gastrointestinal stromal tumor (GIST), head and neck squamous cell carcinoma (HNSCC), pancreatic cancer, breast cancer, prostate cancer, and sarcoma.
59. The method of embodiment 55, wherein the second antigen binding site of the protein binds TROP2, the cancer to be treated is selected from the group consisting of breast, lung, gastric, colorectal, pancreatic, prostatic, cervical, head-and-neck cancer, nasopharyngeal carcinoma, and ovarian carcinoma.
60. The method of embodiment 55, wherein the second antigen binding site of the protein binds CEA, the cancer to be treated is selected from the group consisting of gastrointestinal cancer, colorectal cancer, pancreatic cancer, non-small cell lung cancer and breast cancer.
61. The method of embodiment 55, wherein the second antigen binding site of the protein binds claudin-18.2, the cancer to be treated is selected from the group consisting of esophageal cancer, non-small cell lung carcinoma, ovarian cancer, colon cancer, and several forms of biliary ductal carcinoma.

## Claims

1. A protein comprising:
(a) a first antigen-binding site that binds NKG2D;
(b) a second antigen-binding site that binds CAIX, ANO1, mesothelin, TROP2, or Claudin-18.2; and
(c) an antibody Fc domain or a portion thereof sufficient to bind CD16, or a third antigen-binding site that binds CD16.

2. The protein of claim 1, wherein the first antigen-binding site binds to NKG2D in humans and non-human primates.

3. The protein of claim 1 or 2, wherein the first antigen-binding site comprises a heavy chain variable domain and a light chain variable domain.

4. The protein according to claim 3, wherein the heavy chain variable domain and the light chain variable domain are present on the same polypeptide.

5. The protein according to claims 3 or 4, wherein the second antigen-binding site comprises a heavy chain variable domain and a light chain variable domain.

6. The protein according to claim 5, wherein the heavy chain variable domain and the light chain variable domain of the second antigen-binding site are present on the same polypeptide.

7. The protein according to claim 5 or 6, wherein the light chain variable domain of the first antigen-binding site has an amino acid sequence identical to the amino acid sequence of the light chain variable domain of the second antigen-binding site.

8. A protein according to any one of the preceding claims, wherein the first antigen-binding site comprises a heavy chain variable domain at least 90% identical to SEQ ID NO:1.

9. The protein according to any one of claims 1-7, wherein
a) the first antigen-binding site comprises a heavy chain variable domain at least 90% identical to SEQ ID NO:41 and a light chain variable domain at least 90% identical to SEQ ID NO:42,
b) the first antigen-binding site comprises a heavy chain variable domain at least 90% identical to SEQ ID NO:43 and a light chain variable domain at least 90% identical to SEQ ID NO:44,
c) the first antigen-binding site comprises a heavy chain variable domain at least 90% identical to SEQ ID NO:45 and a light chain variable domain at least 90% identical to SEQ ID NO:46,
d) the first antigen-binding site comprises a heavy chain variable domain at least 90% identical to SEQ ID NO:47 and a light chain variable domain at least 90% identical to SEQ ID NO:48,
e) the first antigen-binding site comprises a heavy chain variable domain at least 90% identical to SEQ ID NO:49 and a light chain variable domain at least 90% identical to SEQ ID NO:50,
f) the first antigen-binding site comprises a heavy chain variable domain at least 90% identical to SEQ ID NO:132 and a light chain variable domain at least 90% identical to SEQ ID NO:133,
g) the first antigen-binding site comprises a heavy chain variable domain at least 90% identical to SEQ ID NO:140 and a light chain variable domain at least 90% identical to SEQ ID NO:141.
h) the first antigen-binding site comprises a heavy chain variable domain at least 90% identical to SEQ ID NO:148 and a light chain variable domain at least 90% identical to SEQ ID NO:149,
i) the first antigen-binding site comprises a heavy chain variable domain at least 90% identical to SEQ ID NO:156 and a light chain variable domain at least 90% identical to SEQ ID NO:157,
or
j) the first antigen-binding site comprises a heavy chain variable domain at least 90% identical to SEQ ID NO:164 and a light chain variable domain at least 90% identical to SEQ ID NO:165.

10. The protein of claim 1 or 2, wherein the first antigen-binding site is a single-domain antibody, optionally wherein the single-domain antibody is a V_{H}H fragment or a V_{NAR} fragment.

11. The protein according to any one of claims 1-2 or 10, wherein the second antigen-binding site comprises a heavy chain variable domain and a light chain variable domain, optionally wherein the heavy chain variable domain and the light chain variable domain of the second antigen-binding site are present on the same polypeptide.

12. The protein according to any of the preceding claims, wherein the second antigen-binding site binds CAIX and comprises a heavy chain variable domain comprising an amino acid sequence at least 90% identical to SEQ ID NO:72 and a light chain variable domain comprising an amino acid sequence at least 90% identical to SEQ ID NO:73.

13. The protein according to any of the preceding claims, wherein the second antigen-binding site binds CAIX and comprises:
a heavy chain CDR1 sequence identical to the amino acid sequence of SEQ ID NO:74;
a heavy chain CDR2 sequence identical to the amino acid sequence of SEQ ID NO:75; and
a heavy chain CDR3 sequence identical to the amino acid sequence of SEQ ID NO:76.

14. The protein according to claim 13, wherein the second antigen-binding site comprises:
a light chain CDR1 sequence identical to the amino acid sequence of SEQ ID NO:77;
a light chain CDR2 sequence identical to the amino acid sequence of SEQ ID NO:78; and
a light chain CDR3 sequence identical to the amino acid sequence of SEQ ID NO:79.

15. The protein according to any one of claims 1-11, wherein the second antigen-binding site binds CAIX and comprises a heavy chain variable domain of comprising an amino acid sequence at least 90% identical to SEQ ID NO:80 and a light chain variable domain comprising an amino acid sequence at least 90% identical to SEQ ID NO:81.

16. The protein according to any one of claims 1-11 or 15, wherein the second antigen-binding site binds CAIX and comprises:
a heavy chain CDR1 sequence identical to the amino acid sequence of SEQ ID NO:82;
a heavy chain CDR2 sequence identical to the amino acid sequence of SEQ ID NO:83; and
a heavy chain CDR3 sequence identical to the amino acid sequence of SEQ ID NO:84.

17. The protein according to claim 16, wherein the second antigen-binding site comprises:
a light chain CDR1 sequence identical to the amino acid sequence of SEQ ID NO:85;
a light chain CDR2 sequence identical to the amino acid sequence of SEQ ID NO:86; and
a light chain CDR3 sequence identical to the amino acid sequence of SEQ ID NO:87.

18. The protein according to any one of claims 1-11, wherein the second antigen-binding site binds CAIX and comprises a heavy chain variable domain comprising an amino acid sequence at least 90% identical to SEQ ID NO:88 and a light chain variable domain comprising an amino acid sequence at least 90% identical to SEQ ID NO:89.

19. The protein according to any one of claims 1-11 or 18, wherein the second antigen-binding site binds CAIX and comprises:
a heavy chain CDR1 sequence identical to the amino acid sequence of SEQ ID NO:90;
a heavy chain CDR2 sequence identical to the amino acid sequence of SEQ ID NO:91; and
a heavy chain CDR3 sequence identical to the amino acid sequence of SEQ ID NO:92.

20. The protein according to claim 19, wherein the second antigen-binding site comprises:
a light chain CDR1 sequence identical to the amino acid sequence of SEQ ID NO:93;
a light chain CDR2 sequence identical to the amino acid sequence of SEQ ID NO:94; and
a light chain CDR3 sequence identical to the amino acid sequence of SEQ ID NO:95.

21. The protein according to any one of claims 1-11, wherein
a) the second antigen-binding site binds ANO1 and comprises a heavy chain variable domain comprising an amino acid sequence at least 90% identical to SEQ ID NO:97 and a light chain variable domain comprising an amino acid sequence at least 90% identical to SEQ ID NO:98,
b) the second antigen-binding site binds mesothelin and comprises a heavy chain variable domain comprising an amino acid sequence at least 90% identical to SEQ ID NO:106 and a light chain variable domain comprising an amino acid sequence at least 90% identical to SEQ ID NO:107,
c) the second antigen-binding site binds mesothelin and comprises a heavy chain variable domain comprising an amino acid sequence at least 90% identical to SEQ ID NO:114 and a light chain variable domain comprising an amino acid sequence at least 90% identical to SEQ ID NO:115,
d) the second antigen-binding site binds mesothelin and comprises a heavy chain variable domain comprising an amino acid sequence at least 90% identical to SEQ ID NO:122 and a light chain variable domain comprising an amino acid sequence at least 90% identical to SEQ ID NO:123,
e) the second antigen-binding site binds TROP2 and comprises a heavy chain variable domain comprising an amino acid sequence at least 90% identical to SEQ ID NO:172 and a light chain variable domain comprising an amino acid sequence at least 90% identical to SEQ ID NO:173,
f) the second antigen-binding site binds TROP2 and comprises a heavy chain variable domain comprising an amino acid sequence at least 90% identical to SEQ ID NO:180 and a light chain variable domain comprising an amino acid sequence at least 90% identical to SEQ ID NO:181,
g) the second antigen-binding site binds TROP2 and comprises a heavy chain variable domain comprising an amino acid sequence at least 90% identical to SEQ ID NO:188 and a light chain variable domain comprising an amino acid sequence at least 90% identical to SEQ ID NO:189,
h) the second antigen-binding site binds TROP2 and comprises a heavy chain variable domain comprising an amino acid sequence at least 90% identical to SEQ ID NO:190 and a light chain variable domain comprising an amino acid sequence at least 90% identical to SEQ ID NO:191,
i) the second antigen-binding site binds TROP2 and comprises a heavy chain variable domain comprising an amino acid sequence at least 90% identical to SEQ ID NO:192 and a light chain variable domain comprising an amino acid sequence at least 90% identical to SEQ ID NO:193,
j) the second antigen-binding site binds claudin-18.2 and comprises a heavy chain variable domain comprising an amino acid sequence at least 90% identical to SEQ ID NO:220 and a light chain variable domain comprising an amino acid sequence at least 90% identical to SEQ ID NO:221,
or
k) the second antigen-binding site binds claudin-18.2 and comprises a heavy chain variable domain comprising an amino acid sequence at least 90% identical to SEQ ID NO:228 and a light chain variable domain comprising an amino acid sequence at least 90% identical to SEQ ID NO:229.

22. A protein according to any one of claims 1-4 or 8-10, wherein the second antigen-binding site is a single-domain antibody, optionally wherein the second antigen-binding site is a V_{H}H fragment or a V_{NAR} fragment.

23. A protein according to any one of the preceding claims, wherein the protein comprises a portion of an antibody Fc domain sufficient to bind CD16, wherein the antibody Fc domain comprises hinge and CH2 domains.

24. A protein according to claim 23, wherein the antibody Fc domain comprises hinge and CH2 domains of a human IgG1 antibody.

25. A protein according to claim 23 or 24, wherein the Fc domain comprises an amino acid sequence at least 90% identical to amino acids 234-332 of a human IgG1 antibody.

26. A protein according to any one of claims 23-25, wherein the Fc domain comprises an amino acid sequence at least 90% identical to the Fc domain of human IgG1 and differs at one or more positions selected from the group consisting of Q347, Y349, L351, S354, E356, E357, K360, Q362, S364, T366, L368, K370, N390, K392, T394, D399, S400, D401, F405, Y407, K409, T411, and K439.

27. A formulation comprising a protein according to any one of the preceding claims and a pharmaceutically acceptable carrier.

28. A cell comprising one or more nucleic acids encoding a protein according to any one of claims 1-26.

29. A method of directly and/or indirectly enhancing tumor cell death, the method comprising exposing a tumor and natural killer cells to a protein according to any one of claims 1-26.

30. A method of treating cancer, wherein the method comprises administering a protein according to any one of claims 1-26 or a formulation according to claim 27 to a patient.

31. The method of claim 30, wherein the second antigen binding site of the protein
a) binds CAIX, and the cancer to be treated is selected from the group consisting of renal cell carcinoma, breast cancer, glioblastoma, head and neck cancer, gastric cancers, bladder cancer, ovarian cancer, tumors of the esophagus, lung, colon, kidney, cervix and non-small cell lung carcinoma,
b) binds mesothelin, and the cancer to be treated is selected from the group consisting of mesothelioma, ovarian cancer, pancreatic cancer, non-small cell lung cancer, breast cancer, cholangiocarcinoma, gastric cancer, uterine serous carcinoma, thymic carcinoma, and acute myeloid leukemia,
c) binds ANO1, and the cancer to be treated is selected from the group consisting of esophageal squamous cell cancer (ESCC), gastrointestinal stromal tumor (GIST), head and neck squamous cell carcinoma (HNSCC), pancreatic cancer, breast cancer, prostate cancer, and sarcoma,
d) binds TROP2, and the cancer to be treated is selected from the group consisting of breast, lung, gastric, colorectal, pancreatic, prostatic, cervical, head-and-neck cancer, nasopharyngeal carcinoma, and ovarian carcinoma,
or
e) binds claudin-18.2, and the cancer to be treated is selected from the group consisting of esophageal cancer, non-small cell lung carcinoma, ovarian cancer, colon cancer, and several forms of biliary ductal carcinoma.


SEQUENCE LISTING

<110> Dragonfly Therapeutics, Inc.

<120> MULTISPECIFIC BINDING PROTEINS TARGETING CAIX, ANO1,
      MESOTHELIN, TROP2, CEA, OR CLAUDIN-18.2

<130> P138262EPPC01/ILF

<140>
<141>

<150> 62/473,659
<151> 2017-03-20

<150> 62/473,652
<151> 2017-03-20

<150> 62/467,557
<151> 2017-03-06

<150> 62/464,347
<151> 2017-02-27

<150> 62/464,344
<151> 2017-02-27

<150> 62/464,341
<151> 2017-02-27

<160> 236

<170> PatentIn version 3.5

<210> 1
<211> 117
<212> PRT
<213> Artificial Sequence

<220>
<223> Description of Artificial Sequence: Synthetic
      polypeptide

<400> 1
Gln Val Gln Leu Gln Gln Trp Gly Ala Gly Leu Leu Lys Pro Ser Glu
1               5                   10                  15

Thr Leu Ser Leu Thr Cys Ala Val Tyr Gly Gly Ser Phe Ser Gly Tyr
            20                  25                  30

Tyr Trp Ser Trp Ile Arg Gln Pro Pro Gly Lys Gly Leu Glu Trp Ile
        35                  40                  45

Gly Glu Ile Asp His Ser Gly Ser Thr Asn Tyr Asn Pro Ser Leu Lys
    50                  55                  60

Ser Arg Val Thr Ile Ser Val Asp Thr Ser Lys Asn Gln Phe Ser Leu
65                  70                  75                  80

Lys Leu Ser Ser Val Thr Ala Ala Asp Thr Ala Val Tyr Tyr Cys Ala
                85                  90                  95

Arg Ala Arg Gly Pro Trp Ser Phe Asp Pro Trp Gly Gln Gly Thr Leu
            100                 105                 110

Val Thr Val Ser Ser
        115

<210> 2
<211> 107
<212> PRT
<213> Artificial Sequence

<220>
<223> Description of Artificial Sequence: Synthetic
      polypeptide

<400> 2
Asp Ile Gln Met Thr Gln Ser Pro Ser Thr Leu Ser Ala Ser Val Gly
1               5                   10                  15

Asp Arg Val Thr Ile Thr Cys Arg Ala Ser Gln Ser Ile Ser Ser Trp
            20                  25                  30

Leu Ala Trp Tyr Gln Gln Lys Pro Gly Lys Ala Pro Lys Leu Leu Ile
        35                  40                  45

Tyr Lys Ala Ser Ser Leu Glu Ser Gly Val Pro Ser Arg Phe Ser Gly
    50                  55                  60

Ser Gly Ser Gly Thr Glu Phe Thr Leu Thr Ile Ser Ser Leu Gln Pro
65                  70                  75                  80

Asp Asp Phe Ala Thr Tyr Tyr Cys Gln Gln Tyr Asn Ser Tyr Pro Ile
                85                  90                  95

Thr Phe Gly Gly Gly Thr Lys Val Glu Ile Lys
            100                 105

<210> 3
<211> 117
<212> PRT
<213> Artificial Sequence

<220>
<223> Description of Artificial Sequence: Synthetic
      polypeptide

<400> 3
Gln Val Gln Leu Gln Gln Trp Gly Ala Gly Leu Leu Lys Pro Ser Glu
1               5                   10                  15

Thr Leu Ser Leu Thr Cys Ala Val Tyr Gly Gly Ser Phe Ser Gly Tyr
            20                  25                  30

Tyr Trp Ser Trp Ile Arg Gln Pro Pro Gly Lys Gly Leu Glu Trp Ile
        35                  40                  45

Gly Glu Ile Asp His Ser Gly Ser Thr Asn Tyr Asn Pro Ser Leu Lys
    50                  55                  60

Ser Arg Val Thr Ile Ser Val Asp Thr Ser Lys Asn Gln Phe Ser Leu
65                  70                  75                  80

Lys Leu Ser Ser Val Thr Ala Ala Asp Thr Ala Val Tyr Tyr Cys Ala
                85                  90                  95

Arg Ala Arg Gly Pro Trp Ser Phe Asp Pro Trp Gly Gln Gly Thr Leu
            100                 105                 110

Val Thr Val Ser Ser
        115

<210> 4
<211> 108
<212> PRT
<213> Artificial Sequence

<220>
<223> Description of Artificial Sequence: Synthetic
      polypeptide

<400> 4
Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Ser Ser Ser
            20                  25                  30

Tyr Leu Ala Trp Tyr Gln Gln Lys Pro Gly Gln Ala Pro Arg Leu Leu
        35                  40                  45

Ile Tyr Gly Ala Ser Ser Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Tyr Gly Ser Ser Pro
                85                  90                  95

Ile Thr Phe Gly Gly Gly Thr Lys Val Glu Ile Lys
            100                 105

<210> 5
<211> 117
<212> PRT
<213> Artificial Sequence

<220>
<223> Description of Artificial Sequence: Synthetic
      polypeptide

<400> 5
Gln Val Gln Leu Gln Gln Trp Gly Ala Gly Leu Leu Lys Pro Ser Glu
1               5                   10                  15

Thr Leu Ser Leu Thr Cys Ala Val Tyr Gly Gly Ser Phe Ser Gly Tyr
            20                  25                  30

Tyr Trp Ser Trp Ile Arg Gln Pro Pro Gly Lys Gly Leu Glu Trp Ile
        35                  40                  45

Gly Glu Ile Asp His Ser Gly Ser Thr Asn Tyr Asn Pro Ser Leu Lys
    50                  55                  60

Ser Arg Val Thr Ile Ser Val Asp Thr Ser Lys Asn Gln Phe Ser Leu
65                  70                  75                  80

Lys Leu Ser Ser Val Thr Ala Ala Asp Thr Ala Val Tyr Tyr Cys Ala
                85                  90                  95

Arg Ala Arg Gly Pro Trp Ser Phe Asp Pro Trp Gly Gln Gly Thr Leu
            100                 105                 110

Val Thr Val Ser Ser
        115

<210> 6
<211> 106
<212> PRT
<213> Artificial Sequence

<220>
<223> Description of Artificial Sequence: Synthetic
      polypeptide

<400> 6
Asp Ile Gln Met Thr Gln Ser Pro Ser Thr Leu Ser Ala Ser Val Gly
1               5                   10                  15

Asp Arg Val Thr Ile Thr Cys Arg Ala Ser Gln Ser Ile Gly Ser Trp
            20                  25                  30

Leu Ala Trp Tyr Gln Gln Lys Pro Gly Lys Ala Pro Lys Leu Leu Ile
        35                  40                  45

Tyr Lys Ala Ser Ser Leu Glu Ser Gly Val Pro Ser Arg Phe Ser Gly
    50                  55                  60

Ser Gly Ser Gly Thr Glu Phe Thr Leu Thr Ile Ser Ser Leu Gln Pro
65                  70                  75                  80

Asp Asp Phe Ala Thr Tyr Tyr Cys Gln Gln Tyr His Ser Phe Tyr Thr
                85                  90                  95

Phe Gly Gly Gly Thr Lys Val Glu Ile Lys
            100                 105

<210> 7
<211> 117
<212> PRT
<213> Artificial Sequence

<220>
<223> Description of Artificial Sequence: Synthetic
      polypeptide

<400> 7
Gln Val Gln Leu Gln Gln Trp Gly Ala Gly Leu Leu Lys Pro Ser Glu
1               5                   10                  15

Thr Leu Ser Leu Thr Cys Ala Val Tyr Gly Gly Ser Phe Ser Gly Tyr
            20                  25                  30

Tyr Trp Ser Trp Ile Arg Gln Pro Pro Gly Lys Gly Leu Glu Trp Ile
        35                  40                  45

Gly Glu Ile Asp His Ser Gly Ser Thr Asn Tyr Asn Pro Ser Leu Lys
    50                  55                  60

Ser Arg Val Thr Ile Ser Val Asp Thr Ser Lys Asn Gln Phe Ser Leu
65                  70                  75                  80

Lys Leu Ser Ser Val Thr Ala Ala Asp Thr Ala Val Tyr Tyr Cys Ala
                85                  90                  95

Arg Ala Arg Gly Pro Trp Ser Phe Asp Pro Trp Gly Gln Gly Thr Leu
            100                 105                 110

Val Thr Val Ser Ser
        115

<210> 8
<211> 106
<212> PRT
<213> Artificial Sequence

<220>
<223> Description of Artificial Sequence: Synthetic
      polypeptide

<400> 8
Asp Ile Gln Met Thr Gln Ser Pro Ser Thr Leu Ser Ala Ser Val Gly
1               5                   10                  15

Asp Arg Val Thr Ile Thr Cys Arg Ala Ser Gln Ser Ile Gly Ser Trp
            20                  25                  30

Leu Ala Trp Tyr Gln Gln Lys Pro Gly Lys Ala Pro Lys Leu Leu Ile
        35                  40                  45

Tyr Lys Ala Ser Ser Leu Glu Ser Gly Val Pro Ser Arg Phe Ser Gly
    50                  55                  60

Ser Gly Ser Gly Thr Glu Phe Thr Leu Thr Ile Ser Ser Leu Gln Pro
65                  70                  75                  80

Asp Asp Phe Ala Thr Tyr Tyr Cys Gln Gln Ser Asn Ser Tyr Tyr Thr
                85                  90                  95

Phe Gly Gly Gly Thr Lys Val Glu Ile Lys
            100                 105


<210> 9
<211> 117
<212> PRT
<213> Artificial Sequence

<220>
<223> Description of Artificial Sequence: Synthetic
      polypeptide

<400> 9
Gln Val Gln Leu Gln Gln Trp Gly Ala Gly Leu Leu Lys Pro Ser Glu
1               5                   10                  15

Thr Leu Ser Leu Thr Cys Ala Val Tyr Gly Gly Ser Phe Ser Gly Tyr
            20                  25                  30

Tyr Trp Ser Trp Ile Arg Gln Pro Pro Gly Lys Gly Leu Glu Trp Ile
        35                  40                  45

Gly Glu Ile Asp His Ser Gly Ser Thr Asn Tyr Asn Pro Ser Leu Lys
    50                  55                  60

Ser Arg Val Thr Ile Ser Val Asp Thr Ser Lys Asn Gln Phe Ser Leu
65                  70                  75                  80

Lys Leu Ser Ser Val Thr Ala Ala Asp Thr Ala Val Tyr Tyr Cys Ala
                85                  90                  95

Arg Ala Arg Gly Pro Trp Ser Phe Asp Pro Trp Gly Gln Gly Thr Leu
            100                 105                 110

Val Thr Val Ser Ser
        115

<210> 10
<211> 106
<212> PRT
<213> Artificial Sequence

<220>
<223> Description of Artificial Sequence: Synthetic
      polypeptide

<400> 10
Asp Ile Gln Met Thr Gln Ser Pro Ser Thr Leu Ser Ala Ser Val Gly
1               5                   10                  15

Asp Arg Val Thr Ile Thr Cys Arg Ala Ser Gln Ser Ile Ser Ser Trp
            20                  25                  30

Leu Ala Trp Tyr Gln Gln Lys Pro Gly Lys Ala Pro Lys Leu Leu Ile
        35                  40                  45

Tyr Lys Ala Ser Ser Leu Glu Ser Gly Val Pro Ser Arg Phe Ser Gly
    50                  55                  60

Ser Gly Ser Gly Thr Glu Phe Thr Leu Thr Ile Ser Ser Leu Gln Pro
65                  70                  75                  80

Asp Asp Phe Ala Thr Tyr Tyr Cys Gln Gln Tyr Asn Ser Tyr Pro Thr
                85                  90                  95

Phe Gly Gly Gly Thr Lys Val Glu Ile Lys
            100                 105

<210> 11
<211> 117
<212> PRT
<213> Artificial Sequence

<220>
<223> Description of Artificial Sequence: Synthetic

polypeptide

<400> 11
Gln Val Gln Leu Gln Gln Trp Gly Ala Gly Leu Leu Lys Pro Ser Glu
1               5                   10                  15

Thr Leu Ser Leu Thr Cys Ala Val Tyr Gly Gly Ser Phe Ser Gly Tyr
            20                  25                  30

Tyr Trp Ser Trp Ile Arg Gln Pro Pro Gly Lys Gly Leu Glu Trp Ile
        35                  40                  45

Gly Glu Ile Asp His Ser Gly Ser Thr Asn Tyr Asn Pro Ser Leu Lys
    50                  55                  60

Ser Arg Val Thr Ile Ser Val Asp Thr Ser Lys Asn Gln Phe Ser Leu
65                  70                  75                  80

Lys Leu Ser Ser Val Thr Ala Ala Asp Thr Ala Val Tyr Tyr Cys Ala
                85                  90                  95

Arg Ala Arg Gly Pro Trp Gly Phe Asp Pro Trp Gly Gln Gly Thr Leu
            100                 105                 110

Val Thr Val Ser Ser
        115


<210> 12
<211> 107
<212> PRT
<213> Artificial Sequence

<220>
<223> Description of Artificial Sequence: Synthetic
      polypeptide

<400> 12
Glu Leu Gln Met Thr Gln Ser Pro Ser Ser Leu Ser Ala Ser Val Gly
1               5                   10                  15

Asp Arg Val Thr Ile Thr Cys Arg Thr Ser Gln Ser Ile Ser Ser Tyr
            20                  25                  30

Leu Asn Trp Tyr Gln Gln Lys Pro Gly Gln Pro Pro Lys Leu Leu Ile
        35                  40                  45

Tyr Trp Ala Ser Thr Arg Glu Ser Gly Val Pro Asp Arg Phe Ser Gly
    50                  55                  60

Ser Gly Ser Gly Thr Asp Phe Thr Leu Thr Ile Ser Ser Leu Gln Pro
65                  70                  75                  80

Glu Asp Ser Ala Thr Tyr Tyr Cys Gln Gln Ser Tyr Asp Ile Pro Tyr
                85                  90                  95

Thr Phe Gly Gln Gly Thr Lys Leu Glu Ile Lys
            100                 105

<210> 13
<211> 117
<212> PRT
<213> Artificial Sequence

<220>
<223> Description of Artificial Sequence: Synthetic
      polypeptide

<400> 13
Gln Val Gln Leu Gln Gln Trp Gly Ala Gly Leu Leu Lys Pro Ser Glu
1               5                   10                  15

Thr Leu Ser Leu Thr Cys Ala Val Tyr Gly Gly Ser Phe Ser Gly Tyr
            20                  25                  30

Tyr Trp Ser Trp Ile Arg Gln Pro Pro Gly Lys Gly Leu Glu Trp Ile
        35                  40                  45

Gly Glu Ile Asp His Ser Gly Ser Thr Asn Tyr Asn Pro Ser Leu Lys
    50                  55                  60

Ser Arg Val Thr Ile Ser Val Asp Thr Ser Lys Asn Gln Phe Ser Leu
65                  70                  75                  80

Lys Leu Ser Ser Val Thr Ala Ala Asp Thr Ala Val Tyr Tyr Cys Ala
                85                  90                  95

Arg Ala Arg Gly Pro Trp Ser Phe Asp Pro Trp Gly Gln Gly Thr Leu
            100                 105                 110

Val Thr Val Ser Ser
        115

<210> 14
<211> 107
<212> PRT
<213> Artificial Sequence

<220>
<223> Description of Artificial Sequence: Synthetic
      polypeptide

<400> 14
Asp Ile Gln Met Thr Gln Ser Pro Ser Thr Leu Ser Ala Ser Val Gly

1               5                   10                  15

Asp Arg Val Thr Ile Thr Cys Arg Ala Ser Gln Ser Ile Ser Ser Trp
            20                  25                  30

Leu Ala Trp Tyr Gln Gln Lys Pro Gly Lys Ala Pro Lys Leu Leu Ile
        35                  40                  45

Tyr Lys Ala Ser Ser Leu Glu Ser Gly Val Pro Ser Arg Phe Ser Gly
    50                  55                  60

Ser Gly Ser Gly Thr Glu Phe Thr Leu Thr Ile Ser Ser Leu Gln Pro
65                  70                  75                  80

Asp Asp Phe Ala Thr Tyr Tyr Cys Gln Gln Tyr Gly Ser Phe Pro Ile
                85                  90                  95

Thr Phe Gly Gly Gly Thr Lys Val Glu Ile Lys
            100                 105

<210> 15
<211> 117
<212> PRT
<213> Artificial Sequence

<220>
<223> Description of Artificial Sequence: Synthetic
      polypeptide

<400> 15
Gln Val Gln Leu Gln Gln Trp Gly Ala Gly Leu Leu Lys Pro Ser Glu
1               5                   10                  15

Thr Leu Ser Leu Thr Cys Ala Val Tyr Gly Gly Ser Phe Ser Gly Tyr
            20                  25                  30

Tyr Trp Ser Trp Ile Arg Gln Pro Pro Gly Lys Gly Leu Glu Trp Ile
        35                  40                  45

Gly Glu Ile Asp His Ser Gly Ser Thr Asn Tyr Asn Pro Ser Leu Lys
    50                  55                  60

Ser Arg Val Thr Ile Ser Val Asp Thr Ser Lys Asn Gln Phe Ser Leu
65                  70                  75                  80

Lys Leu Ser Ser Val Thr Ala Ala Asp Thr Ala Val Tyr Tyr Cys Ala
                85                  90                  95

Arg Ala Arg Gly Pro Trp Ser Phe Asp Pro Trp Gly Gln Gly Thr Leu
            100                 105                 110

Val Thr Val Ser Ser
        115

<210> 16
<211> 107
<212> PRT
<213> Artificial Sequence

<220>
<223> Description of Artificial Sequence: Synthetic
      polypeptide

<400> 16
Asp Ile Gln Met Thr Gln Ser Pro Ser Thr Leu Ser Ala Ser Val Gly
1               5                   10                  15

Asp Arg Val Thr Ile Thr Cys Arg Ala Ser Gln Ser Ile Ser Ser Trp
            20                  25                  30

Leu Ala Trp Tyr Gln Gln Lys Pro Gly Lys Ala Pro Lys Leu Leu Ile
        35                  40                  45

Tyr Lys Ala Ser Ser Leu Glu Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Asp Asp Phe Ala Thr Tyr Tyr Cys Gln Gln Ser Lys Glu Val Pro Trp
                85                  90                  95

Thr Phe Gly Gln Gly Thr Lys Val Glu Ile Lys
            100                 105

<210> 17
<211> 117
<212> PRT
<213> Artificial Sequence

<220>
<223> Description of Artificial Sequence: Synthetic
      polypeptide

<400> 17
Gln Val Gln Leu Gln Gln Trp Gly Ala Gly Leu Leu Lys Pro Ser Glu
1               5                   10                  15

Thr Leu Ser Leu Thr Cys Ala Val Tyr Gly Gly Ser Phe Ser Gly Tyr
            20                  25                  30

Tyr Trp Ser Trp Ile Arg Gln Pro Pro Gly Lys Gly Leu Glu Trp Ile

35                  40                  45

Gly Glu Ile Asp His Ser Gly Ser Thr Asn Tyr Asn Pro Ser Leu Lys
    50                  55                  60

Ser Arg Val Thr Ile Ser Val Asp Thr Ser Lys Asn Gln Phe Ser Leu
65                  70                  75                  80

Lys Leu Ser Ser Val Thr Ala Ala Asp Thr Ala Val Tyr Tyr Cys Ala
                85                  90                  95

Arg Ala Arg Gly Pro Trp Ser Phe Asp Pro Trp Gly Gln Gly Thr Leu
            100                 105                 110

Val Thr Val Ser Ser
        115

<210> 18
<211> 106
<212> PRT
<213> Artificial Sequence

<220>
<223> Description of Artificial Sequence: Synthetic
      polypeptide

<400> 18
Asp Ile Gln Met Thr Gln Ser Pro Ser Thr Leu Ser Ala Ser Val Gly
1               5                   10                  15

Asp Arg Val Thr Ile Thr Cys Arg Ala Ser Gln Ser Ile Ser Ser Trp
            20                  25                  30

Leu Ala Trp Tyr Gln Gln Lys Pro Gly Lys Ala Pro Lys Leu Leu Ile
        35                  40                  45

Tyr Lys Ala Ser Ser Leu Glu Ser Gly Val Pro Ser Arg Phe Ser Gly
    50                  55                  60

Ser Gly Ser Gly Thr Glu Phe Thr Leu Thr Ile Ser Ser Leu Gln Pro
65                  70                  75                  80

Asp Asp Phe Ala Thr Tyr Tyr Cys Gln Gln Tyr Asn Ser Phe Pro Thr
                85                  90                  95

Phe Gly Gly Gly Thr Lys Val Glu Ile Lys
            100                 105

<210> 19
<211> 117

<212> PRT
<213> Artificial Sequence

<220>
<223> Description of Artificial Sequence: Synthetic
      polypeptide

<400> 19
Gln Val Gln Leu Gln Gln Trp Gly Ala Gly Leu Leu Lys Pro Ser Glu
1               5                   10                  15

Thr Leu Ser Leu Thr Cys Ala Val Tyr Gly Gly Ser Phe Ser Gly Tyr
            20                  25                  30

Tyr Trp Ser Trp Ile Arg Gln Pro Pro Gly Lys Gly Leu Glu Trp Ile
        35                  40                  45

Gly Glu Ile Asp His Ser Gly Ser Thr Asn Tyr Asn Pro Ser Leu Lys
    50                  55                  60

Ser Arg Val Thr Ile Ser Val Asp Thr Ser Lys Asn Gln Phe Ser Leu
65                  70                  75                  80

Lys Leu Ser Ser Val Thr Ala Ala Asp Thr Ala Val Tyr Tyr Cys Ala
                85                  90                  95

Arg Ala Arg Gly Pro Trp Ser Phe Asp Pro Trp Gly Gln Gly Thr Leu
            100                 105                 110

Val Thr Val Ser Ser
        115


<210> 20
<211> 106
<212> PRT
<213> Artificial Sequence

<220>
<223> Description of Artificial Sequence: Synthetic
      polypeptide

<400> 20
Asp Ile Gln Met Thr Gln Ser Pro Ser Thr Leu Ser Ala Ser Val Gly
1               5                   10                  15

Asp Arg Val Thr Ile Thr Cys Arg Ala Ser Gln Ser Ile Gly Ser Trp
            20                  25                  30

Leu Ala Trp Tyr Gln Gln Lys Pro Gly Lys Ala Pro Lys Leu Leu Ile
        35                  40                  45

Tyr Lys Ala Ser Ser Leu Glu Ser Gly Val Pro Ser Arg Phe Ser Gly

50                  55                  60

Ser Gly Ser Gly Thr Glu Phe Thr Leu Thr Ile Ser Ser Leu Gln Pro
65                  70                  75                  80

Asp Asp Phe Ala Thr Tyr Tyr Cys Gln Gln Tyr Asp Ile Tyr Pro Thr
                85                  90                  95

Phe Gly Gly Gly Thr Lys Val Glu Ile Lys
            100                 105

<210> 21
<211> 117
<212> PRT
<213> Artificial Sequence

<220>
<223> Description of Artificial Sequence: Synthetic
      polypeptide

<400> 21
Gln Val Gln Leu Gln Gln Trp Gly Ala Gly Leu Leu Lys Pro Ser Glu
1               5                   10                  15

Thr Leu Ser Leu Thr Cys Ala Val Tyr Gly Gly Ser Phe Ser Gly Tyr
            20                  25                  30

Tyr Trp Ser Trp Ile Arg Gln Pro Pro Gly Lys Gly Leu Glu Trp Ile
        35                  40                  45

Gly Glu Ile Asp His Ser Gly Ser Thr Asn Tyr Asn Pro Ser Leu Lys
    50                  55                  60

Ser Arg Val Thr Ile Ser Val Asp Thr Ser Lys Asn Gln Phe Ser Leu
65                  70                  75                  80

Lys Leu Ser Ser Val Thr Ala Ala Asp Thr Ala Val Tyr Tyr Cys Ala
                85                  90                  95

Arg Ala Arg Gly Pro Trp Ser Phe Asp Pro Trp Gly Gln Gly Thr Leu
            100                 105                 110

Val Thr Val Ser Ser
        115

<210> 22
<211> 106
<212> PRT
<213> Artificial Sequence

<220>

<223> Description of Artificial Sequence: Synthetic
      polypeptide

<400> 22
Asp Ile Gln Met Thr Gln Ser Pro Ser Thr Leu Ser Ala Ser Val Gly
1               5                   10                  15

Asp Arg Val Thr Ile Thr Cys Arg Ala Ser Gln Ser Ile Ser Ser Trp
            20                  25                  30

Leu Ala Trp Tyr Gln Gln Lys Pro Gly Lys Ala Pro Lys Leu Leu Ile
        35                  40                  45

Tyr Lys Ala Ser Ser Leu Glu Ser Gly Val Pro Ser Arg Phe Ser Gly
    50                  55                  60

Ser Gly Ser Gly Thr Glu Phe Thr Leu Thr Ile Ser Ser Leu Gln Pro
65                  70                  75                  80

Asp Asp Phe Ala Thr Tyr Tyr Cys Gln Gln Tyr Asp Ser Tyr Pro Thr
                85                  90                  95

Phe Gly Gly Gly Thr Lys Val Glu Ile Lys
            100                 105


<210> 23
<211> 117
<212> PRT
<213> Artificial Sequence

<220>
<223> Description of Artificial Sequence: Synthetic
      polypeptide

<400> 23
Gln Val Gln Leu Gln Gln Trp Gly Ala Gly Leu Leu Lys Pro Ser Glu
1               5                   10                  15

Thr Leu Ser Leu Thr Cys Ala Val Tyr Gly Gly Ser Phe Ser Gly Tyr
            20                  25                  30

Tyr Trp Ser Trp Ile Arg Gln Pro Pro Gly Lys Gly Leu Glu Trp Ile
        35                  40                  45

Gly Glu Ile Asp His Ser Gly Ser Thr Asn Tyr Asn Pro Ser Leu Lys
    50                  55                  60

Ser Arg Val Thr Ile Ser Val Asp Thr Ser Lys Asn Gln Phe Ser Leu
65                  70                  75                  80

Lys Leu Ser Ser Val Thr Ala Ala Asp Thr Ala Val Tyr Tyr Cys Ala

85                  90                  95

Arg Ala Arg Gly Pro Trp Ser Phe Asp Pro Trp Gly Gln Gly Thr Leu
            100                 105                 110

Val Thr Val Ser Ser
        115

<210> 24
<211> 106
<212> PRT
<213> Artificial Sequence

<220>
<223> Description of Artificial Sequence: Synthetic
      polypeptide

<400> 24
Asp Ile Gln Met Thr Gln Ser Pro Ser Thr Leu Ser Ala Ser Val Gly
1               5                   10                  15

Asp Arg Val Thr Ile Thr Cys Arg Ala Ser Gln Ser Ile Ser Ser Trp
            20                  25                  30

Leu Ala Trp Tyr Gln Gln Lys Pro Gly Lys Ala Pro Lys Leu Leu Ile
        35                  40                  45

Tyr Lys Ala Ser Ser Leu Glu Ser Gly Val Pro Ser Arg Phe Ser Gly
    50                  55                  60

Ser Gly Ser Gly Thr Glu Phe Thr Leu Thr Ile Ser Ser Leu Gln Pro
65                  70                  75                  80

Asp Asp Phe Ala Thr Tyr Tyr Cys Gln Gln Tyr Gly Ser Phe Pro Thr
                85                  90                  95

Phe Gly Gly Gly Thr Lys Val Glu Ile Lys
            100                 105

<210> 25
<211> 117
<212> PRT
<213> Artificial Sequence

<220>
<223> Description of Artificial Sequence: Synthetic
      polypeptide

<400> 25
Gln Val Gln Leu Gln Gln Trp Gly Ala Gly Leu Leu Lys Pro Ser Glu
1               5                   10                  15

Thr Leu Ser Leu Thr Cys Ala Val Tyr Gly Gly Ser Phe Ser Gly Tyr
            20                  25                  30

Tyr Trp Ser Trp Ile Arg Gln Pro Pro Gly Lys Gly Leu Glu Trp Ile
        35                  40                  45

Gly Glu Ile Asp His Ser Gly Ser Thr Asn Tyr Asn Pro Ser Leu Lys
    50                  55                  60

Ser Arg Val Thr Ile Ser Val Asp Thr Ser Lys Asn Gln Phe Ser Leu
65                  70                  75                  80

Lys Leu Ser Ser Val Thr Ala Ala Asp Thr Ala Val Tyr Tyr Cys Ala
                85                  90                  95

Arg Ala Arg Gly Pro Trp Ser Phe Asp Pro Trp Gly Gln Gly Thr Leu
            100                 105                 110

Val Thr Val Ser Ser
        115

<210> 26
<211> 106
<212> PRT
<213> Artificial Sequence

<220>
<223> Description of Artificial Sequence: Synthetic
      polypeptide

<400> 26
Asp Ile Gln Met Thr Gln Ser Pro Ser Thr Leu Ser Ala Ser Val Gly
1               5                   10                  15

Asp Arg Val Thr Ile Thr Cys Arg Ala Ser Gln Ser Ile Ser Ser Trp
            20                  25                  30

Leu Ala Trp Tyr Gln Gln Lys Pro Gly Lys Ala Pro Lys Leu Leu Ile
        35                  40                  45

Tyr Lys Ala Ser Ser Leu Glu Ser Gly Val Pro Ser Arg Phe Ser Gly
    50                  55                  60

Ser Gly Ser Gly Thr Glu Phe Thr Leu Thr Ile Ser Ser Leu Gln Pro
65                  70                  75                  80

Asp Asp Phe Ala Thr Tyr Tyr Cys Gln Gln Tyr Gln Ser Phe Pro Thr
                85                  90                  95

Phe Gly Gly Gly Thr Lys Val Glu Ile Lys

            100                 105

<210> 27
<211> 117
<212> PRT
<213> Artificial Sequence

<220>
<223> Description of Artificial Sequence: Synthetic
      polypeptide

<400> 27
Gln Val Gln Leu Gln Gln Trp Gly Ala Gly Leu Leu Lys Pro Ser Glu
1               5                   10                  15

Thr Leu Ser Leu Thr Cys Ala Val Tyr Gly Gly Ser Phe Ser Gly Tyr
            20                  25                  30

Tyr Trp Ser Trp Ile Arg Gln Pro Pro Gly Lys Gly Leu Glu Trp Ile
        35                  40                  45

Gly Glu Ile Asp His Ser Gly Ser Thr Asn Tyr Asn Pro Ser Leu Lys
    50                  55                  60

Ser Arg Val Thr Ile Ser Val Asp Thr Ser Lys Asn Gln Phe Ser Leu
65                  70                  75                  80

Lys Leu Ser Ser Val Thr Ala Ala Asp Thr Ala Val Tyr Tyr Cys Ala
                85                  90                  95

Arg Ala Arg Gly Pro Trp Ser Phe Asp Pro Trp Gly Gln Gly Thr Leu
            100                 105                 110

Val Thr Val Ser Ser
        115

<210> 28
<211> 106
<212> PRT
<213> Artificial Sequence

<220>
<223> Description of Artificial Sequence: Synthetic
      polypeptide

<400> 28
Asp Ile Gln Met Thr Gln Ser Pro Ser Thr Leu Ser Ala Ser Val Gly
1               5                   10                  15

Asp Arg Val Thr Ile Thr Cys Arg Ala Ser Gln Ser Ile Ser Ser Trp
            20                  25                  30

Leu Ala Trp Tyr Gln Gln Lys Pro Gly Lys Ala Pro Lys Leu Leu Ile
        35                  40                  45

Tyr Lys Ala Ser Ser Leu Glu Ser Gly Val Pro Ser Arg Phe Ser Gly
    50                  55                  60

Ser Gly Ser Gly Thr Glu Phe Thr Leu Thr Ile Ser Ser Leu Gln Pro
65                  70                  75                  80

Asp Asp Phe Ala Thr Tyr Tyr Cys Gln Gln Tyr Ser Ser Phe Ser Thr
                85                  90                  95

Phe Gly Gly Gly Thr Lys Val Glu Ile Lys
            100                 105

<210> 29
<211> 117
<212> PRT
<213> Artificial Sequence

<220>
<223> Description of Artificial Sequence: Synthetic
      polypeptide

<400> 29
Gln Val Gln Leu Gln Gln Trp Gly Ala Gly Leu Leu Lys Pro Ser Glu
1               5                   10                  15

Thr Leu Ser Leu Thr Cys Ala Val Tyr Gly Gly Ser Phe Ser Gly Tyr
            20                  25                  30

Tyr Trp Ser Trp Ile Arg Gln Pro Pro Gly Lys Gly Leu Glu Trp Ile
        35                  40                  45

Gly Glu Ile Asp His Ser Gly Ser Thr Asn Tyr Asn Pro Ser Leu Lys
    50                  55                  60

Ser Arg Val Thr Ile Ser Val Asp Thr Ser Lys Asn Gln Phe Ser Leu
65                  70                  75                  80

Lys Leu Ser Ser Val Thr Ala Ala Asp Thr Ala Val Tyr Tyr Cys Ala
                85                  90                  95

Arg Ala Arg Gly Pro Trp Ser Phe Asp Pro Trp Gly Gln Gly Thr Leu
            100                 105                 110

Val Thr Val Ser Ser
        115

<210> 30

<211> 106
<212> PRT
<213> Artificial Sequence

<220>
<223> Description of Artificial Sequence: Synthetic
      polypeptide

<400> 30
Asp Ile Gln Met Thr Gln Ser Pro Ser Thr Leu Ser Ala Ser Val Gly
1               5                   10                  15

Asp Arg Val Thr Ile Thr Cys Arg Ala Ser Gln Ser Ile Ser Ser Trp
            20                  25                  30

Leu Ala Trp Tyr Gln Gln Lys Pro Gly Lys Ala Pro Lys Leu Leu Ile
        35                  40                  45

Tyr Lys Ala Ser Ser Leu Glu Ser Gly Val Pro Ser Arg Phe Ser Gly
    50                  55                  60

Ser Gly Ser Gly Thr Glu Phe Thr Leu Thr Ile Ser Ser Leu Gln Pro
65                  70                  75                  80

Asp Asp Phe Ala Thr Tyr Tyr Cys Gln Gln Tyr Glu Ser Tyr Ser Thr
                85                  90                  95

Phe Gly Gly Gly Thr Lys Val Glu Ile Lys
            100                 105


<210> 31
<211> 117
<212> PRT
<213> Artificial Sequence

<220>
<223> Description of Artificial Sequence: Synthetic
      polypeptide

<400> 31
Gln Val Gln Leu Gln Gln Trp Gly Ala Gly Leu Leu Lys Pro Ser Glu
1               5                   10                  15

Thr Leu Ser Leu Thr Cys Ala Val Tyr Gly Gly Ser Phe Ser Gly Tyr
            20                  25                  30

Tyr Trp Ser Trp Ile Arg Gln Pro Pro Gly Lys Gly Leu Glu Trp Ile
        35                  40                  45

Gly Glu Ile Asp His Ser Gly Ser Thr Asn Tyr Asn Pro Ser Leu Lys
    50                  55                  60

Ser Arg Val Thr Ile Ser Val Asp Thr Ser Lys Asn Gln Phe Ser Leu
65                  70                  75                  80

Lys Leu Ser Ser Val Thr Ala Ala Asp Thr Ala Val Tyr Tyr Cys Ala
                85                  90                  95

Arg Ala Arg Gly Pro Trp Ser Phe Asp Pro Trp Gly Gln Gly Thr Leu
            100                 105                 110

Val Thr Val Ser Ser
        115

<210> 32
<211> 106
<212> PRT
<213> Artificial Sequence

<220>
<223> Description of Artificial Sequence: Synthetic
      polypeptide

<400> 32
Asp Ile Gln Met Thr Gln Ser Pro Ser Thr Leu Ser Ala Ser Val Gly
1               5                   10                  15

Asp Arg Val Thr Ile Thr Cys Arg Ala Ser Gln Ser Ile Ser Ser Trp
            20                  25                  30

Leu Ala Trp Tyr Gln Gln Lys Pro Gly Lys Ala Pro Lys Leu Leu Ile
        35                  40                  45

Tyr Lys Ala Ser Ser Leu Glu Ser Gly Val Pro Ser Arg Phe Ser Gly
    50                  55                  60

Ser Gly Ser Gly Thr Glu Phe Thr Leu Thr Ile Ser Ser Leu Gln Pro
65                  70                  75                  80

Asp Asp Phe Ala Thr Tyr Tyr Cys Gln Gln Tyr Asp Ser Phe Ile Thr
                85                  90                  95

Phe Gly Gly Gly Thr Lys Val Glu Ile Lys
            100                 105

<210> 33
<211> 117
<212> PRT
<213> Artificial Sequence

<220>
<223> Description of Artificial Sequence: Synthetic
      polypeptide

<400> 33
Gln Val Gln Leu Gln Gln Trp Gly Ala Gly Leu Leu Lys Pro Ser Glu
1               5                   10                  15

Thr Leu Ser Leu Thr Cys Ala Val Tyr Gly Gly Ser Phe Ser Gly Tyr
            20                  25                  30

Tyr Trp Ser Trp Ile Arg Gln Pro Pro Gly Lys Gly Leu Glu Trp Ile
        35                  40                  45

Gly Glu Ile Asp His Ser Gly Ser Thr Asn Tyr Asn Pro Ser Leu Lys
    50                  55                  60

Ser Arg Val Thr Ile Ser Val Asp Thr Ser Lys Asn Gln Phe Ser Leu
65                  70                  75                  80

Lys Leu Ser Ser Val Thr Ala Ala Asp Thr Ala Val Tyr Tyr Cys Ala
                85                  90                  95

Arg Ala Arg Gly Pro Trp Ser Phe Asp Pro Trp Gly Gln Gly Thr Leu
            100                 105                 110

Val Thr Val Ser Ser
        115


<210> 34
<211> 106
<212> PRT
<213> Artificial Sequence

<220>
<223> Description of Artificial Sequence: Synthetic
      polypeptide

<400> 34
Asp Ile Gln Met Thr Gln Ser Pro Ser Thr Leu Ser Ala Ser Val Gly
1               5                   10                  15

Asp Arg Val Thr Ile Thr Cys Arg Ala Ser Gln Ser Ile Ser Ser Trp
            20                  25                  30

Leu Ala Trp Tyr Gln Gln Lys Pro Gly Lys Ala Pro Lys Leu Leu Ile
        35                  40                  45

Tyr Lys Ala Ser Ser Leu Glu Ser Gly Val Pro Ser Arg Phe Ser Gly
    50                  55                  60

Ser Gly Ser Gly Thr Glu Phe Thr Leu Thr Ile Ser Ser Leu Gln Pro
65                  70                  75                  80

Asp Asp Phe Ala Thr Tyr Tyr Cys Gln Gln Tyr Gln Ser Tyr Pro Thr
                85                  90                  95

Phe Gly Gly Gly Thr Lys Val Glu Ile Lys
            100                 105

<210> 35
<211> 117
<212> PRT
<213> Artificial Sequence

<220>
<223> Description of Artificial Sequence: Synthetic
      polypeptide

<400> 35
Gln Val Gln Leu Gln Gln Trp Gly Ala Gly Leu Leu Lys Pro Ser Glu
1               5                   10                  15

Thr Leu Ser Leu Thr Cys Ala Val Tyr Gly Gly Ser Phe Ser Gly Tyr
            20                  25                  30

Tyr Trp Ser Trp Ile Arg Gln Pro Pro Gly Lys Gly Leu Glu Trp Ile
        35                  40                  45

Gly Glu Ile Asp His Ser Gly Ser Thr Asn Tyr Asn Pro Ser Leu Lys
    50                  55                  60

Ser Arg Val Thr Ile Ser Val Asp Thr Ser Lys Asn Gln Phe Ser Leu
65                  70                  75                  80

Lys Leu Ser Ser Val Thr Ala Ala Asp Thr Ala Val Tyr Tyr Cys Ala
                85                  90                  95

Arg Ala Arg Gly Pro Trp Ser Phe Asp Pro Trp Gly Gln Gly Thr Leu
            100                 105                 110

Val Thr Val Ser Ser
        115

<210> 36
<211> 106
<212> PRT
<213> Artificial Sequence

<220>
<223> Description of Artificial Sequence: Synthetic
      polypeptide

<400> 36
Asp Ile Gln Met Thr Gln Ser Pro Ser Thr Leu Ser Ala Ser Val Gly
1               5                   10                  15

Asp Arg Val Thr Ile Thr Cys Arg Ala Ser Gln Ser Ile Gly Ser Trp
            20                  25                  30

Leu Ala Trp Tyr Gln Gln Lys Pro Gly Lys Ala Pro Lys Leu Leu Ile
        35                  40                  45

Tyr Lys Ala Ser Ser Leu Glu Ser Gly Val Pro Ser Arg Phe Ser Gly
    50                  55                  60

Ser Gly Ser Gly Thr Glu Phe Thr Leu Thr Ile Ser Ser Leu Gln Pro
65                  70                  75                  80

Asp Asp Phe Ala Thr Tyr Tyr Cys Gln Gln Tyr His Ser Phe Pro Thr
                85                  90                  95

Phe Gly Gly Gly Thr Lys Val Glu Ile Lys
            100                 105

<210> 37
<211> 117
<212> PRT
<213> Artificial Sequence

<220>
<223> Description of Artificial Sequence: Synthetic
      polypeptide

<400> 37
Gln Val Gln Leu Gln Gln Trp Gly Ala Gly Leu Leu Lys Pro Ser Glu
1               5                   10                  15

Thr Leu Ser Leu Thr Cys Ala Val Tyr Gly Gly Ser Phe Ser Gly Tyr
            20                  25                  30

Tyr Trp Ser Trp Ile Arg Gln Pro Pro Gly Lys Gly Leu Glu Trp Ile
        35                  40                  45

Gly Glu Ile Asp His Ser Gly Ser Thr Asn Tyr Asn Pro Ser Leu Lys
    50                  55                  60

Ser Arg Val Thr Ile Ser Val Asp Thr Ser Lys Asn Gln Phe Ser Leu
65                  70                  75                  80

Lys Leu Ser Ser Val Thr Ala Ala Asp Thr Ala Val Tyr Tyr Cys Ala
                85                  90                  95

Arg Ala Arg Gly Pro Trp Ser Phe Asp Pro Trp Gly Gln Gly Thr Leu
            100                 105                 110

Val Thr Val Ser Ser
        115

<210> 38
<211> 107
<212> PRT
<213> Artificial Sequence

<220>
<223> Description of Artificial Sequence: Synthetic
      polypeptide

<400> 38
Asp Ile Gln Met Thr Gln Ser Pro Ser Thr Leu Ser Ala Ser Val Gly
1               5                   10                  15

Asp Arg Val Thr Ile Thr Cys Arg Ala Ser Gln Ser Ile Gly Ser Trp
            20                  25                  30

Leu Ala Trp Tyr Gln Gln Lys Pro Gly Lys Ala Pro Lys Leu Leu Ile
        35                  40                  45

Tyr Lys Ala Ser Ser Leu Glu Ser Gly Val Pro Ser Arg Phe Ser Gly
    50                  55                  60

Ser Gly Ser Gly Thr Glu Phe Thr Leu Thr Ile Ser Ser Leu Gln Pro
65                  70                  75                  80

Asp Asp Phe Ala Thr Tyr Tyr Cys Gln Gln Tyr Glu Leu Tyr Ser Tyr
                85                  90                  95

Thr Phe Gly Gly Gly Thr Lys Val Glu Ile Lys
            100                 105

<210> 39
<211> 117
<212> PRT
<213> Artificial Sequence

<220>
<223> Description of Artificial Sequence: Synthetic
      polypeptide

<400> 39
Gln Val Gln Leu Gln Gln Trp Gly Ala Gly Leu Leu Lys Pro Ser Glu
1               5                   10                  15

Thr Leu Ser Leu Thr Cys Ala Val Tyr Gly Gly Ser Phe Ser Gly Tyr
            20                  25                  30

Tyr Trp Ser Trp Ile Arg Gln Pro Pro Gly Lys Gly Leu Glu Trp Ile
        35                  40                  45

Gly Glu Ile Asp His Ser Gly Ser Thr Asn Tyr Asn Pro Ser Leu Lys
    50                  55                  60

Ser Arg Val Thr Ile Ser Val Asp Thr Ser Lys Asn Gln Phe Ser Leu
65                  70                  75                  80

Lys Leu Ser Ser Val Thr Ala Ala Asp Thr Ala Val Tyr Tyr Cys Ala
                85                  90                  95

Arg Ala Arg Gly Pro Trp Ser Phe Asp Pro Trp Gly Gln Gly Thr Leu
            100                 105                 110

Val Thr Val Ser Ser
        115

<210> 40
<211> 106
<212> PRT
<213> Artificial Sequence

<220>
<223> Description of Artificial Sequence: Synthetic
      polypeptide

<400> 40
Asp Ile Gln Met Thr Gln Ser Pro Ser Thr Leu Ser Ala Ser Val Gly
1               5                   10                  15

Asp Arg Val Thr Ile Thr Cys Arg Ala Ser Gln Ser Ile Ser Ser Trp
            20                  25                  30

Leu Ala Trp Tyr Gln Gln Lys Pro Gly Lys Ala Pro Lys Leu Leu Ile
        35                  40                  45

Tyr Lys Ala Ser Ser Leu Glu Ser Gly Val Pro Ser Arg Phe Ser Gly
    50                  55                  60

Ser Gly Ser Gly Thr Glu Phe Thr Leu Thr Ile Ser Ser Leu Gln Pro
65                  70                  75                  80

Asp Asp Phe Ala Thr Tyr Tyr Cys Gln Gln Tyr Asp Thr Phe Ile Thr
                85                  90                  95

Phe Gly Gly Gly Thr Lys Val Glu Ile Lys
            100                 105

<210> 41
<211> 125
<212> PRT
<213> Artificial Sequence

<220>
<223> Description of Artificial Sequence: Synthetic
      polypeptide

<400> 41
Gln Val Gln Leu Val Gln Ser Gly Ala Glu Val Lys Lys Pro Gly Ser
1               5                   10                  15

Ser Val Lys Val Ser Cys Lys Ala Ser Gly Gly Thr Phe Ser Ser Tyr
            20                  25                  30

Ala Ile Ser Trp Val Arg Gln Ala Pro Gly Gln Gly Leu Glu Trp Met
        35                  40                  45

Gly Gly Ile Ile Pro Ile Phe Gly Thr Ala Asn Tyr Ala Gln Lys Phe
    50                  55                  60

Gln Gly Arg Val Thr Ile Thr Ala Asp Glu Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gly Asp Ser Ser Ile Arg His Ala Tyr Tyr Tyr Tyr Gly Met
            100                 105                 110

Asp Val Trp Gly Gln Gly Thr Thr Val Thr Val Ser Ser
        115                 120                 125


<210> 42
<211> 113
<212> PRT
<213> Artificial Sequence

<220>
<223> Description of Artificial Sequence: Synthetic
      polypeptide

<400> 42
Asp Ile Val Met Thr Gln Ser Pro Asp Ser Leu Ala Val Ser Leu Gly
1               5                   10                  15

Glu Arg Ala Thr Ile Asn Cys Lys Ser Ser Gln Ser Val Leu Tyr Ser
            20                  25                  30

Ser Asn Asn Lys Asn Tyr Leu Ala Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Leu Gln Ala Glu Asp Val Ala Val Tyr Tyr Cys Gln Gln
                85                  90                  95

Tyr Tyr Ser Thr Pro Ile Thr Phe Gly Gly Gly Thr Lys Val Glu Ile
            100                 105                 110

Lys

<210> 43
<211> 121
<212> PRT
<213> Artificial Sequence

<220>
<223> Description of Artificial Sequence: Synthetic
      polypeptide

<400> 43
Gln Leu Gln Leu Gln Glu Ser Gly Pro Gly Leu Val Lys Pro Ser Glu
1               5                   10                  15

Thr Leu Ser Leu Thr Cys Thr Val Ser Gly Gly Ser Ile Ser Ser Ser
            20                  25                  30

Ser Tyr Tyr Trp Gly Trp Ile Arg Gln Pro Pro Gly Lys Gly Leu Glu
        35                  40                  45

Trp Ile Gly Ser Ile Tyr Tyr Ser Gly Ser Thr Tyr Tyr Asn Pro Ser
    50                  55                  60

Leu Lys Ser Arg Val Thr Ile Ser Val Asp Thr Ser Lys Asn Gln Phe
65                  70                  75                  80

Ser Leu Lys Leu Ser Ser Val Thr Ala Ala Asp Thr Ala Val Tyr Tyr
                85                  90                  95

Cys Ala Arg Gly Ser Asp Arg Phe His Pro Tyr Phe Asp Tyr Trp Gly
            100                 105                 110

Gln Gly Thr Leu Val Thr Val Ser Ser
        115                 120

<210> 44
<211> 107
<212> PRT
<213> Artificial Sequence

<220>
<223> Description of Artificial Sequence: Synthetic
      polypeptide

<400> 44
Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Ser Arg Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Phe Asp Thr Trp Pro Pro
                85                  90                  95

Thr Phe Gly Gly Gly Thr Lys Val Glu Ile Lys
            100                 105


<210> 45
<211> 126
<212> PRT
<213> Artificial Sequence

<220>
<223> Description of Artificial Sequence: Synthetic
      polypeptide

<400> 45
Gln Val Gln Leu Val Gln Ser Gly Ala Glu Val Lys Lys Pro Gly Ser
1               5                   10                  15

Ser Val Lys Val Ser Cys Lys Ala Ser Gly Gly Thr Phe Ser Ser Tyr
            20                  25                  30

Ala Ile Ser Trp Val Arg Gln Ala Pro Gly Gln Gly Leu Glu Trp Met
        35                  40                  45

Gly Gly Ile Ile Pro Ile Phe Gly Thr Ala Asn Tyr Ala Gln Lys Phe
    50                  55                  60

Gln Gly Arg Val Thr Ile Thr Ala Asp Glu Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Arg Gly Arg Lys Ala Ser Gly Ser Phe Tyr Tyr Tyr Tyr Gly
            100                 105                 110

Met Asp Val Trp Gly Gln Gly Thr Thr Val Thr Val Ser Ser
        115                 120                 125

<210> 46
<211> 113
<212> PRT
<213> Artificial Sequence

<220>
<223> Description of Artificial Sequence: Synthetic
      polypeptide

<400> 46

<210> 47
<211> 121
<212> PRT
<213> Homo sapiens

<400> 47
Gln Val Gln Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Ser Tyr
            20                  25                  30

Gly Met His Trp Val Arg Gln Ala Pro Gly Lys Gly Leu Glu Trp Val
        35                  40                  45

Ala Phe Ile Arg Tyr Asp Gly Ser Asn Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Asp Arg Gly Leu Gly Asp Gly Thr Tyr Phe Asp Tyr Trp Gly
            100                 105                 110

Gln Gly Thr Thr Val Thr Val Ser Ser
        115                 120

<210> 48
<211> 110
<212> PRT
<213> Homo sapiens

<400> 48
Gln Ser Ala Leu Thr Gln Pro Ala Ser Val Ser Gly Ser Pro Gly Gln
1               5                   10                  15

Ser Ile Thr Ile Ser Cys Ser Gly Ser Ser Ser Asn Ile Gly Asn Asn
            20                  25                  30

Ala Val Asn Trp Tyr Gln Gln Leu Pro Gly Lys Ala Pro Lys Leu Leu
        35                  40                  45

Ile Tyr Tyr Asp Asp Leu Leu Pro Ser Gly Val Ser Asp Arg Phe Ser
    50                  55                  60

Gly Ser Lys Ser Gly Thr Ser Ala Phe Leu Ala Ile Ser Gly Leu Gln
65                  70                  75                  80

Ser Glu Asp Glu Ala Asp Tyr Tyr Cys Ala Ala Trp Asp Asp Ser Leu
                85                  90                  95

Asn Gly Pro Val Phe Gly Gly Gly Thr Lys Leu Thr Val Leu
            100                 105                 110

<210> 49
<211> 115
<212> PRT
<213> Homo sapiens

<400> 49
Gln Val His Leu Gln Glu Ser Gly Pro Gly Leu Val Lys Pro Ser Glu
1               5                   10                  15

Thr Leu Ser Leu Thr Cys Thr Val Ser Asp Asp Ser Ile Ser Ser Tyr
            20                  25                  30

Tyr Trp Ser Trp Ile Arg Gln Pro Pro Gly Lys Gly Leu Glu Trp Ile
        35                  40                  45

Gly His Ile Ser Tyr Ser Gly Ser Ala Asn Tyr Asn Pro Ser Leu Lys
    50                  55                  60

Ser Arg Val Thr Ile Ser Val Asp Thr Ser Lys Asn Gln Phe Ser Leu
65                  70                  75                  80

Lys Leu Ser Ser Val Thr Ala Ala Asp Thr Ala Val Tyr Tyr Cys Ala
                85                  90                  95

Asn Trp Asp Asp Ala Phe Asn Ile Trp Gly Gln Gly Thr Met Val Thr
            100                 105                 110

Val Ser Ser
        115


<210> 50
<211> 108
<212> PRT
<213> Homo sapiens

<400> 50
Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Ser Ser Ser
            20                  25                  30

Tyr Leu Ala Trp Tyr Gln Gln Lys Pro Gly Gln Ala Pro Arg Leu Leu
        35                  40                  45

Ile Tyr Gly Ala Ser Ser Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Tyr Gly Ser Ser Pro
                85                  90                  95

Trp Thr Phe Gly Gln Gly Thr Lys Val Glu Ile Lys
            100                 105

<210> 51
<211> 9
<212> PRT
<213> Artificial Sequence

<220>
<223> Description of Artificial Sequence: Synthetic
      peptide

<400> 51
Gly Ser Phe Ser Gly Tyr Tyr Trp Ser
1               5

<210> 52
<211> 16
<212> PRT
<213> Artificial Sequence

<220>
<223> Description of Artificial Sequence: Synthetic
      peptide

<400> 52
Glu Ile Asp His Ser Gly Ser Thr Asn Tyr Asn Pro Ser Leu Lys Ser
1               5                   10                  15

<210> 53
<211> 11
<212> PRT
<213> Artificial Sequence

<220>
<223> Description of Artificial Sequence: Synthetic
      peptide

<400> 53
Ala Arg Ala Arg Gly Pro Trp Ser Phe Asp Pro
1               5                   10

<210> 54
<211> 9
<212> PRT
<213> Artificial Sequence

<220>
<223> Description of Artificial Sequence: Synthetic
      peptide

<400> 54
Gly Thr Phe Ser Ser Tyr Ala Ile Ser

1               5


<210> 55
<211> 17
<212> PRT
<213> Artificial Sequence

<220>
<223> Description of Artificial Sequence: Synthetic
      peptide

<400> 55
Gly Ile Ile Pro Ile Phe Gly Thr Ala Asn Tyr Ala Gln Lys Phe Gln
1               5                   10                  15


Gly




<210> 56
<211> 18
<212> PRT
<213> Artificial Sequence

<220>
<223> Description of Artificial Sequence: Synthetic
      peptide

<400> 56
Ala Arg Gly Asp Ser Ser Ile Arg His Ala Tyr Tyr Tyr Tyr Gly Met
1               5                   10                  15


Asp Val




<210> 57
<211> 17
<212> PRT
<213> Artificial Sequence

<220>
<223> Description of Artificial Sequence: Synthetic
      peptide

<400> 57
Lys Ser Ser Gln Ser Val Leu Tyr Ser Ser Asn Asn Lys Asn Tyr Leu
1               5                   10                  15


Ala




<210> 58
<211> 7
<212> PRT
<213> Artificial Sequence

<220>

<223> Description of Artificial Sequence: Synthetic
      peptide

<400> 58
Trp Ala Ser Thr Arg Glu Ser
1               5


<210> 59
<211> 9
<212> PRT
<213> Artificial Sequence

<220>
<223> Description of Artificial Sequence: Synthetic
      peptide

<400> 59
Gln Gln Tyr Tyr Ser Thr Pro Ile Thr
1               5


<210> 60
<211> 11
<212> PRT
<213> Artificial Sequence

<220>
<223> Description of Artificial Sequence: Synthetic
      peptide

<400> 60
Gly Ser Ile Ser Ser Ser Ser Tyr Tyr Trp Gly
1               5                   10


<210> 61
<211> 16
<212> PRT
<213> Artificial Sequence

<220>
<223> Description of Artificial Sequence: Synthetic
      peptide

<400> 61
Ser Ile Tyr Tyr Ser Gly Ser Thr Tyr Tyr Asn Pro Ser Leu Lys Ser
1               5                   10                  15


<210> 62
<211> 13
<212> PRT
<213> Artificial Sequence

<220>
<223> Description of Artificial Sequence: Synthetic
      peptide

<400> 62
Ala Arg Gly Ser Asp Arg Phe His Pro Tyr Phe Asp Tyr
1               5                   10

<210> 63
<211> 11
<212> PRT
<213> Artificial Sequence

<220>
<223> Description of Artificial Sequence: Synthetic
      peptide

<400> 63
Arg Ala Ser Gln Ser Val Ser Arg Tyr Leu Ala
1               5                   10


<210> 64
<211> 7
<212> PRT
<213> Artificial Sequence

<220>
<223> Description of Artificial Sequence: Synthetic
      peptide

<400> 64
Asp Ala Ser Asn Arg Ala Thr
1               5


<210> 65
<211> 9
<212> PRT
<213> Artificial Sequence

<220>
<223> Description of Artificial Sequence: Synthetic
      peptide

<400> 65
Gln Gln Phe Asp Thr Trp Pro Pro Thr
1               5


<210> 66
<211> 9
<212> PRT
<213> Artificial Sequence

<220>
<223> Description of Artificial Sequence: Synthetic
      peptide

<400> 66
Gly Thr Phe Ser Ser Tyr Ala Ile Ser
1               5


<210> 67
<211> 17
<212> PRT
<213> Artificial Sequence

<220>

<223> Description of Artificial Sequence: Synthetic
      peptide

<400> 67
Gly Ile Ile Pro Ile Phe Gly Thr Ala Asn Tyr Ala Gln Lys Phe Gln
1               5                   10                  15

Gly

<210> 68
<211> 19
<212> PRT
<213> Artificial Sequence

<220>
<223> Description of Artificial Sequence: Synthetic
      peptide

<400> 68
Ala Arg Arg Gly Arg Lys Ala Ser Gly Ser Phe Tyr Tyr Tyr Tyr Gly
1               5                   10                  15

Met Asp Val

<210> 69
<211> 17
<212> PRT
<213> Artificial Sequence

<220>
<223> Description of Artificial Sequence: Synthetic
      peptide

<400> 69
Glu Ser Ser Gln Ser Leu Leu Asn Ser Gly Asn Gln Lys Asn Tyr Leu
1               5                   10                  15

Thr

<210> 70
<211> 7
<212> PRT
<213> Artificial Sequence

<220>
<223> Description of Artificial Sequence: Synthetic
      peptide

<400> 70
Trp Ala Ser Thr Arg Glu Ser
1               5

<210> 71

<211> 9
<212> PRT
<213> Artificial Sequence

<220>
<223> Description of Artificial Sequence: Synthetic
      peptide

<400> 71
Gln Asn Asp Tyr Ser Tyr Pro Tyr Thr
1               5


<210> 72
<211> 119
<212> PRT
<213> Artificial Sequence

<220>
<223> Description of Artificial Sequence: Synthetic
      polypeptide

<400> 72
Asp Val Lys Leu Val Glu Ser Gly Gly Gly Leu Val Lys Leu Gly Gly
1               5                   10                  15


Ser Leu Lys Leu Ser Cys Ala Ala Ser Gly Phe Thr Phe Ser Asn Tyr
            20                  25                  30


Tyr Met Ser Trp Val Arg Gln Thr Pro Glu Lys Arg Leu Glu Leu Val
        35                  40                  45


Ala Ala Ile Asn Ser Asp Gly Gly Ile Thr Tyr Tyr Leu Asp Thr Val
    50                  55                  60


Lys Gly Arg Phe Thr Ile Ser Arg Asp Asn Ala Lys Asn Thr Leu Tyr
65                  70                  75                  80


Leu Gln Met Ser Ser Leu Lys Ser Glu Asp Thr Ala Leu Phe Tyr Cys
                85                  90                  95


Ala Arg His Arg Ser Gly Tyr Phe Ser Met Asp Tyr Trp Gly Gln Gly
            100                 105                 110


Thr Ser Val Thr Val Ser Ser
        115


<210> 73
<211> 108
<212> PRT
<213> Artificial Sequence

<220>
<223> Description of Artificial Sequence: Synthetic
      polypeptide

<400> 73
Asp Ile Val Met Thr Gln Ser Gln Arg Phe Met Ser Thr Thr Val Gly
1               5                   10                  15

Asp Arg Val Ser Ile Thr Cys Lys Ala Ser Gln Asn Val Val Ser Ala
            20                  25                  30

Val Ala Trp Tyr Gln Gln Lys Pro Gly Gln Ser Pro Lys Leu Leu Ile
        35                  40                  45

Tyr Ser Ala Ser Asn Arg Tyr Thr Gly Val Pro Asp Arg Phe Thr Gly
    50                  55                  60

Ser Gly Ser Gly Thr Asp Phe Thr Leu Thr Ile Ser Asn Met Gln Ser
65                  70                  75                  80

Glu Asp Leu Ala Asp Phe Phe Cys Gln Gln Tyr Ser Asn Tyr Pro Trp
                85                  90                  95

Thr Phe Gly Gly Gly Thr Lys Leu Glu Ile Lys Arg
            100                 105

<210> 74
<211> 7
<212> PRT
<213> Artificial Sequence

<220>
<223> Description of Artificial Sequence: Synthetic
      peptide

<400> 74
Gly Phe Thr Phe Ser Asn Tyr
1               5

<210> 75
<211> 6
<212> PRT
<213> Artificial Sequence

<220>
<223> Description of Artificial Sequence: Synthetic
      peptide

<400> 75
Asn Ser Asp Gly Gly Ile
1               5

<210> 76
<211> 10
<212> PRT
<213> Artificial Sequence

<220>
<223> Description of Artificial Sequence: Synthetic
      peptide

<400> 76
His Arg Ser Gly Tyr Phe Ser Met Asp Tyr
1               5                   10


<210> 77
<211> 8
<212> PRT
<213> Artificial Sequence

<220>
<223> Description of Artificial Sequence: Synthetic
      peptide

<400> 77
Gln Asn Val Val Ser Ala Val Ala
1               5


<210> 78
<211> 7
<212> PRT
<213> Artificial Sequence

<220>
<223> Description of Artificial Sequence: Synthetic
      peptide

<400> 78
Ser Ala Ser Asn Arg Tyr Thr
1               5


<210> 79
<211> 9
<212> PRT
<213> Artificial Sequence

<220>
<223> Description of Artificial Sequence: Synthetic
      peptide

<400> 79
Gln Gln Tyr Ser Asn Tyr Pro Trp Thr
1               5


<210> 80
<211> 121
<212> PRT
<213> Artificial Sequence

<220>
<223> Description of Artificial Sequence: Synthetic
      polypeptide

<400> 80
Gln Val Glu Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Gly Met Ser Trp Val Arg Gln Ala Pro Gly Lys Gly Leu Glu Trp Val
        35                  40                  45

Ser Gly Ile Ser Ser Leu Gly Ser Thr Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Thr Gly Ser Pro Gly Thr Phe Met His Gly Asp His Trp Gly
            100                 105                 110

Gln Gly Thr Leu Val Thr Val Ser Ser
        115                 120

<210> 81
<211> 107
<212> PRT
<213> Artificial Sequence

<220>
<223> Description of Artificial Sequence: Synthetic
polypeptide

<400> 81
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Ile Asn Asn Tyr
            20                  25                  30

Leu Ser Trp Tyr Gln Gln Lys Pro Gly Lys Ala Pro Lys Leu Leu Ile
        35                  40                  45

Tyr Gly Ala Ser Asn Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Val Tyr Tyr Cys Gln Gln Tyr Tyr Gly Arg Pro Thr
                85                  90                  95

Thr Phe Gly Gln Gly Thr Lys Val Glu Ile Lys
            100                 105

<210> 82
<211> 10
<212> PRT
<213> Artificial Sequence

<220>
<223> Description of Artificial Sequence: Synthetic
      peptide

<400> 82
Gly Phe Thr Phe Ser Ser Tyr Gly Met Ser
1               5                   10

<210> 83
<211> 17
<212> PRT
<213> Artificial Sequence

<220>
<223> Description of Artificial Sequence: Synthetic
      peptide

<400> 83
Gly Ile Ser Ser Leu Gly Ser Thr Thr Tyr Tyr Ala Asp Ser Val Lys
1               5                   10                  15

Gly

<210> 84
<211> 12
<212> PRT
<213> Artificial Sequence

<220>
<223> Description of Artificial Sequence: Synthetic
      peptide

<400> 84
Thr Gly Ser Pro Gly Thr Phe Met His Gly Asp His
1               5                   10

<210> 85
<211> 11
<212> PRT
<213> Artificial Sequence

<220>
<223> Description of Artificial Sequence: Synthetic
      peptide

<400> 85
Arg Ala Ser Gln Asp Ile Asn Asn Tyr Leu Ser
1               5                   10

<210> 86
<211> 8
<212> PRT
<213> Artificial Sequence

<220>
<223> Description of Artificial Sequence: Synthetic
      peptide

<400> 86
Tyr Gly Ala Ser Asn Leu Gln Ser
1               5


<210> 87
<211> 8
<212> PRT
<213> Artificial Sequence

<220>
<223> Description of Artificial Sequence: Synthetic
      peptide

<400> 87
Gln Gln Tyr Tyr Gly Arg Pro Thr
1               5


<210> 88
<211> 119
<212> PRT
<213> Artificial Sequence

<220>
<223> Description of Artificial Sequence: Synthetic
      polypeptide

<400> 88
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Tyr Met Ser Trp Val Arg Gln Ala Pro Gly Gln Arg Leu Glu Leu Val
        35                  40                  45

Ser Ala Ile Asn Ser Asp Gly Gly Ile Thr Tyr Tyr Leu Asp Ser Val
    50                  55                  60

Lys Gly Arg Phe Thr Ile Ser Arg Asp Asn Ala Lys Asn Thr Leu Tyr
65                  70                  75                  80

Leu Gln Met Gly Ser Leu Arg Ala Glu Asp Met Ala Val Tyr Tyr Cys
                85                  90                  95

Ala Arg His Arg Ser Gly Tyr Phe Ser Met Asp Tyr Trp Gly Gln Gly
            100                 105                 110

Thr Leu Val Thr Val Ser Ser
        115

<210> 89
<211> 107
<212> PRT
<213> Artificial Sequence

<220>
<223> Description of Artificial Sequence: Synthetic
      polypeptide

<400> 89
Asp Ile Val Met Thr Gln Ser Pro Ser Ser Leu Ser Ala Ser Val Gly
1               5                   10                  15

Asp Arg Val Thr Ile Thr Cys Arg Ala Ser Gln Asn Val Val Ser Ala
            20                  25                  30

Val Ala Trp Tyr Leu Gln Lys Pro Gly Lys Ser Pro Lys Leu Leu Ile
        35                  40                  45

Tyr Ser Ala Ser Asn Arg Tyr Thr Gly Val Pro Asp Arg Phe Thr Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Phe Cys Gln Gln Tyr Ser Asn Tyr Pro Trp
                85                  90                  95

Thr Phe Gly Gly Gly Thr Lys Leu Glu Ile Lys
            100                 105

<210> 90
<211> 7
<212> PRT
<213> Artificial Sequence

<220>
<223> Description of Artificial Sequence: Synthetic
      peptide

<400> 90
Gly Phe Thr Phe Ser Asn Tyr
1               5

<210> 91
<211> 6

<212> PRT
<213> Artificial Sequence

<220>
<223> Description of Artificial Sequence: Synthetic
      peptide

<400> 91
Asn Ser Asp Gly Gly Ile
1               5


<210> 92
<211> 10
<212> PRT
<213> Artificial Sequence

<220>
<223> Description of Artificial Sequence: Synthetic
      peptide

<400> 92
His Arg Ser Gly Tyr Phe Ser Met Asp Tyr
1               5                   10


<210> 93
<211> 8
<212> PRT
<213> Artificial Sequence

<220>
<223> Description of Artificial Sequence: Synthetic
      peptide

<400> 93
Gln Asn Val Val Ser Ala Val Ala
1               5


<210> 94
<211> 7
<212> PRT
<213> Artificial Sequence

<220>
<223> Description of Artificial Sequence: Synthetic
      peptide

<400> 94
Ser Ala Ser Asn Arg Tyr Thr
1               5


<210> 95
<211> 9
<212> PRT
<213> Artificial Sequence

<220>
<223> Description of Artificial Sequence: Synthetic
      peptide

<400> 95
Gln Gln Tyr Ser Asn Tyr Pro Trp Thr
1               5

<210> 96
<211> 459
<212> PRT
<213> Unknown

<220>
<223> Description of Unknown:
      CAIX polypeptide

<400> 96
Met Ala Pro Leu Cys Pro Ser Pro Trp Leu Pro Leu Leu Ile Pro Ala
1               5                   10                  15

Pro Ala Pro Gly Leu Thr Val Gln Leu Leu Leu Ser Leu Leu Leu Leu
            20                  25                  30

Val Pro Val His Pro Gln Arg Leu Pro Arg Met Gln Glu Asp Ser Pro
        35                  40                  45

Leu Gly Gly Gly Ser Ser Gly Glu Asp Asp Pro Leu Gly Glu Glu Asp
    50                  55                  60

Leu Pro Ser Glu Glu Asp Ser Pro Arg Glu Glu Asp Pro Pro Gly Glu
65                  70                  75                  80

Glu Asp Leu Pro Gly Glu Glu Asp Leu Pro Gly Glu Glu Asp Leu Pro
                85                  90                  95

Glu Val Lys Pro Lys Ser Glu Glu Glu Gly Ser Leu Lys Leu Glu Asp
            100                 105                 110

Leu Pro Thr Val Glu Ala Pro Gly Asp Pro Gln Glu Pro Gln Asn Asn
        115                 120                 125

Ala His Arg Asp Lys Glu Gly Asp Asp Gln Ser His Trp Arg Tyr Gly
    130                 135                 140

Gly Asp Pro Pro Trp Pro Arg Val Ser Pro Ala Cys Ala Gly Arg Phe
145                 150                 155                 160

Gln Ser Pro Val Asp Ile Arg Pro Gln Leu Ala Ala Phe Cys Pro Ala
                165                 170                 175

Leu Arg Pro Leu Glu Leu Leu Gly Phe Gln Leu Pro Pro Leu Pro Glu
            180                 185                 190

Leu Arg Leu Arg Asn Asn Gly His Ser Val Gln Leu Thr Leu Pro Pro
        195                 200                 205

Gly Leu Glu Met Ala Leu Gly Pro Gly Arg Glu Tyr Arg Ala Leu Gln
    210                 215                 220

Leu His Leu His Trp Gly Ala Ala Gly Arg Pro Gly Ser Glu His Thr
225                 230                 235                 240

Val Glu Gly His Arg Phe Pro Ala Glu Ile His Val Val His Leu Ser
                245                 250                 255

Thr Ala Phe Ala Arg Val Asp Glu Ala Leu Gly Arg Pro Gly Gly Leu
            260                 265                 270

Ala Val Leu Ala Ala Phe Leu Glu Glu Gly Pro Glu Glu Asn Ser Ala
        275                 280                 285

Tyr Glu Gln Leu Leu Ser Arg Leu Glu Glu Ile Ala Glu Glu Gly Ser
    290                 295                 300

Glu Thr Gln Val Pro Gly Leu Asp Ile Ser Ala Leu Leu Pro Ser Asp
305                 310                 315                 320

Phe Ser Arg Tyr Phe Gln Tyr Glu Gly Ser Leu Thr Thr Pro Pro Cys
                325                 330                 335

Ala Gln Gly Val Ile Trp Thr Val Phe Asn Gln Thr Val Met Leu Ser
            340                 345                 350

Ala Lys Gln Leu His Thr Leu Ser Asp Thr Leu Trp Gly Pro Gly Asp
        355                 360                 365

Ser Arg Leu Gln Leu Asn Phe Arg Ala Thr Gln Pro Leu Asn Gly Arg
    370                 375                 380

Val Ile Glu Ala Ser Phe Pro Ala Gly Val Asp Ser Ser Pro Arg Ala
385                 390                 395                 400

Ala Glu Pro Val Gln Leu Asn Ser Cys Leu Ala Ala Gly Asp Ile Leu
                405                 410                 415

Ala Leu Val Phe Gly Leu Leu Phe Ala Val Thr Ser Val Ala Phe Leu
            420                 425                 430

Val Gln Met Arg Arg Gln His Arg Arg Gly Thr Lys Gly Gly Val Ser
        435                 440                 445

Tyr Arg Pro Ala Glu Val Ala Glu Thr Gly Ala
    450                 455

<210> 97
<211> 123
<212> PRT
<213> Artificial Sequence

<220>
<223> Description of Artificial Sequence: Synthetic
      polypeptide

<400> 97
Gln Val Gln Leu Lys Glu Ser Gly Pro Gly Leu Val Gln Pro Ser Gln
1               5                   10                  15

Thr Leu Thr Leu Thr Cys Thr Val Ser Gly Phe Ser Leu Ser Asn Tyr
            20                  25                  30

Asp Met His Trp Val Arg Gln Ser Pro Gly Lys Gly Leu Asp Trp Met
        35                  40                  45

Gly Val Ile Trp Gly Asn Gly Lys Thr Gln Tyr Asn Ser Gly Leu Thr
    50                  55                  60

Ser Arg Leu Ser Ile Ser Arg Asp Thr Ser Lys Ser Gln Val Phe Leu
65                  70                  75                  80

Lys Met Asn Ser Leu Gln Thr Glu Asp Thr Ala Ile Tyr Phe Cys Thr
                85                  90                  95

Arg Ser Gly Tyr Tyr Tyr Asp Gly Ser Tyr Tyr Ser Leu Phe Asp Tyr
            100                 105                 110

Trp Gly Gln Gly Val Met Val Thr Val Ser Ser
        115                 120

<210> 98
<211> 111
<212> PRT
<213> Artificial Sequence

<220>
<223> Description of Artificial Sequence: Synthetic
      polypeptide

<400> 98
Asp Val Val Leu Thr Gln Thr Pro Pro Thr Leu Ser Ala Thr Ile Gly
1               5                   10                  15

Gln Ser Val Ser Ile Ser Cys Arg Ser Ser Gln Ser Leu Leu His Ser
            20                  25                  30

Asn Gly Asn Thr Tyr Leu Asn Trp Leu Leu Gln Arg Pro Gly Gln Pro
        35                  40                  45

Pro Gln Leu Leu Ile Tyr Leu Val Ser Arg Leu Glu Ser Gly Val Pro
    50                  55                  60

Asn Arg Phe Ser Gly Ser Gly Ser Gly Thr Asp Phe Thr Leu Lys Ile
65                  70                  75                  80

Ser Gly Val Glu Ala Glu Asp Leu Gly Val Tyr Tyr Cys Val Gln Ser
                85                  90                  95

Thr His Ala Pro Ala Phe Gly Gly Gly Thr Lys Leu Glu Leu Lys
            100                 105                 110

<210> 99
<211> 5
<212> PRT
<213> Artificial Sequence

<220>
<223> Description of Artificial Sequence: Synthetic
      peptide

<400> 99
Asn Tyr Asp Met His
1               5

<210> 100
<211> 16
<212> PRT
<213> Artificial Sequence

<220>
<223> Description of Artificial Sequence: Synthetic
      peptide

<400> 100
Val Ile Trp Gly Asn Gly Lys Thr Gln Tyr Asn Ser Gly Leu Thr Ser
1               5                   10                  15

<210> 101
<211> 15
<212> PRT
<213> Artificial Sequence

<220>
<223> Description of Artificial Sequence: Synthetic
      peptide

<400> 101
Ser Gly Tyr Tyr Tyr Asp Gly Ser Tyr Tyr Ser Leu Phe Asp Tyr
1               5                   10                  15

<210> 102
<211> 16
<212> PRT
<213> Artificial Sequence

<220>
<223> Description of Artificial Sequence: Synthetic
      peptide

<400> 102
Arg Ser Ser Gln Ser Leu Leu His Ser Asn Gly Asn Thr Tyr Leu Asn
1               5                   10                  15


<210> 103
<211> 7
<212> PRT
<213> Artificial Sequence

<220>
<223> Description of Artificial Sequence: Synthetic
      peptide

<400> 103
Leu Val Ser Arg Leu Glu Ser
1               5


<210> 104
<211> 8
<212> PRT
<213> Artificial Sequence

<220>
<223> Description of Artificial Sequence: Synthetic
      peptide

<400> 104
Val Gln Ser Thr His Ala Pro Ala
1               5


<210> 105
<211> 986
<212> PRT
<213> Unknown

<220>
<223> Description of Unknown:
      ANO1 polypeptide

<400> 105
Met Arg Val Asn Glu Lys Tyr Ser Thr Leu Pro Ala Glu Asp Arg Ser
1               5                   10                  15


Val His Ile Ile Asn Ile Cys Ala Ile Glu Asp Ile Gly Tyr Leu Pro
            20                  25                  30


Ser Glu Gly Thr Leu Leu Asn Ser Leu Ser Val Asp Pro Asp Ala Glu
        35                  40                  45

Cys Lys Tyr Gly Leu Tyr Phe Arg Asp Gly Arg Arg Lys Val Asp Tyr
    50                  55                  60

Ile Leu Val Tyr His His Lys Arg Pro Ser Gly Asn Arg Thr Leu Val
65                  70                  75                  80

Arg Arg Val Gln His Ser Asp Thr Pro Ser Gly Ala Arg Ser Val Lys
                85                  90                  95

Gln Asp His Pro Leu Pro Gly Lys Gly Ala Ser Leu Asp Ala Gly Ser
            100                 105                 110

Gly Glu Pro Pro Met Asp Tyr His Glu Asp Asp Lys Arg Phe Arg Arg
        115                 120                 125

Glu Glu Tyr Glu Gly Asn Leu Leu Glu Ala Gly Leu Glu Leu Glu Arg
    130                 135                 140

Asp Glu Asp Thr Lys Ile His Gly Val Gly Phe Val Lys Ile His Ala
145                 150                 155                 160

Pro Trp Asn Val Leu Cys Arg Glu Ala Glu Phe Leu Lys Leu Lys Met
                165                 170                 175

Pro Thr Lys Lys Met Tyr His Ile Asn Glu Thr Arg Gly Leu Leu Lys
            180                 185                 190

Lys Ile Asn Ser Val Leu Gln Lys Ile Thr Asp Pro Ile Gln Pro Lys
        195                 200                 205

Val Ala Glu His Arg Pro Gln Thr Met Lys Arg Leu Ser Tyr Pro Phe
    210                 215                 220

Ser Arg Glu Lys Gln His Leu Phe Asp Leu Ser Asp Lys Asp Ser Phe
225                 230                 235                 240

Phe Asp Ser Lys Thr Arg Ser Thr Ile Val Tyr Glu Ile Leu Lys Arg
                245                 250                 255

Thr Thr Cys Thr Lys Ala Lys Tyr Ser Met Gly Ile Thr Ser Leu Leu
            260                 265                 270

Ala Asn Gly Val Tyr Ala Ala Ala Tyr Pro Leu His Asp Gly Asp Tyr
        275                 280                 285

Asn Gly Glu Asn Val Glu Phe Asn Asp Arg Lys Leu Leu Tyr Glu Glu

290                 295                 300

Trp Ala Arg Tyr Gly Val Phe Tyr Lys Tyr Gln Pro Ile Asp Leu Val
305                 310                 315                 320

Arg Lys Tyr Phe Gly Glu Lys Ile Gly Leu Tyr Phe Ala Trp Leu Gly
                325                 330                 335

Val Tyr Thr Gln Met Leu Ile Pro Ala Ser Ile Val Gly Ile Ile Val
            340                 345                 350

Phe Leu Tyr Gly Cys Ala Thr Met Asp Glu Asn Ile Pro Ser Met Glu
        355                 360                 365

Met Cys Asp Gln Arg His Asn Ile Thr Met Cys Pro Leu Cys Asp Lys
    370                 375                 380

Thr Cys Ser Tyr Trp Lys Met Ser Ser Ala Cys Ala Thr Ala Arg Ala
385                 390                 395                 400

Ser His Leu Phe Asp Asn Pro Ala Thr Val Phe Phe Ser Val Phe Met
                405                 410                 415

Ala Leu Trp Ala Ala Thr Phe Met Glu His Trp Lys Arg Lys Gln Met
            420                 425                 430

Arg Leu Asn Tyr Arg Trp Asp Leu Thr Gly Phe Glu Glu Glu Glu Glu
        435                 440                 445

Ala Val Lys Asp His Pro Arg Ala Glu Tyr Glu Ala Arg Val Leu Glu
    450                 455                 460

Lys Ser Leu Lys Lys Glu Ser Arg Asn Lys Glu Lys Arg Arg His Ile
465                 470                 475                 480

Pro Glu Glu Ser Thr Asn Lys Trp Lys Gln Arg Val Lys Thr Ala Met
                485                 490                 495

Ala Gly Val Lys Leu Thr Asp Lys Val Lys Leu Thr Trp Arg Asp Arg
            500                 505                 510

Phe Pro Ala Tyr Leu Thr Asn Leu Val Ser Ile Ile Phe Met Ile Ala
        515                 520                 525

Val Thr Phe Ala Ile Val Leu Gly Val Ile Ile Tyr Arg Ile Ser Met
    530                 535                 540

Ala Ala Ala Leu Ala Met Asn Ser Ser Pro Ser Val Arg Ser Asn Ile
545                 550                 555                 560

Arg Val Thr Val Thr Ala Thr Ala Val Ile Ile Asn Leu Val Val Ile
                565                 570                 575

Ile Leu Leu Asp Glu Val Tyr Gly Cys Ile Ala Arg Trp Leu Thr Lys
            580                 585                 590

Ile Glu Val Pro Lys Thr Glu Lys Ser Phe Glu Glu Arg Leu Ile Phe
        595                 600                 605

Lys Ala Phe Leu Leu Lys Phe Val Asn Ser Tyr Thr Pro Ile Phe Tyr
    610                 615                 620

Val Ala Phe Phe Lys Gly Arg Phe Val Gly Arg Pro Gly Asp Tyr Val
625                 630                 635                 640

Tyr Ile Phe Arg Ser Phe Arg Met Glu Glu Cys Ala Pro Gly Gly Cys
                645                 650                 655

Leu Met Glu Leu Cys Ile Gln Leu Ser Ile Ile Met Leu Gly Lys Gln
            660                 665                 670

Leu Ile Gln Asn Asn Leu Phe Glu Ile Gly Ile Pro Lys Met Lys Lys
        675                 680                 685

Leu Ile Arg Tyr Leu Lys Leu Lys Gln Gln Ser Pro Pro Asp His Glu
    690                 695                 700

Glu Cys Val Lys Arg Lys Gln Arg Tyr Glu Val Asp Tyr Asn Leu Glu
705                 710                 715                 720

Pro Phe Ala Gly Leu Thr Pro Glu Tyr Met Glu Met Ile Ile Gln Phe
                725                 730                 735

Gly Phe Val Thr Leu Phe Val Ala Ser Phe Pro Leu Ala Pro Leu Phe
            740                 745                 750

Ala Leu Leu Asn Asn Ile Ile Glu Ile Arg Leu Asp Ala Lys Lys Phe
        755                 760                 765

Val Thr Glu Leu Arg Arg Pro Val Ala Val Arg Ala Lys Asp Ile Gly
    770                 775                 780

Ile Trp Tyr Asn Ile Leu Arg Gly Ile Gly Lys Leu Ala Val Ile Ile
785                 790                 795                 800

Asn Ala Phe Val Ile Ser Phe Thr Ser Asp Phe Ile Pro Arg Leu Val
                805                 810                 815

Tyr Leu Tyr Met Tyr Ser Lys Asn Gly Thr Met His Gly Phe Val Asn
            820                 825                 830

His Thr Leu Ser Ser Phe Asn Val Ser Asp Phe Gln Asn Gly Thr Ala
        835                 840                 845

Pro Asn Asp Pro Leu Asp Leu Gly Tyr Glu Val Gln Ile Cys Arg Tyr
    850                 855                 860

Lys Asp Tyr Arg Glu Pro Pro Trp Ser Glu Asn Lys Tyr Asp Ile Ser
865                 870                 875                 880

Lys Asp Phe Trp Ala Val Leu Ala Ala Arg Leu Ala Phe Val Ile Val
                885                 890                 895

Phe Gln Asn Leu Val Met Phe Met Ser Asp Phe Val Asp Trp Val Ile
            900                 905                 910

Pro Asp Ile Pro Lys Asp Ile Ser Gln Gln Ile His Lys Glu Lys Val
        915                 920                 925

Leu Met Val Glu Leu Phe Met Arg Glu Glu Gln Asp Lys Gln Gln Leu
    930                 935                 940

Leu Glu Thr Trp Met Glu Lys Glu Arg Gln Lys Asp Glu Pro Pro Cys
945                 950                 955                 960

Asn His His Asn Thr Lys Ala Cys Pro Asp Ser Leu Gly Ser Pro Ala
                965                 970                 975

Pro Ser His Ala Tyr His Gly Gly Val Leu
            980                 985

<210> 106
<211> 120
<212> PRT
<213> Artificial Sequence

<220>
<223> Description of Artificial Sequence: Synthetic
      polypeptide

<400> 106
Gln Val Gln Leu Gln Gln Ser Gly Pro Glu Leu Glu Lys Pro Gly Ala
1               5                   10                  15

Ser Val Lys Ile Ser Cys Lys Ala Ser Gly Tyr Ser Phe Thr Gly Tyr
            20                  25                  30

Thr Met Asn Trp Val Lys Gln Ser His Gly Lys Ser Leu Glu Trp Ile
        35                  40                  45

Gly Leu Ile Thr Pro Tyr Asn Gly Ala Ser Ser Tyr Asn Gln Lys Phe
    50                  55                  60

Arg Gly Lys Ala Thr Leu Thr Val Asp Lys Ser Ser Ser Thr Ala Tyr
65                  70                  75                  80

Met Asp Leu Leu Ser Leu Thr Ser Glu Asp Ser Ala Val Tyr Phe Cys
                85                  90                  95

Ala Arg Gly Gly Tyr Asp Gly Arg Gly Phe Asp Tyr Trp Gly Ser Gly
            100                 105                 110

Thr Pro Val Thr Val Ser Ser Ala
        115                 120

<210> 107
<211> 107
<212> PRT
<213> Artificial Sequence

<220>
<223> Description of Artificial Sequence: Synthetic
      polypeptide

<400> 107
Asp Ile Glu Leu Thr Gln Ser Pro Ala Ile Met Ser Ala Ser Pro Gly
1               5                   10                  15

Glu Lys Val Thr Met Thr Cys Ser Ala Ser Ser Ser Val Ser Tyr Met
            20                  25                  30

His Trp Tyr Gln Gln Lys Ser Gly Thr Ser Pro Lys Arg Trp Ile Tyr
        35                  40                  45

Asp Thr Ser Lys Leu Ala Ser Gly Val Pro Gly Arg Phe Ser Gly Ser
    50                  55                  60

Gly Ser Gly Asn Ser Tyr Ser Leu Thr Ile Ser Ser Val Glu Ala Glu
65                  70                  75                  80

Asp Asp Ala Thr Tyr Tyr Cys Gln Gln Trp Ser Lys His Pro Leu Thr
                85                  90                  95

Phe Gly Ser Gly Thr Lys Val Glu Ile Lys Arg

100                 105

<210> 108
<211> 7
<212> PRT
<213> Artificial Sequence

<220>
<223> Description of Artificial Sequence: Synthetic
      peptide

<400> 108
Gly Tyr Ser Phe Thr Gly Tyr
1               5

<210> 109
<211> 6
<212> PRT
<213> Artificial Sequence

<220>
<223> Description of Artificial Sequence: Synthetic
      peptide

<400> 109
Thr Pro Tyr Asn Gly Ala
1               5

<210> 110
<211> 10
<212> PRT
<213> Artificial Sequence

<220>
<223> Description of Artificial Sequence: Synthetic
      peptide

<400> 110
Gly Gly Tyr Asp Gly Arg Gly Phe Asp Tyr
1               5                   10

<210> 111
<211> 7
<212> PRT
<213> Artificial Sequence

<220>
<223> Description of Artificial Sequence: Synthetic
      peptide

<400> 111
Ser Ser Val Ser Tyr Met His
1               5

<210> 112
<211> 7
<212> PRT
<213> Artificial Sequence

<220>
<223> Description of Artificial Sequence: Synthetic
      peptide

<400> 112
Asp Thr Ser Lys Leu Ala Ser
1               5


<210> 113
<211> 9
<212> PRT
<213> Artificial Sequence

<220>
<223> Description of Artificial Sequence: Synthetic
      peptide

<400> 113
Gln Gln Trp Ser Lys His Pro Leu Thr
1               5


<210> 114
<211> 131
<212> PRT
<213> Artificial Sequence

<220>
<223> Description of Artificial Sequence: Synthetic
      polypeptide

<400> 114
Glu Val His Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Arg Tyr
            20                  25                  30

Trp Met Ser Trp Val Arg Gln Ala Gln Gly Lys Gly Leu Glu Trp Val
        35                  40                  45

Ala Ser Ile Lys Gln Ala Gly Ser Glu Lys Thr Tyr Val Asp Ser Val
    50                  55                  60

Lys Gly Arg Phe Thr Ile Ser Arg Asp Asn Ala Lys Asn Ser Leu Ser
65                  70                  75                  80

Leu Gln Met Asn Ser Leu Arg Ala Glu Asp Thr Ala Val Tyr Tyr Cys
                85                  90                  95

Ala Arg Glu Gly Ala Tyr Tyr Tyr Asp Ser Ala Ser Tyr Tyr Pro Tyr
            100                 105                 110

Tyr Tyr Tyr Tyr Ser Met Asp Val Trp Gly Gln Gly Thr Thr Val Thr

115                 120                 125

Val Ser Ser
    130

<210> 115
<211> 108
<212> PRT
<213> Artificial Sequence

<220>
<223> Description of Artificial Sequence: Synthetic
      polypeptide

<400> 115
Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Ser Ser Ser
            20                  25                  30

Tyr Leu Ala Trp Tyr Gln Gln Lys Pro Gly Gln Ala Pro Arg Leu Leu
        35                  40                  45

Ile Tyr Gly Ala Ser Ser Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Tyr Gly Ser Ser Gln
                85                  90                  95

Tyr Thr Phe Gly Gln Gly Thr Lys Leu Glu Ile Lys
            100                 105

<210> 116
<211> 7
<212> PRT
<213> Artificial Sequence

<220>
<223> Description of Artificial Sequence: Synthetic
      peptide

<400> 116
Gly Phe Thr Phe Ser Arg Tyr
1               5

<210> 117
<211> 6
<212> PRT
<213> Artificial Sequence

<220>
<223> Description of Artificial Sequence: Synthetic
      peptide

<400> 117
Lys Gln Ala Gly Ser Glu
1               5


<210> 118
<211> 22
<212> PRT
<213> Artificial Sequence

<220>
<223> Description of Artificial Sequence: Synthetic
      peptide

<400> 118
Glu Gly Ala Tyr Tyr Tyr Asp Ser Ala Ser Tyr Tyr Pro Tyr Tyr Tyr
1               5                   10                  15


Tyr Tyr Ser Met Asp Val
            20


<210> 119
<211> 9
<212> PRT
<213> Artificial Sequence

<220>
<223> Description of Artificial Sequence: Synthetic
      peptide

<400> 119
Gln Ser Val Ser Ser Ser Tyr Leu Ala
1               5


<210> 120
<211> 7
<212> PRT
<213> Artificial Sequence

<220>
<223> Description of Artificial Sequence: Synthetic
      peptide

<400> 120
Gly Ala Ser Ser Arg Ala Thr
1               5


<210> 121
<211> 9
<212> PRT
<213> Artificial Sequence

<220>
<223> Description of Artificial Sequence: Synthetic

peptide

<400> 121
Gln Gln Tyr Gly Ser Ser Gln Tyr Thr
1               5

<210> 122
<211> 121
<212> PRT
<213> Artificial Sequence

<220>
<223> Description of Artificial Sequence: Synthetic
      polypeptide

<400> 122
Gln Val Glu Leu Val Gln Ser Gly Ala Glu Val Lys Lys Pro Gly Glu
1               5                   10                  15

Ser Leu Lys Ile Ser Cys Lys Gly Ser Gly Tyr Ser Phe Thr Ser Tyr
            20                  25                  30

Trp Ile Gly Trp Val Arg Gln Ala Pro Gly Lys Gly Leu Glu Trp Met
        35                  40                  45

Gly Ile Ile Asp Pro Gly Asp Ser Arg Thr Arg Tyr Ser Pro Ser Phe
    50                  55                  60

Gln Gly Gln Val Thr Ile Ser Ala Asp Lys Ser Ile Ser Thr Ala Tyr
65                  70                  75                  80

Leu Gln Trp Ser Ser Leu Lys Ala Ser Asp Thr Ala Met Tyr Tyr Cys
                85                  90                  95

Ala Arg Gly Gln Leu Tyr Gly Gly Thr Tyr Met Asp Gly Trp Gly Gln
            100                 105                 110

Gly Thr Leu Val Thr Val Ser Ser Ala
        115                 120

<210> 123
<211> 112
<212> PRT
<213> Artificial Sequence

<220>
<223> Description of Artificial Sequence: Synthetic
      polypeptide

<400> 123
Asp Ile Ala Leu Thr Gln Pro Ala Ser Val Ser Gly Ser Pro Gly Gln
1               5                   10                  15

Ser Ile Thr Ile Ser Cys Thr Gly Thr Ser Ser Asp Ile Gly Gly Tyr
            20                  25                  30

Asn Ser Val Ser Trp Tyr Gln Gln His Pro Gly Lys Ala Pro Lys Leu
        35                  40                  45

Met Ile Tyr Gly Val Asn Asn Arg Pro Ser Gly Val Ser Asn Arg Phe
    50                  55                  60

Ser Gly Ser Lys Ser Gly Asn Thr Ala Ser Leu Thr Ile Ser Gly Leu
65                  70                  75                  80

Gln Ala Glu Asp Glu Ala Asp Tyr Tyr Cys Ser Ser Tyr Asp Ile Glu
                85                  90                  95

Ser Ala Thr Pro Val Phe Gly Gly Gly Thr Lys Leu Thr Val Leu Gly
            100                 105                 110

<210> 124
<211> 7
<212> PRT
<213> Artificial Sequence

<220>
<223> Description of Artificial Sequence: Synthetic
      peptide

<400> 124
Gly Tyr Ser Phe Thr Ser Tyr
1               5

<210> 125
<211> 6
<212> PRT
<213> Artificial Sequence

<220>
<223> Description of Artificial Sequence: Synthetic
      peptide

<400> 125
Asp Pro Gly Asp Ser Arg
1               5

<210> 126
<211> 11
<212> PRT
<213> Artificial Sequence

<220>
<223> Description of Artificial Sequence: Synthetic
      peptide

<400> 126
Gly Gln Leu Tyr Gly Gly Thr Tyr Met Asp Gly

1               5                   10


<210> 127
<211> 11
<212> PRT
<213> Artificial Sequence

<220>
<223> Description of Artificial Sequence: Synthetic
      peptide

<400> 127
Ser Ser Asp Ile Gly Gly Tyr Asn Ser Val Ser
1               5                   10


<210> 128
<211> 7
<212> PRT
<213> Artificial Sequence

<220>
<223> Description of Artificial Sequence: Synthetic
      peptide

<400> 128
Gly Val Asn Asn Arg Pro Ser
1               5


<210> 129
<211> 11
<212> PRT
<213> Artificial Sequence

<220>
<223> Description of Artificial Sequence: Synthetic
      peptide

<400> 129
Ser Ser Tyr Asp Ile Glu Ser Ala Thr Pro Val
1               5                   10


<210> 130
<211> 630
<212> PRT
<213> Unknown

<220>
<223> Description of Unknown:
      mesothelin polypeptide

<400> 130
Met Ala Leu Pro Thr Ala Arg Pro Leu Leu Gly Ser Cys Gly Thr Pro
1               5                   10                  15


Ala Leu Gly Ser Leu Leu Phe Leu Leu Phe Ser Leu Gly Trp Val Gln
            20                  25                  30

Pro Ser Arg Thr Leu Ala Gly Glu Thr Gly Gln Glu Ala Ala Pro Leu
        35                  40                  45

Asp Gly Val Leu Ala Asn Pro Pro Asn Ile Ser Ser Leu Ser Pro Arg
    50                  55                  60

Gln Leu Leu Gly Phe Pro Cys Ala Glu Val Ser Gly Leu Ser Thr Glu
65                  70                  75                  80

Arg Val Arg Glu Leu Ala Val Ala Leu Ala Gln Lys Asn Val Lys Leu
                85                  90                  95

Ser Thr Glu Gln Leu Arg Cys Leu Ala His Arg Leu Ser Glu Pro Pro
            100                 105                 110

Glu Asp Leu Asp Ala Leu Pro Leu Asp Leu Leu Leu Phe Leu Asn Pro
        115                 120                 125

Asp Ala Phe Ser Gly Pro Gln Ala Cys Thr Arg Phe Phe Ser Arg Ile
    130                 135                 140

Thr Lys Ala Asn Val Asp Leu Leu Pro Arg Gly Ala Pro Glu Arg Gln
145                 150                 155                 160

Arg Leu Leu Pro Ala Ala Leu Ala Cys Trp Gly Val Arg Gly Ser Leu
                165                 170                 175

Leu Ser Glu Ala Asp Val Arg Ala Leu Gly Gly Leu Ala Cys Asp Leu
            180                 185                 190

Pro Gly Arg Phe Val Ala Glu Ser Ala Glu Val Leu Leu Pro Arg Leu
        195                 200                 205

Val Ser Cys Pro Gly Pro Leu Asp Gln Asp Gln Gln Glu Ala Ala Arg
    210                 215                 220

Ala Ala Leu Gln Gly Gly Gly Pro Pro Tyr Gly Pro Pro Ser Thr Trp
225                 230                 235                 240

Ser Val Ser Thr Met Asp Ala Leu Arg Gly Leu Leu Pro Val Leu Gly
                245                 250                 255

Gln Pro Ile Ile Arg Ser Ile Pro Gln Gly Ile Val Ala Ala Trp Arg
            260                 265                 270

Gln Arg Ser Ser Arg Asp Pro Ser Trp Arg Gln Pro Glu Arg Thr Ile
        275                 280                 285

Leu Arg Pro Arg Phe Arg Arg Glu Val Glu Lys Thr Ala Cys Pro Ser
    290                 295                 300

Gly Lys Lys Ala Arg Glu Ile Asp Glu Ser Leu Ile Phe Tyr Lys Lys
305                 310                 315                 320

Trp Glu Leu Glu Ala Cys Val Asp Ala Ala Leu Leu Ala Thr Gln Met
                325                 330                 335

Asp Arg Val Asn Ala Ile Pro Phe Thr Tyr Glu Gln Leu Asp Val Leu
            340                 345                 350

Lys His Lys Leu Asp Glu Leu Tyr Pro Gln Gly Tyr Pro Glu Ser Val
        355                 360                 365

Ile Gln His Leu Gly Tyr Leu Phe Leu Lys Met Ser Pro Glu Asp Ile
    370                 375                 380

Arg Lys Trp Asn Val Thr Ser Leu Glu Thr Leu Lys Ala Leu Leu Glu
385                 390                 395                 400

Val Asn Lys Gly His Glu Met Ser Pro Gln Ala Pro Arg Arg Pro Leu
                405                 410                 415

Pro Gln Val Ala Thr Leu Ile Asp Arg Phe Val Lys Gly Arg Gly Gln
            420                 425                 430

Leu Asp Lys Asp Thr Leu Asp Thr Leu Thr Ala Phe Tyr Pro Gly Tyr
        435                 440                 445

Leu Cys Ser Leu Ser Pro Glu Glu Leu Ser Ser Val Pro Pro Ser Ser
    450                 455                 460

Ile Trp Ala Val Arg Pro Gln Asp Leu Asp Thr Cys Asp Pro Arg Gln
465                 470                 475                 480

Leu Asp Val Leu Tyr Pro Lys Ala Arg Leu Ala Phe Gln Asn Met Asn
                485                 490                 495

Gly Ser Glu Tyr Phe Val Lys Ile Gln Ser Phe Leu Gly Gly Ala Pro
            500                 505                 510

Thr Glu Asp Leu Lys Ala Leu Ser Gln Gln Asn Val Ser Met Asp Leu
        515                 520                 525

Ala Thr Phe Met Lys Leu Arg Thr Asp Ala Val Leu Pro Leu Thr Val
    530                 535                 540

Ala Glu Val Gln Lys Leu Leu Gly Pro His Val Glu Gly Leu Lys Ala
545                 550                 555                 560

Glu Glu Arg His Arg Pro Val Arg Asp Trp Ile Leu Arg Gln Arg Gln
                565                 570                 575

Asp Asp Leu Asp Thr Leu Gly Leu Gly Leu Gln Gly Gly Ile Pro Asn
            580                 585                 590

Gly Tyr Leu Val Leu Asp Leu Ser Met Gln Glu Ala Leu Ser Gly Thr
        595                 600                 605

Pro Cys Leu Leu Gly Pro Gly Pro Val Leu Thr Val Leu Ala Leu Leu
    610                 615                 620

Leu Ala Ser Thr Leu Ala
625                 630

<210> 131
<211> 246
<212> PRT
<213> Unknown

<220>
<223> Description of Unknown:
      ULBP-6 polypeptide

<400> 131
Met Ala Ala Ala Ala Ile Pro Ala Leu Leu Leu Cys Leu Pro Leu Leu
1               5                   10                  15

Phe Leu Leu Phe Gly Trp Ser Arg Ala Arg Arg Asp Asp Pro His Ser
            20                  25                  30

Leu Cys Tyr Asp Ile Thr Val Ile Pro Lys Phe Arg Pro Gly Pro Arg
        35                  40                  45

Trp Cys Ala Val Gln Gly Gln Val Asp Glu Lys Thr Phe Leu His Tyr
    50                  55                  60

Asp Cys Gly Asn Lys Thr Val Thr Pro Val Ser Pro Leu Gly Lys Lys
65                  70                  75                  80

Leu Asn Val Thr Met Ala Trp Lys Ala Gln Asn Pro Val Leu Arg Glu
                85                  90                  95

Val Val Asp Ile Leu Thr Glu Gln Leu Leu Asp Ile Gln Leu Glu Asn
            100                 105                 110

Tyr Thr Pro Lys Glu Pro Leu Thr Leu Gln Ala Arg Met Ser Cys Glu
        115                 120                 125

Gln Lys Ala Glu Gly His Ser Ser Gly Ser Trp Gln Phe Ser Ile Asp
    130                 135                 140

Gly Gln Thr Phe Leu Leu Phe Asp Ser Glu Lys Arg Met Trp Thr Thr
145                 150                 155                 160

Val His Pro Gly Ala Arg Lys Met Lys Glu Lys Trp Glu Asn Asp Lys
                165                 170                 175

Asp Val Ala Met Ser Phe His Tyr Ile Ser Met Gly Asp Cys Ile Gly
            180                 185                 190

Trp Leu Glu Asp Phe Leu Met Gly Met Asp Ser Thr Leu Glu Pro Ser
        195                 200                 205

Ala Gly Ala Pro Leu Ala Met Ser Ser Gly Thr Thr Gln Leu Arg Ala
    210                 215                 220

Thr Ala Thr Thr Leu Ile Leu Cys Cys Leu Leu Ile Ile Leu Pro Cys
225                 230                 235                 240

Phe Ile Leu Pro Gly Ile
                245

<210> 132
<211> 121
<212> PRT
<213> Artificial Sequence

<220>
<223> Description of Artificial Sequence: Synthetic
      polypeptide

<400> 132
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Asp Gly Gly Tyr Tyr Asp Ser Gly Ala Gly Asp Tyr Trp Gly
            100                 105                 110

Gln Gly Thr Leu Val Thr Val Ser Ser
        115                 120

<210> 133
<211> 107
<212> PRT
<213> Artificial Sequence

<220>
<223> Description of Artificial Sequence: Synthetic
      polypeptide

<400> 133
Asp Ile Gln Met Thr Gln Ser Pro Ser Ser Val Ser Ala Ser Val Gly
1               5                   10                  15

Asp Arg Val Thr Ile Thr Cys Arg Ala Ser Gln Gly Ile Asp Ser Trp
            20                  25                  30

Leu Ala Trp Tyr Gln Gln Lys Pro Gly Lys Ala Pro Lys Leu Leu Ile
        35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Gly Val Ser Tyr Pro Arg
                85                  90                  95

Thr Phe Gly Gly Gly Thr Lys Val Glu Ile Lys
            100                 105

<210> 134
<211> 9
<212> PRT
<213> Artificial Sequence

<220>
<223> Description of Artificial Sequence: Synthetic
      peptide

<400> 134
Phe Thr Phe Ser Ser Tyr Ala Met Ser
1               5

<210> 135
<211> 17
<212> PRT
<213> Artificial Sequence

<220>
<223> Description of Artificial Sequence: Synthetic
      peptide

<400> 135
Ala Ile Ser Gly Ser Gly Gly Ser Thr Tyr Tyr Ala Asp Ser Val Lys
1               5                   10                  15

Gly

<210> 136
<211> 14
<212> PRT
<213> Artificial Sequence

<220>
<223> Description of Artificial Sequence: Synthetic
      peptide

<400> 136
Ala Lys Asp Gly Gly Tyr Tyr Asp Ser Gly Ala Gly Asp Tyr
1               5                   10

<210> 137
<211> 11
<212> PRT
<213> Artificial Sequence

<220>
<223> Description of Artificial Sequence: Synthetic
      peptide

<400> 137
Arg Ala Ser Gln Gly Ile Asp Ser Trp Leu Ala
1               5                   10

<210> 138
<211> 7
<212> PRT
<213> Artificial Sequence

<220>
<223> Description of Artificial Sequence: Synthetic
      peptide

<400> 138
Ala Ala Ser Ser Leu Gln Ser
1               5

<210> 139
<211> 9
<212> PRT
<213> Artificial Sequence

<220>
<223> Description of Artificial Sequence: Synthetic
      peptide

<400> 139
Gln Gln Gly Val Ser Tyr Pro Arg Thr
1               5


<210> 140
<211> 122
<212> PRT
<213> Artificial Sequence

<220>
<223> Description of Artificial Sequence: Synthetic
      polypeptide

<400> 140
Glu Val Gln Leu Val Glu Ser Gly Gly Gly Leu Val Lys Pro Gly Gly
1               5                   10                  15


Ser Leu Arg Leu Ser Cys Ala Ala Ser Gly Phe Thr Phe Ser Ser Tyr
            20                  25                  30


Ser Met Asn Trp Val Arg Gln Ala Pro Gly Lys Gly Leu Glu Trp Val
        35                  40                  45


Ser Ser Ile Ser Ser Ser Ser Ser Tyr Ile Tyr Tyr Ala Asp Ser Val
    50                  55                  60


Lys Gly Arg Phe Thr Ile Ser Arg Asp Asn Ala Lys Asn Ser Leu Tyr
65                  70                  75                  80


Leu Gln Met Asn Ser Leu Arg Ala Glu Asp Thr Ala Val Tyr Tyr Cys
                85                  90                  95


Ala Arg Gly Ala Pro Met Gly Ala Ala Ala Gly Trp Phe Asp Pro Trp
            100                 105                 110


Gly Gln Gly Thr Leu Val Thr Val Ser Ser
        115                 120


<210> 141
<211> 107
<212> PRT
<213> Artificial Sequence

<220>
<223> Description of Artificial Sequence: Synthetic
      polypeptide

<400> 141
Asp Ile Gln Met Thr Gln Ser Pro Ser Ser Val Ser Ala Ser Val Gly
1               5                   10                  15

Asp Arg Val Thr Ile Thr Cys Arg Ala Ser Gln Gly Ile Ser Ser Trp
            20                  25                  30

Leu Ala Trp Tyr Gln Gln Lys Pro Gly Lys Ala Pro Lys Leu Leu Ile
        35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Gly Val Ser Phe Pro Arg
                85                  90                  95

Thr Phe Gly Gly Gly Thr Lys Val Glu Ile Lys
            100                 105


<210> 142
<211> 9
<212> PRT
<213> Artificial Sequence

<220>
<223> Description of Artificial Sequence: Synthetic
      peptide

<400> 142
Phe Thr Phe Ser Ser Tyr Ser Met Asn
1               5


<210> 143
<211> 17
<212> PRT
<213> Artificial Sequence

<220>
<223> Description of Artificial Sequence: Synthetic
      peptide

<400> 143
Ser Ile Ser Ser Ser Ser Ser Tyr Ile Tyr Tyr Ala Asp Ser Val Lys
1               5                   10                  15

Gly

<210> 144
<211> 15
<212> PRT
<213> Artificial Sequence

<220>
<223> Description of Artificial Sequence: Synthetic
      peptide

<400> 144
Ala Arg Gly Ala Pro Met Gly Ala Ala Ala Gly Trp Phe Asp Pro
1               5                   10                  15


<210> 145
<211> 11
<212> PRT
<213> Artificial Sequence

<220>
<223> Description of Artificial Sequence: Synthetic
      peptide

<400> 145
Arg Ala Ser Gln Gly Ile Ser Ser Trp Leu Ala
1               5                   10


<210> 146
<211> 7
<212> PRT
<213> Artificial Sequence

<220>
<223> Description of Artificial Sequence: Synthetic
      peptide

<400> 146
Ala Ala Ser Ser Leu Gln Ser
1               5


<210> 147
<211> 9
<212> PRT
<213> Artificial Sequence

<220>
<223> Description of Artificial Sequence: Synthetic
      peptide

<400> 147
Gln Gln Gly Val Ser Phe Pro Arg Thr
1               5


<210> 148
<211> 124
<212> PRT
<213> Artificial Sequence

<220>
<223> Description of Artificial Sequence: Synthetic
      polypeptide

<400> 148
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Tyr Met His Trp Val Arg Gln Ala Pro Gly Gln Gly Leu Glu Trp Met
        35                  40                  45

Gly Trp Ile Asn Pro Asn Ser Gly Gly Thr Asn Tyr Ala Gln Lys Phe
    50                  55                  60

Gln Gly Arg Val Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Asp Thr Gly Glu Tyr Tyr Asp Thr Asp Asp His Gly Met Asp
            100                 105                 110

Val Trp Gly Gln Gly Thr Thr Val Thr Val Ser Ser
        115                 120


<210> 149
<211> 107
<212> PRT
<213> Artificial Sequence

<220>
<223> Description of Artificial Sequence: Synthetic
      polypeptide

<400> 149
Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Ser Ser Asn
            20                  25                  30

Leu Ala Trp Tyr Gln Gln Lys Pro Gly Gln Ala Pro Arg Leu Leu Ile
        35                  40                  45

Tyr Gly Ala Ser Thr Arg Ala Thr Gly Ile Pro Ala Arg Phe Ser Gly
    50                  55                  60

Ser Gly Ser Gly Thr Glu Phe Thr Leu Thr Ile Ser Ser Leu Gln Ser
65                  70                  75                  80

Glu Asp Phe Ala Val Tyr Tyr Cys Gln Gln Asp Asp Tyr Trp Pro Pro
                85                  90                  95

Thr Phe Gly Gly Gly Thr Lys Val Glu Ile Lys
            100                 105

<210> 150
<211> 9
<212> PRT
<213> Artificial Sequence

<220>
<223> Description of Artificial Sequence: Synthetic
      peptide

<400> 150
Tyr Thr Phe Thr Gly Tyr Tyr Met His
1               5

<210> 151
<211> 17
<212> PRT
<213> Artificial Sequence

<220>
<223> Description of Artificial Sequence: Synthetic
      peptide

<400> 151
Trp Ile Asn Pro Asn Ser Gly Gly Thr Asn Tyr Ala Gln Lys Phe Gln
1               5                   10                  15

Gly

<210> 152
<211> 17
<212> PRT
<213> Artificial Sequence

<220>
<223> Description of Artificial Sequence: Synthetic
      peptide

<400> 152
Ala Arg Asp Thr Gly Glu Tyr Tyr Asp Thr Asp Asp His Gly Met Asp
1               5                   10                  15

Val

<210> 153

<211> 11
<212> PRT
<213> Artificial Sequence

<220>
<223> Description of Artificial Sequence: Synthetic
      peptide

<400> 153
Arg Ala Ser Gln Ser Val Ser Ser Asn Leu Ala
1               5                   10


<210> 154
<211> 7
<212> PRT
<213> Artificial Sequence

<220>
<223> Description of Artificial Sequence: Synthetic
      peptide

<400> 154
Gly Ala Ser Thr Arg Ala Thr
1               5


<210> 155
<211> 9
<212> PRT
<213> Artificial Sequence

<220>
<223> Description of Artificial Sequence: Synthetic
      peptide

<400> 155
Gln Gln Asp Asp Tyr Trp Pro Pro Thr
1               5


<210> 156
<211> 126
<212> PRT
<213> Artificial Sequence

<220>
<223> Description of Artificial Sequence: Synthetic
      polypeptide

<400> 156
Gln Val Gln Leu Val Gln Ser Gly Ala Glu Val Lys Lys Pro Gly Ala
1               5                   10                  15


Ser Val Lys Val Ser Cys Lys Ala Ser Gly Tyr Thr Phe Thr Ser Tyr
            20                  25                  30


Tyr Met His Trp Val Arg Gln Ala Pro Gly Gln Gly Leu Glu Trp Met
        35                  40                  45

Gly Ile Ile Asn Pro Ser Gly Gly Ser Thr Ser Tyr Ala Gln Lys Phe
    50                  55                  60

Gln Gly Arg Val Thr Met Thr Arg Asp Thr Ser Thr Ser Thr Val Tyr
65                  70                  75                  80

Met Glu Leu Ser Ser Leu Arg Ser Glu Asp Thr Ala Val Tyr Tyr Cys
                85                  90                  95

Ala Arg Gly Ala Pro Asn Tyr Gly Asp Thr Thr His Asp Tyr Tyr Tyr
            100                 105                 110

Met Asp Val Trp Gly Lys Gly Thr Thr Val Thr Val Ser Ser
        115                 120                 125

<210> 157
<211> 107
<212> PRT
<213> Artificial Sequence

<220>
<223> Description of Artificial Sequence: Synthetic
      polypeptide

<400> 157
Glu Ile Val Met Thr Gln Ser Pro Ala Thr Leu Ser Val Ser Pro Gly
1               5                   10                  15

Glu Arg Ala Thr Leu Ser Cys Arg Ala Ser Gln Ser Val Ser Ser Asn
            20                  25                  30

Leu Ala Trp Tyr Gln Gln Lys Pro Gly Gln Ala Pro Arg Leu Leu Ile
        35                  40                  45

Tyr Gly Ala Ser Thr Arg Ala Thr Gly Ile Pro Ala Arg Phe Ser Gly
    50                  55                  60

Ser Gly Ser Gly Thr Glu Phe Thr Leu Thr Ile Ser Ser Leu Gln Ser
65                  70                  75                  80

Glu Asp Phe Ala Val Tyr Tyr Cys Gln Gln Tyr Asp Asp Trp Pro Phe
                85                  90                  95

Thr Phe Gly Gly Gly Thr Lys Val Glu Ile Lys
            100                 105

<210> 158
<211> 9
<212> PRT
<213> Artificial Sequence

<220>
<223> Description of Artificial Sequence: Synthetic
      peptide

<400> 158
Tyr Thr Phe Thr Ser Tyr Tyr Met His
1               5

<210> 159
<211> 17
<212> PRT
<213> Artificial Sequence

<220>
<223> Description of Artificial Sequence: Synthetic
      peptide

<400> 159
Ile Ile Asn Pro Ser Gly Gly Ser Thr Ser Tyr Ala Gln Lys Phe Gln
1               5                   10                  15

Gly

<210> 160
<211> 19
<212> PRT
<213> Artificial Sequence

<220>
<223> Description of Artificial Sequence: Synthetic
      peptide

<400> 160
Ala Arg Gly Ala Pro Asn Tyr Gly Asp Thr Thr His Asp Tyr Tyr Tyr
1               5                   10                  15

Met Asp Val

<210> 161
<211> 11
<212> PRT
<213> Artificial Sequence

<220>
<223> Description of Artificial Sequence: Synthetic
      peptide

<400> 161
Arg Ala Ser Gln Ser Val Ser Ser Asn Leu Ala
1               5                   10

<210> 162
<211> 7
<212> PRT
<213> Artificial Sequence

<220>
<223> Description of Artificial Sequence: Synthetic
      peptide

<400> 162
Gly Ala Ser Thr Arg Ala Thr
1               5


<210> 163
<211> 9
<212> PRT
<213> Artificial Sequence

<220>
<223> Description of Artificial Sequence: Synthetic
      peptide

<400> 163
Gln Gln Tyr Asp Asp Trp Pro Phe Thr
1               5


<210> 164
<211> 117
<212> PRT
<213> Artificial Sequence

<220>
<223> Description of Artificial Sequence: Synthetic
      polypeptide

<400> 164
Gln Val Gln Leu Gln Gln Trp Gly Ala Gly Leu Leu Lys Pro Ser Glu
1               5                   10                  15

Thr Leu Ser Leu Thr Cys Ala Val Tyr Gly Gly Ser Phe Ser Gly Tyr
            20                  25                  30

Tyr Trp Ser Trp Ile Arg Gln Pro Pro Gly Lys Gly Leu Glu Trp Ile
        35                  40                  45

Gly Glu Ile Asp His Ser Gly Ser Thr Asn Tyr Asn Pro Ser Leu Lys
    50                  55                  60

Ser Arg Val Thr Ile Ser Val Asp Thr Ser Lys Asn Gln Phe Ser Leu
65                  70                  75                  80

Lys Leu Ser Ser Val Thr Ala Ala Asp Thr Ala Val Tyr Tyr Cys Ala
                85                  90                  95

Arg Ala Arg Gly Pro Trp Ser Phe Asp Pro Trp Gly Gln Gly Thr Leu
            100                 105                 110

Val Thr Val Ser Ser

        115

<210> 165
<211> 106
<212> PRT
<213> Artificial Sequence

<220>
<223> Description of Artificial Sequence: Synthetic
      polypeptide

<400> 165
Asp Ile Gln Met Thr Gln Ser Pro Ser Thr Leu Ser Ala Ser Val Gly
1               5                   10                  15

Asp Arg Val Thr Ile Thr Cys Arg Ala Ser Gln Ser Ile Ser Ser Trp
            20                  25                  30

Leu Ala Trp Tyr Gln Gln Lys Pro Gly Lys Ala Pro Lys Leu Leu Ile
        35                  40                  45

Tyr Lys Ala Ser Ser Leu Glu Ser Gly Val Pro Ser Arg Phe Ser Gly
    50                  55                  60

Ser Gly Ser Gly Thr Glu Phe Thr Leu Thr Ile Ser Ser Leu Gln Pro
65                  70                  75                  80

Asp Asp Phe Ala Thr Tyr Tyr Cys Gln Gln Tyr Asp Thr Phe Ile Thr
                85                  90                  95

Phe Gly Gly Gly Thr Lys Val Glu Ile Lys
            100                 105


<210> 166
<211> 9
<212> PRT
<213> Artificial Sequence

<220>
<223> Description of Artificial Sequence: Synthetic
      peptide

<400> 166
Gly Ser Phe Ser Gly Tyr Tyr Trp Ser
1               5


<210> 167
<211> 16
<212> PRT
<213> Artificial Sequence

<220>
<223> Description of Artificial Sequence: Synthetic
      peptide

<400> 167
Glu Ile Asp His Ser Gly Ser Thr Asn Tyr Asn Pro Ser Leu Lys Ser
1               5                   10                  15

<210> 168
<211> 11
<212> PRT
<213> Artificial Sequence

<220>
<223> Description of Artificial Sequence: Synthetic
      peptide

<400> 168
Ala Arg Ala Arg Gly Pro Trp Ser Phe Asp Pro
1               5                   10

<210> 169
<211> 11
<212> PRT
<213> Artificial Sequence

<220>
<223> Description of Artificial Sequence: Synthetic
      peptide

<400> 169
Arg Ala Ser Gln Ser Ile Ser Ser Trp Leu Ala
1               5                   10

<210> 170
<211> 7
<212> PRT
<213> Artificial Sequence

<220>
<223> Description of Artificial Sequence: Synthetic
      peptide

<400> 170
Lys Ala Ser Ser Leu Glu Ser
1               5

<210> 171
<211> 8
<212> PRT
<213> Artificial Sequence

<220>
<223> Description of Artificial Sequence: Synthetic
      peptide

<400> 171
Gln Gln Tyr Asp Thr Phe Ile Thr
1               5

<210> 172

<211> 121
<212> PRT
<213> Artificial Sequence

<220>
<223> Description of Artificial Sequence: Synthetic
      polypeptide

<400> 172
Ser Val Gln Leu Gln Gln Ser Gly Ser Glu Leu Lys Lys Pro Gly Ala
1               5                   10                  15

Ser Val Lys Val Ser Cys Lys Ala Ser Gly Tyr Thr Phe Thr Asn Tyr
            20                  25                  30

Gly Met Asn Trp Val Lys Gln Ala Pro Gly Gln Gly Leu Lys Trp Met
        35                  40                  45

Gly Trp Ile Asn Thr Tyr Thr Gly Glu Pro Thr Tyr Thr Asp Asp Phe
    50                  55                  60

Lys Gly Arg Phe Ala Phe Ser Leu Asp Thr Ser Val Ser Thr Ala Tyr
65                  70                  75                  80

Leu Gln Ile Ser Ser Leu Lys Ala Asp Asp Thr Ala Val Tyr Phe Cys
                85                  90                  95

Ala Arg Gly Gly Phe Gly Ser Ser Tyr Trp Tyr Phe Asp Val Trp Gly
            100                 105                 110

Gln Gly Ser Leu Val Thr Val Ser Ser
        115                 120


<210> 173
<211> 108
<212> PRT
<213> Artificial Sequence

<220>
<223> Description of Artificial Sequence: Synthetic
      polypeptide

<400> 173
Asp Ile Gln Leu Thr Gln Ser Pro Ser Ser Leu Ser Ala Ser Val Gly
1               5                   10                  15

Asp Arg Val Ser Ile Thr Cys Lys Ala Ser Gln Asp Val Ser Ile Ala
            20                  25                  30

Val Ala Trp Tyr Gln Gln Lys Pro Gly Lys Ala Pro Lys Leu Leu Ile
        35                  40                  45

Tyr Ser Ala Ser Tyr Arg Tyr Thr Gly Val Pro Asp Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Val Tyr Tyr Cys Gln Gln His Tyr Ile Thr Pro Leu
                85                  90                  95

Thr Phe Gly Ala Gly Thr Lys Val Glu Ile Lys Arg
            100                 105

<210> 174
<211> 7
<212> PRT
<213> Artificial Sequence

<220>
<223> Description of Artificial Sequence: Synthetic
      peptide

<400> 174
Gly Tyr Thr Phe Thr Asn Tyr
1               5

<210> 175
<211> 6
<212> PRT
<213> Artificial Sequence

<220>
<223> Description of Artificial Sequence: Synthetic
      peptide

<400> 175
Asn Thr Tyr Thr Gly Glu
1               5

<210> 176
<211> 12
<212> PRT
<213> Artificial Sequence

<220>
<223> Description of Artificial Sequence: Synthetic
      peptide

<400> 176
Gly Gly Phe Gly Ser Ser Tyr Trp Tyr Phe Asp Val
1               5                   10

<210> 177
<211> 8
<212> PRT
<213> Artificial Sequence

<220>
<223> Description of Artificial Sequence: Synthetic
      peptide

<400> 177
Gln Asp Val Ser Ile Ala Val Ala
1               5


<210> 178
<211> 7
<212> PRT
<213> Artificial Sequence

<220>
<223> Description of Artificial Sequence: Synthetic
      peptide

<400> 178
Ser Ala Ser Tyr Arg Tyr Thr
1               5


<210> 179
<211> 9
<212> PRT
<213> Artificial Sequence

<220>
<223> Description of Artificial Sequence: Synthetic
      peptide

<400> 179
Gln Gln His Tyr Ile Thr Pro Leu Thr
1               5


<210> 180
<211> 121
<212> PRT
<213> Artificial Sequence

<220>
<223> Description of Artificial Sequence: Synthetic
      polypeptide

<400> 180
Gln Ile Gln Leu Val Gln Ser Gly His Glu Val Lys Lys Pro Gly Ala
1               5                   10                  15


Ser Val Lys Val Ser Cys Lys Ala Ser Gly Tyr Thr Phe Thr Asn Tyr
            20                  25                  30


Gly Met Asn Trp Val Arg Gln Ala Pro Gly Gln Gly Leu Glu Trp Met
        35                  40                  45


Gly Trp Ile Asn Thr Lys Thr Gly Glu Pro Thr Tyr Ala Glu Glu Phe
    50                  55                  60

Lys Gly Arg Phe Val Phe Ser Leu Glu Thr Ser Ala Ser Thr Ala Tyr
65                  70                  75                  80

Leu Gln Ile Ser Ser Leu Lys Ala Glu Asp Thr Ala Met Tyr Phe Cys
                85                  90                  95

Gly Arg Gly Gly Tyr Gly Ser Ser Tyr Trp Tyr Phe Asp Val Trp Gly
            100                 105                 110

Gln Gly Thr Thr Val Thr Val Ser Ser
        115                 120

<210> 181
<211> 107
<212> PRT
<213> Artificial Sequence

<220>
<223> Description of Artificial Sequence: Synthetic
      polypeptide

<400> 181
Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Asp Val Ser Ile Ala
            20                  25                  30

Val Ala Trp Tyr Gln Gln Lys Pro Gly Lys Ala Pro Lys Val Leu Ile
        35                  40                  45

Tyr Ser Ala Ser Tyr Arg Tyr Thr Gly Val Pro Asp Arg Phe Ser Gly
    50                  55                  60

Ser Gly Ser Gly Thr Asp Phe Thr Phe Thr Ile Ser Ser Leu Gln Pro
65                  70                  75                  80

Glu Asp Ile Ala Val Tyr Tyr Cys Gln Gln His Tyr Ile Thr Pro Leu
                85                  90                  95

Thr Phe Gly Gly Gly Thr Lys Val Glu Ile Lys
            100                 105

<210> 182
<211> 7
<212> PRT
<213> Artificial Sequence

<220>
<223> Description of Artificial Sequence: Synthetic
      peptide

<400> 182
Gly Tyr Thr Phe Thr Asn Tyr
1               5

<210> 183
<211> 6
<212> PRT
<213> Artificial Sequence

<220>
<223> Description of Artificial Sequence: Synthetic
      peptide

<400> 183
Asn Thr Lys Thr Gly Glu
1               5

<210> 184
<211> 12
<212> PRT
<213> Artificial Sequence

<220>
<223> Description of Artificial Sequence: Synthetic
      peptide

<400> 184
Gly Gly Tyr Gly Ser Ser Tyr Trp Tyr Phe Asp Val
1               5                   10

<210> 185
<211> 8
<212> PRT
<213> Artificial Sequence

<220>
<223> Description of Artificial Sequence: Synthetic
      peptide

<400> 185
Gln Asp Val Ser Ile Ala Val Ala
1               5

<210> 186
<211> 7
<212> PRT
<213> Artificial Sequence

<220>
<223> Description of Artificial Sequence: Synthetic
      peptide

<400> 186
Ser Ala Ser Tyr Arg Tyr Thr
1               5

<210> 187
<211> 9

<212> PRT
<213> Artificial Sequence

<220>
<223> Description of Artificial Sequence: Synthetic
      peptide

<400> 187
Gln Gln His Tyr Ile Thr Pro Leu Thr
1               5


<210> 188
<211> 121
<212> PRT
<213> Artificial Sequence

<220>
<223> Description of Artificial Sequence: Synthetic
      polypeptide

<400> 188
Gln Val Gln Leu Val Gln Ser Gly Ala Glu Val Lys Lys Pro Gly Ala
1               5                   10                  15


Ser Val Lys Val Ser Cys Lys Ala Ser Gly Tyr Thr Phe Thr Thr Ala
            20                  25                  30


Gly Met Gln Trp Val Arg Gln Ala Pro Gly Gln Gly Leu Glu Trp Met
        35                  40                  45


Gly Trp Ile Asn Thr His Ser Gly Val Pro Lys Tyr Ala Glu Asp Phe
    50                  55                  60


Lys Gly Arg Val Thr Ile Ser Ala Asp Thr Ser Thr Ser Thr Ala Tyr
65                  70                  75                  80


Leu Gln Leu Ser Ser Leu Lys Ser Glu Asp Thr Ala Val Tyr Tyr Cys
                85                  90                  95


Ala Arg Ser Gly Phe Gly Ser Ser Tyr Trp Tyr Phe Asp Val Trp Gly
            100                 105                 110


Gln Gly Thr Leu Val Thr Val Ser Ser
        115                 120


<210> 189
<211> 109
<212> PRT
<213> Artificial Sequence

<220>
<223> Description of Artificial Sequence: Synthetic
      polypeptide

<400> 189
Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Asp Val Ser Thr Ala
            20                  25                  30

Val Ala Trp Tyr Gln Gln Lys Pro Gly Lys Ala Pro Lys Leu Leu Ile
        35                  40                  45

Tyr Ser Ala Ser Tyr Arg Tyr Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Val Tyr Tyr Cys Gln Gln His Tyr Ile Thr Pro Leu
                85                  90                  95

Thr Phe Gly Gln Gly Thr Lys Leu Glu Ile Lys Arg Thr
            100                 105

<210> 190
<211> 121
<212> PRT
<213> Artificial Sequence

<220>
<223> Description of Artificial Sequence: Synthetic
      polypeptide

<400> 190
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Thr Ala
            20                  25                  30

Gly Met Gln Trp Val Arg Gln Ala Pro Gly Gln Gly Leu Glu Trp Met
        35                  40                  45

Gly Trp Ile Asn Thr His Ser Gly Val Pro Lys Tyr Ala Glu Asp Phe
    50                  55                  60

Lys Gly Arg Val Thr Ile Ser Leu Asp Thr Ser Thr Ser Thr Ala Tyr
65                  70                  75                  80

Leu Gln Leu Ser Ser Leu Lys Ser Glu Asp Thr Ala Val Tyr Tyr Cys
                85                  90                  95

Ala Arg Ser Gly Phe Gly Ser Ser Tyr Trp Tyr Phe Asp Val Trp Gly
            100                 105                 110

Gln Gly Thr Leu Val Thr Val Ser Ser
        115                 120

<210> 191
<211> 109
<212> PRT
<213> Artificial Sequence

<220>
<223> Description of Artificial Sequence: Synthetic
      polypeptide

<400> 191
Asp Ile Val Met Thr Gln Ser Pro Ser Ser Leu Ser Ala Ser Val Gly
1               5                   10                  15

Asp Arg Val Thr Ile Thr Cys Lys Ala Ser Gln Asp Val Ser Thr Ala
            20                  25                  30

Val Ala Trp Tyr Gln Gln Lys Pro Gly Lys Ala Pro Lys Leu Leu Ile
        35                  40                  45

Tyr Ser Ala Ser Tyr Arg Tyr Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Val Tyr Tyr Cys Gln Gln His Tyr Ile Thr Pro Leu
                85                  90                  95

Thr Phe Gly Gln Gly Thr Lys Leu Glu Ile Lys Arg Thr
            100                 105

<210> 192
<211> 121
<212> PRT
<213> Artificial Sequence

<220>
<223> Description of Artificial Sequence: Synthetic
      polypeptide

<400> 192
Gln Ile Gln Leu Val Gln Ser Gly Ala Glu Val Lys Lys Pro Gly Glu
1               5                   10                  15

Ser Val Lys Val Ser Cys Lys Ala Ser Gly Tyr Thr Phe Thr Thr Ala
            20                  25                  30

Gly Met Gln Trp Val Gln Gln Met Pro Gly Lys Gly Leu Glu Trp Met
            35                  40                  45

Gly Trp Ile Asn Thr His Ser Gly Val Pro Lys Tyr Ala Glu Asp Phe
    50                  55                  60

Lys Gly Arg Val Thr Phe Ser Leu Asp Thr Ser Thr Ser Thr Ala Tyr
65                  70                  75                  80

Leu Gln Leu Ser Ser Leu Lys Ser Glu Asp Thr Ala Val Tyr Tyr Cys
                85                  90                  95

Ala Arg Ser Gly Phe Gly Ser Ser Tyr Trp Tyr Phe Asp Val Trp Gly
            100                 105                 110

Gln Gly Thr Leu Val Thr Val Ser Ser
        115                 120

<210> 193
<211> 108
<212> PRT
<213> Artificial Sequence

<220>
<223> Description of Artificial Sequence: Synthetic
      polypeptide

<400> 193
Asp Ile Val Met Thr Gln Ser Pro Ser Ser Leu Ser Ala Ser Val Gly
1               5                   10                  15

Asp Arg Val Thr Ile Thr Cys Lys Ala Ser Gln Asp Val Ser Thr Ala
            20                  25                  30

Val Ala Trp Tyr Gln Gln Lys Pro Gly Lys Gln Pro Lys Leu Leu Ile
        35                  40                  45

Tyr Ser Ala Ser Tyr Arg Tyr Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Val Tyr Tyr Cys Gln Gln His Tyr Ile Thr Pro Leu
                85                  90                  95

Thr Phe Gly Gln Gly Thr Lys Leu Glu Ile Lys Arg
            100                 105

<210> 194
<211> 323
<212> PRT
<213> Unknown

<220>
<223> Description of Unknown:
      TROP2 polypeptide

<400> 194
Met Ala Arg Gly Pro Gly Leu Ala Pro Pro Pro Leu Arg Leu Pro Leu
1               5                   10                  15

Leu Leu Leu Val Leu Ala Ala Val Thr Gly His Thr Ala Ala Gln Asp
            20                  25                  30

Asn Cys Thr Cys Pro Thr Asn Lys Met Thr Val Cys Ser Pro Asp Gly
        35                  40                  45

Pro Gly Gly Arg Cys Gln Cys Arg Ala Leu Gly Ser Gly Met Ala Val
    50                  55                  60

Asp Cys Ser Thr Leu Thr Ser Lys Cys Leu Leu Leu Lys Ala Arg Met
65                  70                  75                  80

Ser Ala Pro Lys Asn Ala Arg Thr Leu Val Arg Pro Ser Glu His Ala
                85                  90                  95

Leu Val Asp Asn Asp Gly Leu Tyr Asp Pro Asp Cys Asp Pro Glu Gly
            100                 105                 110

Arg Phe Lys Ala Arg Gln Cys Asn Gln Thr Ser Val Cys Trp Cys Val
        115                 120                 125

Asn Ser Val Gly Val Arg Arg Thr Asp Lys Gly Asp Leu Ser Leu Arg
    130                 135                 140

Cys Asp Glu Leu Val Arg Thr His His Ile Leu Ile Asp Leu Arg His
145                 150                 155                 160

Arg Pro Thr Ala Gly Ala Phe Asn His Ser Asp Leu Asp Ala Glu Leu
                165                 170                 175

Arg Arg Leu Phe Arg Glu Arg Tyr Arg Leu His Pro Lys Phe Val Ala
            180                 185                 190

Ala Val His Tyr Glu Gln Pro Thr Ile Gln Ile Glu Leu Arg Gln Asn
        195                 200                 205

Thr Ser Gln Lys Ala Ala Gly Asp Val Asp Ile Gly Asp Ala Ala Tyr

210                 215                 220

Tyr Phe Glu Arg Asp Ile Lys Gly Glu Ser Leu Phe Gln Gly Arg Gly
225                 230                 235                 240

Gly Leu Asp Leu Arg Val Arg Gly Glu Pro Leu Gln Val Glu Arg Thr
                245                 250                 255

Leu Ile Tyr Tyr Leu Asp Glu Ile Pro Pro Lys Phe Ser Met Lys Arg
            260                 265                 270

Leu Thr Ala Gly Leu Ile Ala Val Ile Val Val Val Val Ala Leu
        275                 280                 285

Val Ala Gly Met Ala Val Leu Val Ile Thr Asn Arg Arg Lys Ser Gly
    290                 295                 300

Lys Tyr Lys Lys Val Glu Ile Lys Glu Leu Gly Glu Leu Arg Lys Glu
305                 310                 315                 320

Pro Ser Leu

<210> 195
<211> 119
<212> PRT
<213> Artificial Sequence

<220>
<223> Description of Artificial Sequence: Synthetic
      polypeptide

<400> 195
Glu Val Gln Leu Val Glu Ser Gly Gly Gly Val Val Gln Pro Gly Arg
1               5                   10                  15

Ser Leu Arg Leu Ser Cys Ser Ala Ser Gly Phe Asp Phe Thr Thr Tyr
            20                  25                  30

Trp Met Ser Trp Val Arg Gln Ala Pro Gly Lys Gly Leu Glu Trp Ile
        35                  40                  45

Gly Glu Ile His Pro Asp Ser Ser Thr Ile Asn Tyr Ala Pro Ser Leu
    50                  55                  60

Lys Asp Arg Phe Thr Ile Ser Arg Asp Asn Ala Lys Asn Thr Leu Phe
65                  70                  75                  80

Leu Gln Met Asp Ser Leu Arg Pro Glu Asp Thr Gly Val Tyr Phe Cys
                85                  90                  95

Ala Ser Leu Tyr Phe Gly Phe Pro Trp Phe Ala Tyr Trp Gly Gln Gly
            100                 105                 110

Thr Pro Val Thr Val Ser Ser
        115

<210> 196
<211> 106
<212> PRT
<213> Artificial Sequence

<220>
<223> Description of Artificial Sequence: Synthetic
      polypeptide

<400> 196
Asp Ile Gln Leu Thr Gln Ser Pro Ser Ser Leu Ser Ala Ser Val Gly
1               5                   10                  15

Asp Arg Val Thr Ile Thr Cys Lys Ala Ser Gln Asp Val Gly Thr Ser
            20                  25                  30

Val Ala Trp Tyr Gln Gln Lys Pro Gly Lys Ala Pro Lys Leu Leu Ile
        35                  40                  45

Tyr Trp Thr Ser Thr Arg His Thr Gly Val Pro Ser Arg Phe Ser Gly
    50                  55                  60

Ser Gly Ser Gly Thr Asp Phe Thr Phe Thr Ile Ser Ser Leu Gln Pro
65                  70                  75                  80

Glu Asp Ile Ala Thr Tyr Tyr Cys Gln Gln Tyr Ser Leu Tyr Arg Ser
                85                  90                  95

Phe Gly Gln Gly Thr Lys Val Glu Ile Lys
            100                 105

<210> 197
<211> 7
<212> PRT
<213> Artificial Sequence

<220>
<223> Description of Artificial Sequence: Synthetic
      peptide

<400> 197
Gly Phe Asp Phe Thr Thr Tyr
1               5

<210> 198

<211> 6
<212> PRT
<213> Artificial Sequence

<220>
<223> Description of Artificial Sequence: Synthetic
      peptide

<400> 198
His Pro Asp Ser Ser Thr
1               5


<210> 199
<211> 10
<212> PRT
<213> Artificial Sequence

<220>
<223> Description of Artificial Sequence: Synthetic
      peptide

<400> 199
Leu Tyr Phe Gly Phe Pro Trp Phe Ala Tyr
1               5                   10


<210> 200
<211> 8
<212> PRT
<213> Artificial Sequence

<220>
<223> Description of Artificial Sequence: Synthetic
      peptide

<400> 200
Gln Asp Val Gly Thr Ser Val Ala
1               5


<210> 201
<211> 7
<212> PRT
<213> Artificial Sequence

<220>
<223> Description of Artificial Sequence: Synthetic
      peptide

<400> 201
Trp Thr Ser Thr Arg His Thr
1               5


<210> 202
<211> 8
<212> PRT
<213> Artificial Sequence

<220>
<223> Description of Artificial Sequence: Synthetic
      peptide

<400> 202
Gln Gln Tyr Ser Leu Tyr Arg Ser
1               5

<210> 203
<211> 122
<212> PRT
<213> Artificial Sequence

<220>
<223> Description of Artificial Sequence: Synthetic
      polypeptide

<400> 203
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Glu Phe
            20                  25                  30

Gly Met Asn Trp Val Arg Gln Ala Pro Gly Gln Gly Leu Glu Trp Met
        35                  40                  45

Gly Trp Ile Asn Thr Lys Thr Gly Glu Ala Thr Tyr Val Glu Glu Phe
    50                  55                  60

Lys Gly Arg Val Thr Phe Thr Thr Asp Thr Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Ala Arg Trp Asp Phe Ala Tyr Tyr Val Glu Ala Met Asp Tyr Trp Gly
            100                 105                 110

Gln Gly Thr Thr Val Thr Val Ser Ser Ala
        115                 120

<210> 204
<211> 109
<212> PRT
<213> Artificial Sequence

<220>
<223> Description of Artificial Sequence: Synthetic
      polypeptide

<400> 204
Asp Ile Gln Met Thr Gln Ser Pro Ser Ser Leu Ser Ala Ser Val Gly
1               5                   10                  15

Asp Arg Val Thr Ile Thr Cys Lys Ala Ser Ala Ala Val Gly Thr Tyr

20                  25                  30

Val Ala Trp Tyr Gln Gln Lys Pro Gly Lys Ala Pro Lys Leu Leu Ile
        35                  40                  45

Tyr Ser Ala Ser Tyr Arg Lys Arg Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys His Gln Tyr Tyr Thr Tyr Pro Leu
                85                  90                  95

Phe Thr Phe Gly Gln Gly Thr Lys Leu Glu Ile Lys Arg
            100                 105

<210> 205
<211> 7
<212> PRT
<213> Artificial Sequence

<220>
<223> Description of Artificial Sequence: Synthetic
      peptide

<400> 205
Gly Tyr Thr Phe Thr Glu Phe
1               5

<210> 206
<211> 6
<212> PRT
<213> Artificial Sequence

<220>
<223> Description of Artificial Sequence: Synthetic
      peptide

<400> 206
Asn Thr Lys Thr Gly Glu
1               5

<210> 207
<211> 12
<212> PRT
<213> Artificial Sequence

<220>
<223> Description of Artificial Sequence: Synthetic
      peptide

<400> 207
Trp Asp Phe Ala Tyr Tyr Val Glu Ala Met Asp Tyr
1               5                   10

<210> 208
<211> 8
<212> PRT
<213> Artificial Sequence

<220>
<223> Description of Artificial Sequence: Synthetic
      peptide

<400> 208
Ala Ala Val Gly Thr Tyr Val Ala
1               5


<210> 209
<211> 7
<212> PRT
<213> Artificial Sequence

<220>
<223> Description of Artificial Sequence: Synthetic
      peptide

<400> 209
Ser Ala Ser Tyr Arg Lys Arg
1               5


<210> 210
<211> 10
<212> PRT
<213> Artificial Sequence

<220>
<223> Description of Artificial Sequence: Synthetic
      peptide

<400> 210
His Gln Tyr Tyr Thr Tyr Pro Leu Phe Thr
1               5                   10


<210> 211
<211> 121
<212> PRT
<213> Artificial Sequence

<220>
<223> Description of Artificial Sequence: Synthetic
      polypeptide

<400> 211
Gln Val Gln Leu Gln Gln Ser Gly Ala Glu Leu Val Arg Ser Gly Ala
1               5                   10                  15


Ser Ile Lys Leu Ser Cys Thr Ala Ser Gly Phe Asn Ile Lys His Tyr
            20                  25                  30


Tyr Met His Trp Val Lys Gln Arg Pro Glu Gln Gly Leu Glu Trp Ile

35                  40                  45

Gly Trp Ile Asn Pro Glu Asn Val Asp Thr Glu Tyr Ala Pro Lys Phe
    50                  55                  60

Gln Gly Lys Ala Thr Met Thr Ala Asp Thr Ser Ser Asn Thr Ala Tyr
65                  70                  75                  80

Leu Gln Leu Ser Ser Leu Thr Ser Glu Asp Thr Ala Val Tyr Tyr Cys
                85                  90                  95

Asn His Tyr Arg Tyr Ala Gly Gly Gly Ala Leu Asp Tyr Trp Gly Gln
            100                 105                 110

Gly Thr Thr Val Thr Val Ser Ser Ala
        115                 120

<210> 212
<211> 108
<212> PRT
<213> Artificial Sequence

<220>
<223> Description of Artificial Sequence: Synthetic
      polypeptide

<400> 212
Asp Ile Gln Leu Thr Gln Ser Pro Ala Ile Met Ser Ala Ser Pro Gly
1               5                   10                  15

Glu Lys Val Thr Met Thr Cys Ser Ala Ser Ser Ser Val Ser Tyr Ile
            20                  25                  30

His Trp Tyr Gln Gln Lys Ser Gly Thr Ser Pro Lys Arg Trp Val Tyr
        35                  40                  45

Asp Thr Ser Lys Leu Ala Ser Gly Val Pro Ala Arg Phe Ser Gly Ser
    50                  55                  60

Gly Ser Gly Thr Ser Tyr Ser Leu Thr Ile Ser Thr Met Glu Ala Glu
65                  70                  75                  80

Asp Ala Ala Thr Tyr Tyr Cys Gln Gln Trp Asn Asn Asn Pro Tyr Ser
                85                  90                  95

Phe Gly Gly Gly Thr Lys Val Val Ile Lys Thr Val
            100                 105

<210> 213
<211> 7

<212> PRT
<213> Artificial Sequence

<220>
<223> Description of Artificial Sequence: Synthetic
      peptide

<400> 213
Gly Phe Asn Ile Lys His Tyr
1               5


<210> 214
<211> 6
<212> PRT
<213> Artificial Sequence

<220>
<223> Description of Artificial Sequence: Synthetic
      peptide

<400> 214
Asn Pro Glu Asn Val Asp
1               5


<210> 215
<211> 11
<212> PRT
<213> Artificial Sequence

<220>
<223> Description of Artificial Sequence: Synthetic
      peptide

<400> 215
Tyr Arg Tyr Ala Gly Gly Gly Ala Leu Asp Tyr
1               5                   10


<210> 216
<211> 7
<212> PRT
<213> Artificial Sequence

<220>
<223> Description of Artificial Sequence: Synthetic
      peptide

<400> 216
Ser Ser Val Ser Tyr Ile His
1               5


<210> 217
<211> 7
<212> PRT
<213> Artificial Sequence

<220>
<223> Description of Artificial Sequence: Synthetic
      peptide

<400> 217
Asp Thr Ser Lys Leu Ala Ser
1               5

<210> 218
<211> 9
<212> PRT
<213> Artificial Sequence

<220>
<223> Description of Artificial Sequence: Synthetic
      peptide

<400> 218
Gln Gln Trp Asn Asn Asn Pro Tyr Ser
1               5

<210> 219
<211> 702
<212> PRT
<213> Unknown

<220>
<223> Description of Unknown:
      CEA polypeptide

<400> 219
Met Glu Ser Pro Ser Ala Pro Pro His Arg Trp Cys Ile Pro Trp Gln
1               5                   10                  15

Arg Leu Leu Leu Thr Ala Ser Leu Leu Thr Phe Trp Asn Pro Pro Thr
            20                  25                  30

Thr Ala Lys Leu Thr Ile Glu Ser Thr Pro Phe Asn Val Ala Glu Gly
        35                  40                  45

Lys Glu Val Leu Leu Leu Val His Asn Leu Pro Gln His Leu Phe Gly
    50                  55                  60

Tyr Ser Trp Tyr Lys Gly Glu Arg Val Asp Gly Asn Arg Gln Ile Ile
65                  70                  75                  80

Gly Tyr Val Ile Gly Thr Gln Gln Ala Thr Pro Gly Pro Ala Tyr Ser
                85                  90                  95

Gly Arg Glu Ile Ile Tyr Pro Asn Ala Ser Leu Leu Ile Gln Asn Ile
            100                 105                 110

Ile Gln Asn Asp Thr Gly Phe Tyr Thr Leu His Val Ile Lys Ser Asp
        115                 120                 125

Leu Val Asn Glu Glu Ala Thr Gly Gln Phe Arg Val Tyr Pro Glu Leu
    130                 135                 140

Pro Lys Pro Ser Ile Ser Ser Asn Asn Ser Lys Pro Val Glu Asp Lys
145                 150                 155                 160

Asp Ala Val Ala Phe Thr Cys Glu Pro Glu Thr Gln Asp Ala Thr Tyr
                165                 170                 175

Leu Trp Trp Val Asn Asn Gln Ser Leu Pro Val Ser Pro Arg Leu Gln
            180                 185                 190

Leu Ser Asn Gly Asn Arg Thr Leu Thr Leu Phe Asn Val Thr Arg Asn
        195                 200                 205

Asp Thr Ala Ser Tyr Lys Cys Glu Thr Gln Asn Pro Val Ser Ala Arg
    210                 215                 220

Arg Ser Asp Ser Val Ile Leu Asn Val Leu Tyr Gly Pro Asp Ala Pro
225                 230                 235                 240

Thr Ile Ser Pro Leu Asn Thr Ser Tyr Arg Ser Gly Glu Asn Leu Asn
                245                 250                 255

Leu Ser Cys His Ala Ala Ser Asn Pro Pro Ala Gln Tyr Ser Trp Phe
            260                 265                 270

Val Asn Gly Thr Phe Gln Gln Ser Thr Gln Glu Leu Phe Ile Pro Asn
        275                 280                 285

Ile Thr Val Asn Asn Ser Gly Ser Tyr Thr Cys Gln Ala His Asn Ser
    290                 295                 300

Asp Thr Gly Leu Asn Arg Thr Thr Val Thr Thr Ile Thr Val Tyr Ala
305                 310                 315                 320

Glu Pro Pro Lys Pro Phe Ile Thr Ser Asn Asn Ser Asn Pro Val Glu
                325                 330                 335

Asp Glu Asp Ala Val Ala Leu Thr Cys Glu Pro Glu Ile Gln Asn Thr
            340                 345                 350

Thr Tyr Leu Trp Trp Val Asn Asn Gln Ser Leu Pro Val Ser Pro Arg
        355                 360                 365

Leu Gln Leu Ser Asn Asp Asn Arg Thr Leu Thr Leu Leu Ser Val Thr
    370                 375                 380

Arg Asn Asp Val Gly Pro Tyr Glu Cys Gly Ile Gln Asn Lys Leu Ser

385                 390                 395                 400

Val Asp His Ser Asp Pro Val Ile Leu Asn Val Leu Tyr Gly Pro Asp
                405                 410                 415

Asp Pro Thr Ile Ser Pro Ser Tyr Thr Tyr Tyr Arg Pro Gly Val Asn
            420                 425                 430

Leu Ser Leu Ser Cys His Ala Ala Ser Asn Pro Pro Ala Gln Tyr Ser
        435                 440                 445

Trp Leu Ile Asp Gly Asn Ile Gln Gln His Thr Gln Glu Leu Phe Ile
    450                 455                 460

Ser Asn Ile Thr Glu Lys Asn Ser Gly Leu Tyr Thr Cys Gln Ala Asn
465                 470                 475                 480

Asn Ser Ala Ser Gly His Ser Arg Thr Thr Val Lys Thr Ile Thr Val
                485                 490                 495

Ser Ala Glu Leu Pro Lys Pro Ser Ile Ser Ser Asn Asn Ser Lys Pro
            500                 505                 510

Val Glu Asp Lys Asp Ala Val Ala Phe Thr Cys Glu Pro Glu Ala Gln
        515                 520                 525

Asn Thr Thr Tyr Leu Trp Trp Val Asn Gly Gln Ser Leu Pro Val Ser
    530                 535                 540

Pro Arg Leu Gln Leu Ser Asn Gly Asn Arg Thr Leu Thr Leu Phe Asn
545                 550                 555                 560

Val Thr Arg Asn Asp Ala Arg Ala Tyr Val Cys Gly Ile Gln Asn Ser
                565                 570                 575

Val Ser Ala Asn Arg Ser Asp Pro Val Thr Leu Asp Val Leu Tyr Gly
            580                 585                 590

Pro Asp Thr Pro Ile Ile Ser Pro Pro Asp Ser Ser Tyr Leu Ser Gly
        595                 600                 605

Ala Asn Leu Asn Leu Ser Cys His Ser Ala Ser Asn Pro Ser Pro Gln
    610                 615                 620

Tyr Ser Trp Arg Ile Asn Gly Ile Pro Gln Gln His Thr Gln Val Leu
625                 630                 635                 640

Phe Ile Ala Lys Ile Thr Pro Asn Asn Asn Gly Thr Tyr Ala Cys Phe
                645                 650                 655

Val Ser Asn Leu Ala Thr Gly Arg Asn Asn Ser Ile Val Lys Ser Ile
            660                 665                 670

Thr Val Ser Ala Ser Gly Thr Ser Pro Gly Leu Ser Ala Gly Ala Thr
        675                 680                 685

Val Gly Ile Met Ile Gly Val Leu Val Gly Val Ala Leu Ile
    690                 695                 700

<210> 220
<211> 118
<212> PRT
<213> Artificial Sequence

<220>
<223> Description of Artificial Sequence: Synthetic
      polypeptide

<400> 220
Gln Ile Gln Leu Val Gln Ser Gly Pro Glu Leu Lys Lys Pro Gly Glu
1               5                   10                  15

Thr Val Lys Ile Ser Cys Lys Ala Ser Gly Tyr Thr Phe Thr Asn Tyr
            20                  25                  30

Gly Met Asn Trp Val Lys Gln Ala Pro Gly Lys Gly Leu Lys Trp Met
        35                  40                  45

Gly Trp Ile Asn Thr Asn Thr Gly Glu Pro Thr Tyr Ala Glu Glu Phe
    50                  55                  60

Lys Gly Arg Phe Ala Phe Ser Leu Glu Thr Ser Ala Ser Thr Ala Tyr
65                  70                  75                  80

Leu Gln Ile Asn Asn Leu Lys Asn Glu Asp Thr Ala Thr Tyr Phe Cys
                85                  90                  95

Ala Arg Leu Gly Phe Gly Asn Ala Met Asp Tyr Trp Gly Gln Gly Thr
            100                 105                 110

Ser Val Thr Val Ser Ser
        115

<210> 221
<211> 113
<212> PRT
<213> Artificial Sequence

<220>
<223> Description of Artificial Sequence: Synthetic
      polypeptide

<400> 221
Asp Ile Val Met Thr Gln Ser Pro Ser Ser Leu Thr Val Thr Ala Gly
1               5                   10                  15

Glu Lys Val Thr Met Ser Cys Lys Ser Ser Gln Ser Leu Leu Asn Ser
            20                  25                  30

Gly Asn Gln Lys Asn Tyr Leu Thr Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Gln Ala Glu Asp Leu Ala Val Tyr Tyr Cys Gln Asn
                85                  90                  95

Asp Tyr Ser Tyr Pro Leu Thr Phe Gly Ala Gly Thr Lys Leu Glu Leu
            100                 105                 110

Lys

<210> 222
<211> 8
<212> PRT
<213> Artificial Sequence

<220>
<223> Description of Artificial Sequence: Synthetic
      peptide

<400> 222
Gly Tyr Thr Phe Thr Asn Tyr Gly
1               5

<210> 223
<211> 8
<212> PRT
<213> Artificial Sequence

<220>
<223> Description of Artificial Sequence: Synthetic
      peptide

<400> 223
Ile Asn Thr Asn Thr Gly Glu Pro
1               5

<210> 224
<211> 11
<212> PRT
<213> Artificial Sequence

<220>
<223> Description of Artificial Sequence: Synthetic
      peptide

<400> 224
Ala Arg Leu Gly Phe Gly Asn Ala Met Asp Tyr
1               5                   10


<210> 225
<211> 12
<212> PRT
<213> Artificial Sequence

<220>
<223> Description of Artificial Sequence: Synthetic
      peptide

<400> 225
Gln Ser Leu Leu Asn Ser Gly Asn Gln Lys Asn Tyr
1               5                   10


<210> 226
<211> 3
<212> PRT
<213> Artificial Sequence

<220>
<223> Description of Artificial Sequence: Synthetic
      peptide

<400> 226
Trp Ala Ser
1


<210> 227
<211> 9
<212> PRT
<213> Artificial Sequence

<220>
<223> Description of Artificial Sequence: Synthetic
      peptide

<400> 227
Gln Asn Asp Tyr Ser Tyr Pro Leu Thr
1               5


<210> 228
<211> 118
<212> PRT
<213> Artificial Sequence

<220>
<223> Description of Artificial Sequence: Synthetic
      polypeptide

<400> 228
Gln Val Gln Leu Gln Gln Pro Gly Ala Glu Leu Val Arg Pro Gly Ala
1               5                   10                  15

Ser Val Lys Leu Ser Cys Lys Ala Ser Gly Tyr Thr Phe Thr Ser Tyr
            20                  25                  30

Trp Ile Asn Trp Val Lys Gln Arg Pro Gly Gln Gly Leu Glu Trp Ile
        35                  40                  45

Gly Asn Ile Tyr Pro Ser Asp Ser Tyr Thr Asn Tyr Asn Gln Lys Phe
    50                  55                  60

Lys Asp Lys Ala Thr Leu Thr Val Asp Lys Ser Ser Ser Thr Ala Tyr
65                  70                  75                  80

Met Gln Leu Ser Ser Pro Thr Ser Glu Asp Ser Ala Val Tyr Tyr Cys
                85                  90                  95

Thr Arg Ser Trp Arg Gly Asn Ser Phe Asp Tyr Trp Gly Gln Gly Thr
            100                 105                 110

Thr Leu Thr Val Ser Ser
        115


<210> 229
<211> 113
<212> PRT
<213> Artificial Sequence

<220>
<223> Description of Artificial Sequence: Synthetic
      polypeptide

<400> 229
Asp Ile Val Met Thr Gln Ser Pro Ser Ser Leu Thr Val Thr Ala Gly
1               5                   10                  15

Glu Lys Val Thr Met Ser Cys Lys Ser Ser Gln Ser Leu Leu Asn Ser
            20                  25                  30

Gly Asn Gln Lys Asn Tyr Leu Thr Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Gln Ala Glu Asp Leu Ala Val Tyr Tyr Cys Gln Asn
                85                  90                  95

Asp Tyr Ser Tyr Pro Phe Thr Phe Gly Ser Gly Thr Lys Leu Glu Ile
            100                 105                 110

Lys

<210> 230
<211> 8
<212> PRT
<213> Artificial Sequence

<220>
<223> Description of Artificial Sequence: Synthetic
      peptide

<400> 230
Gly Tyr Thr Phe Thr Ser Tyr Trp
1               5

<210> 231
<211> 8
<212> PRT
<213> Artificial Sequence

<220>
<223> Description of Artificial Sequence: Synthetic
      peptide

<400> 231
Ile Tyr Pro Ser Asp Ser Tyr Thr
1               5

<210> 232
<211> 11
<212> PRT
<213> Artificial Sequence

<220>
<223> Description of Artificial Sequence: Synthetic
      peptide

<400> 232
Thr Arg Ser Trp Arg Gly Asn Ser Phe Asp Tyr
1               5                   10

<210> 233
<211> 12
<212> PRT
<213> Artificial Sequence

<220>
<223> Description of Artificial Sequence: Synthetic
      peptide

<400> 233
Gln Ser Leu Leu Asn Ser Gly Asn Gln Lys Asn Tyr
1               5                   10


<210> 234
<211> 3
<212> PRT
<213> Artificial Sequence

<220>
<223> Description of Artificial Sequence: Synthetic
      peptide

<400> 234
Trp Ala Ser
1


<210> 235
<211> 9
<212> PRT
<213> Artificial Sequence

<220>
<223> Description of Artificial Sequence: Synthetic
      peptide

<400> 235
Gln Asn Asp Tyr Ser Tyr Pro Phe Thr
1               5


<210> 236
<211> 261
<212> PRT
<213> Unknown

<220>
<223> Description of Unknown:
      Claudin-18.2 polypeptide

<400> 236
Met Ala Val Thr Ala Cys Gln Gly Leu Gly Phe Val Val Ser Leu Ile
1               5                   10                  15


Gly Ile Ala Gly Ile Ile Ala Ala Thr Cys Met Asp Gln Trp Ser Thr
            20                  25                  30


Gln Asp Leu Tyr Asn Asn Pro Val Thr Ala Val Phe Asn Tyr Gln Gly
        35                  40                  45


Leu Trp Arg Ser Cys Val Arg Glu Ser Ser Gly Phe Thr Glu Cys Arg
    50                  55                  60

Gly Tyr Phe Thr Leu Leu Gly Leu Pro Ala Met Leu Gln Ala Val Arg
65                  70                  75                  80

Ala Leu Met Ile Val Gly Ile Val Leu Gly Ala Ile Gly Leu Leu Val
                85                  90                  95

Ser Ile Phe Ala Leu Lys Cys Ile Arg Ile Gly Ser Met Glu Asp Ser
            100                 105                 110

Ala Lys Ala Asn Met Thr Leu Thr Ser Gly Ile Met Phe Ile Val Ser
        115                 120                 125

Gly Leu Cys Ala Ile Ala Gly Val Ser Val Phe Ala Asn Met Leu Val
    130                 135                 140

Thr Asn Phe Trp Met Ser Thr Ala Asn Met Tyr Thr Gly Met Gly Gly
145                 150                 155                 160

Met Val Gln Thr Val Gln Thr Arg Tyr Thr Phe Gly Ala Ala Leu Phe
                165                 170                 175

Val Gly Trp Val Ala Gly Gly Leu Thr Leu Ile Gly Gly Val Met Met
            180                 185                 190

Cys Ile Ala Cys Arg Gly Leu Ala Pro Glu Glu Thr Asn Tyr Lys Ala
        195                 200                 205

Val Ser Tyr His Ala Ser Gly His Ser Val Ala Tyr Lys Pro Gly Gly
    210                 215                 220

Phe Lys Ala Ser Thr Gly Phe Gly Ser Asn Thr Lys Asn Lys Lys Ile
225                 230                 235                 240

Tyr Asp Gly Gly Ala Arg Thr Glu Asp Glu Val Gln Ser Tyr Pro Ser
                245                 250                 255

Lys His Asp Tyr Val
                260